(19) Europäisches Patentamt
European Patent Office
Office européen des brevets

(11) **EP 3 739 644 A2**

(12) **EUROPEAN PATENT APPLICATION**

(43) Date of publication:
**18.11.2020 Bulletin 2020/47**

(21) Application number: **20165051.2**

(22) Date of filing: **23.03.2020**

(51) Int Cl.:
***H01L 51/50*** *(2006.01)* ***C07D 209/82*** *(2006.01)*
***C07F 15/00*** *(2006.01)* ***C09K 13/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.03.2019 KR 20190034492**

(71) Applicant: **Samsung Display Co., Ltd.**
**Gyeonggi-Do (KR)**

(72) Inventors:
- **KIM, Sehun**
  **Yongin-si (KR)**
- **LYU, Jaejin**
  **Yongin-si (KR)**

(74) Representative: **Shearman, James Ward**
**Marks & Clerk LLP**
**15 Fetter Lane**
**London EC4A 1BW (GB)**

(54) **ORGANIC LIGHT-EMITTING DEVICE AND ELECTRONIC APPARATUS**

(57) Provided are an organic light-emitting device and an electronic apparatus including the organic light-emitting device. The organic light emitting device includes a first electrode, a second electrode facing the first electrode, and an organic layer disposed between the first electrode and the second electrode. The organic layer includes an emission layer that has a host and a phosphorescent dopant, the host satisfying Equation 1 and Equation 2, and the host and the phosphorescent dopant satisfy Equation 3, where Equation 1: $S1(H) - T1(H) \geq 0.3$ eV; Equation 2: $T1(H) \leq 2.7$ eV; and Equation 3: $T1(D) \geq T1(H)$. In Equations 1, 2, and 3, T1(H) indicates a triplet energy of the host, S1(H) indicates a singlet energy of the host, and T1(D) indicates a triplet energy of the phosphorescent dopant.

FIG. 1

10

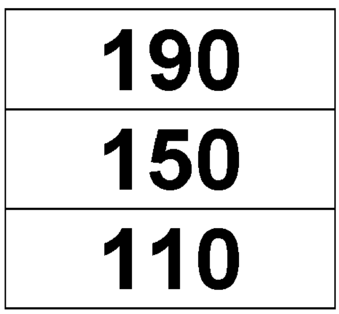


EP 3 739 644 A2

Printed by Jouve, 75001 PARIS (FR)

**Description**

BACKGROUND

FIELD

Embodiments of the invention relate generally to an organic light-emitting device and an electronic apparatus including the organic light-emitting device.

DISCUSSION OF THE BACKGROUND

Organic light-emitting devices (OLEDs) are self-emission devices that, as compared with conventional devices, have wide viewing angles, high contrast ratios, short response times, and excellent characteristics in terms of brightness, driving voltage, and response speed, and produce full-color images.

The OLEDs may include a first electrode on a substrate, and a hole transport region, an emission layer, an electron transport region, and a second electrode sequentially stacked on the first electrode. Holes provided from the first electrode may move toward the emission layer through the hole transport region. Electrons provided from the second electrode may move toward the emission layer through the electron transport region. Carriers, such as holes and electrons, recombine in the emission layer to produce excitons. These excitons transit from an excited state to a ground state to thereby generate light.

The above information disclosed in this Background section is only for understanding of the background of the inventive concepts, and, therefore, it may contain information that does not constitute prior art.

SUMMARY

One or more embodiments include an organic light-emitting device having a low driving voltage and excellent external quantum efficiency and an electronic apparatus including the organic light-emitting device.

Additional features of the inventive concepts will be set forth in the description which follows, and in part will be apparent from the description, or may be learned by practice of the inventive concepts.

According to one or more embodiments, an organic light-emitting device includes a first electrode; a second electrode facing the first electrode; and an organic layer between the first electrode and the second electrode, wherein the organic layer includes an emission layer, the emission layer includes a host and a phosphorescent dopant, the host satisfies Equations 1 and 2, and the host and the phosphorescent dopant satisfy Equation 3:

Equation 1

$$S1(H) - T1(H) \geq 0.3 \text{ eV}$$

Equation 2

$$T1(H) \leq 2.7 \text{ eV}$$

Equation 3

$$T1(D) \geq T1(H)$$

wherein, in Equations 1 to 3, T1(H) indicates a triplet energy of the host, S1(H) indicates a singlet energy of the host, and T1(D) indicates a triplet energy of the phosphorescent dopant.

According to one or more embodiments, an electronic apparatus includes the organic light-emitting device and a thin film transistor, wherein the first electrode of the organic light-emitting device is electrically connected to one of a source electrode and a drain electrode of the thin film transistor.

At least some of the above and other features of the invention are set out in the claims.

It is to be understood that both the foregoing general description and the following detailed description are explanatory and are intended to provide further explanation of the invention as claimed.

BRIEF DESCRIPTION OF THE DRAWINGS

The accompanying drawings, which are included to provide a further understanding of the invention and are incorporated in and constitute a part of this specification, illustrate embodiments of the invention, and together with the description serve to explain the inventive concepts.
FIG. 1 is a schematic cross-sectional view illustrating an organic light-emitting device according to an embodiment;
FIG. 2 is a schematic cross-sectional view illustrating an organic light-emitting device according to an embodiment;
FIG. 3 is a schematic cross-sectional view illustrating an organic light-emitting device according to an embodiment; and
FIG. 4 is a schematic cross-sectional view illustrating an organic light-emitting device according to an embodiment.

DETAILED DESCRIPTION

In the following description, for the purposes of explanation, numerous specific details are set forth in order to provide a thorough understanding of various embodiments or implementations of the invention. As used herein "embodiments" and "implementations" are interchangeable words that are non-limiting examples of devices or methods employing one or more of the inventive concepts disclosed herein. It is apparent, however, that various embodiments may be practiced without these specific details or with one or more equivalent arrangements. In other instances, well-known structures and devices are shown in block diagram form in order to avoid unnecessarily obscuring various embodiments. Further, various embodiments may be different, but do not have to be exclusive. For example, specific shapes, configurations, and characteristics of an embodiment may be used or implemented in another embodiment without departing from the inventive concepts.
Unless otherwise specified, the illustrated embodiments are to be understood as providing features of varying detail of some ways in which the inventive concepts may be implemented in practice. Therefore, unless otherwise specified, the features, components, modules, layers, films, panels, regions, and/or aspects, etc. (hereinafter individually or collectively referred to as "elements"), of the various embodiments may be otherwise combined, separated, interchanged, and/or rearranged without departing from the inventive concepts.
In the accompanying drawings, the size and relative sizes of elements may be exaggerated for clarity and/or descriptive purposes. When an embodiment may be implemented differently, a specific process order may be performed differently from the described order. For example, two consecutively described processes may be performed substantially at the same time or performed in an order opposite to the described order. Also, like reference numerals denote like elements.
When an element, such as a layer, is referred to as being "on," "connected to," or "coupled to" another element or layer, it may be directly on, connected to, or coupled to the other element or layer or intervening elements or layers may be present. When, however, an element or layer is referred to as being "directly on," "directly connected to," or "directly coupled to" another element or layer, there are no intervening elements or layers present. To this end, the term "connected" may refer to physical, electrical, and/or fluid connection, with or without intervening elements. Further, the D1-axis, the D2-axis, and the D3-axis are not limited to three axes of a rectangular coordinate system, such as the x, y, and z - axes, and may be interpreted in a broader sense. For example, the D1-axis, the D2-axis, and the D3-axis may be perpendicular to one another, or may represent different directions that are not perpendicular to one another. For the purposes of this disclosure, "at least one of X, Y, and Z" and "at least one selected from the group consisting of X, Y, and Z" may be construed as X only, Y only, Z only, or any combination of two or more of X, Y, and Z, such as, for instance, XYZ, XYY, YZ, and ZZ. As used herein, the term "and/or" includes any and all combinations of one or more of the associated listed items.
Although the terms "first," "second," etc. may be used herein to describe various types of elements, these elements should not be limited by these terms. These terms are used to distinguish one element from another element. Thus, a first element discussed below could be termed a second element without departing from the teachings of the disclosure.
Spatially relative terms, such as "beneath," "below," "under," "lower," "above," "upper," "over," "higher," "side" (e.g., as in "sidewall"), and the like, may be used herein for descriptive purposes, and, thereby, to describe one elements relationship to another element(s) as illustrated in the drawings. Spatially relative terms are intended to encompass different orientations of an apparatus in use, operation, and/or manufacture in addition to the orientation depicted in the drawings. For example, if the apparatus in the drawings is turned over, elements described as "below" or "beneath" other elements or features would then be oriented "above" the other elements or features. Thus, the term "below" can encompass both an orientation of above and below. Furthermore, the apparatus may be otherwise oriented (e.g., rotated

90 degrees or at other orientations), and, as such, the spatially relative descriptors used herein interpreted accordingly.

The terminology used herein is for the purpose of describing particular embodiments and is not intended to be limiting. As used herein, the singular forms, "a," "an," and "the" are intended to include the plural forms as well, unless the context clearly indicates otherwise. Moreover, the terms "comprises," "comprising," "includes," and/or "including," when used in this specification, specify the presence of stated features, integers, steps, operations, elements, components, and/or groups thereof, but do not preclude the presence or addition of one or more other features, integers, steps, operations, elements, components, and/or groups thereof. It is also noted that, as used herein, the terms "substantially," "about," and other similar terms, are used as terms of approximation and not as terms of degree, and, as such, are utilized to account for inherent deviations in measured, calculated, and/or provided values that would be recognized by one of ordinary skill in the art.

Various embodiments are described herein with reference to sectional and/or exploded illustrations that are schematic illustrations of embodiments and/or intermediate structures. As such, variations from the shapes of the illustrations as a result, for example, of manufacturing techniques and/or tolerances, are to be expected. Thus, embodiments disclosed herein should not necessarily be construed as limited to the particular illustrated shapes of regions, but are to include deviations in shapes that result from, for instance, manufacturing. In this manner, regions illustrated in the drawings may be schematic in nature and the shapes of these regions may not reflect actual shapes of regions of a device and, as such, are not necessarily intended to be limiting.

Unless otherwise defined, all terms (including technical and scientific terms) used herein have the same meaning as commonly understood by one of ordinary skill in the art to which this disclosure is a part. Terms, such as those defined in commonly used dictionaries, should be interpreted as having a meaning that is consistent with their meaning in the context of the relevant art and should not be interpreted in an idealized or overly formal sense, unless expressly so defined herein.

An organic light-emitting device includes a first electrode; a second electrode facing the first electrode; and an organic layer between the first electrode and the second electrode, wherein the organic layer includes an emission layer, the emission layer includes a host and a phosphorescent dopant, the host satisfies Equations 1 and 2, and the host and the phosphorescent dopant satisfy Equation 3:

Equation 1

$$S1(H) - T1(H) \geq 0.3 \text{ eV}$$

Equation 2

$$T1(H) \leq 2.7 \text{ eV}$$

Equation 3

$$T1(D) \geq T1(H)$$

wherein, in Equations 1 to 3, T1(H) indicates a triplet energy of the host, S1(H) indicates a singlet energy of the host, and T1(D) indicates a triplet energy of the phosphorescent dopant.

Unless otherwise defined, the triplet energy T1(H) of the host refers to a lowest excited triplet energy of the host. Unless otherwise defined, the singlet energy S1(H) of the host refers to a lowest excited singlet energy of the host. Unless otherwise defined, the triplet energy T1(D) of the phosphorescent dopant refers to a lowest excited triplet energy of the phosphorescent dopant.

In the organic light-emitting device, as the host satisfies Equations 1 and 2, a triplet energy of the host may be very high. Accordingly, $\Delta E_{st}$, i.e., an energy level difference between a singlet energy and the triplet energy of the host, may be very small. For this reason, even at room temperature, reverse inter-system crossing (RISC) from a triplet excited state to a singlet excited state through thermal activation may become possible.

In addition, in the organic light-emitting device, as the host and the phosphorescent dopant satisfy Equation 3, the triplet energy of the host may be equal to or greater than the triplet energy of the phosphorescent dopant. Accordingly, excitons in a triplet state may be used in light emission. Thus, the organic light-emitting device may have a low driving voltage, improved luminescence efficiency, and high external quantum efficiency. According to one or more embodiments, the host may include a heterocyclic compound represented by Formula 1:

Formula 1 $(Ar_1)_{n1}-(L_1)_{m1}-(Ar_2)_{n2}$

wherein, in Formula 1,

$L_1$ is a single bond, a $C_5$-$C_{60}$ (*e.g.* $C_5$-$C_{30}$) carbocyclic group, or a $C_1$-$C_{60}$ (*e.g.* $C_1$-$C_{20}$) heterocyclic group,
n1 and n2 are each independently an integer from 0 to 3, n1 + n2 ≤ 1,
m1 is an integer from 0 to 5, and
$Ar_1$ and $Ar_2$ are each independently a group represented by Formula 1A or Formula 1B:

Formula 1A

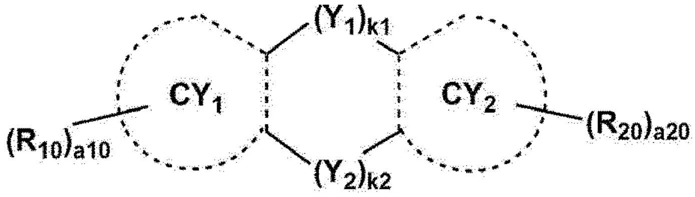

Formula 1B

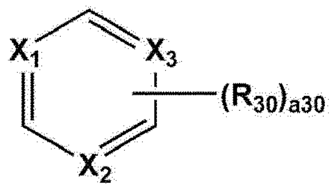

wherein, in Formulae 1A and 1B,

$Y_1$ and $Y_2$ are each independently selected from a single bond, *-O-*', *-S-*', *-C($R_1$)($R_2$)- *', *-N($R_1$)- *', *-Si($R_1$)($R_2$)-*', *-C(=O)-*', *-S(=O)$_2$-*' *-B($R_1$)-*', *-P($R_1$)-*', and *-P(=O)($R_1$)-*',
k1 and k2 are each independently 0 or 1, k1 + k2 ≤ 1,
CY1 and CY2 are each independently a $C_5$-$C_{60}$ (*e.g.* $C_5$-$C_{30}$) carbocyclic group or a $C_1$-$C_{60}$ (*e.g.* $C_1$-$C_{20}$) heterocyclic group,
$X_1$ to $X_3$ are each independently C or N,
in a case where $X_1$ to $X_3$ are each C, at least one selected from $R_{30}$(s) is a cyano group,
$R_1$, $R_2$, $R_{10}$, $R_{20}$, and $R_{30}$ are each independently selected from hydrogen, deuterium, -F, -Cl, -Br, -I, a hydroxyl group, a cyano group, a nitro group, an amidino group, a hydrazino group, a hydrazono group, a substituted or unsubstituted $C_1$-$C_{60}$ (*e.g.* $C_1$-$C_{20}$) alkyl group, a substituted or unsubstituted $C_2$-$C_{60}$ (*e.g.* $C_2$-$C_{20}$) alkenyl group, a substituted or unsubstituted $C_2$-$C_{60}$ (*e.g.* $C_2$-$C_{20}$) alkynyl group, a substituted or unsubstituted $C_1$-$C_{60}$ (*e.g.* $C_1$-$C_{20}$) alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ (*e.g.* $C_6$-$C_{30}$) aryl group, a substituted or unsubstituted $C_6$-$C_{60}$ (*e.g.* $C_6$-$C_{30}$) aryloxy group, a substituted or unsubstituted $C_6$-$C_{60}$ (*e.g.* $C_6$-$C_{30}$) arylthio group, a substituted or unsubstituted $C_1$-$C_{60}$ (*e.g.* $C_1$-$C_{20}$) heteroaryl group, a substituted or unsubstituted $C_1$-$C_{60}$ (*e.g.* $C_1$-$C_{20}$) heteroaryloxy group, a substituted or unsubstituted $C_1$-$C_{60}$ (*e.g.* $C_1$-$C_{20}$) heteroarylthio group, a substituted or unsubstituted monovalent non-aromatic condensed polycyclic group, a substituted or unsubstituted monovalent non-aromatic condensed heteropolycyclic group,-Si($Q_1$)($Q_2$)($Q_3$), -N($Q_1$)($Q_2$), -B($Q_1$)($Q_2$), -C(=O)($Q_1$), -S(=O)$_2$($Q_1$), and -P(=O)($Q_1$)($Q_2$),
a10 and a20 are each independently an integer from 1 to 10,
a30 is an integer from 1 to 6,
$R_1$ and $R_2$ are optionally bound to form a substituted or unsubstituted $C_5$-$C_{60}$ (*e.g.* $C_5$-$C_{30}$) carbocyclic group or a substituted or unsubstituted $C_1$-$C_{60}$ (*e.g.* $C_1$-$C_{20}$) heterocyclic group,
$R_{10}$(s) and $R_{20}$(s) are optionally bound to at least one selected from $R_{10}$(s) and $R_{20}$(s) to form a substituted or unsubstituted $C_5$-$C_{60}$ (*e.g.* $C_5$-$C_{30}$) carbocyclic group or a substituted or unsubstituted $C_1$-$C_{60}$ (*e.g.* $C_1$-$C_{20}$) heterocyclic group,
when a30 is 2 or greater, at least two $R_{30}$(s) are optionally bound to form a substituted or unsubstituted $C_5$-$C_{60}$

(*e.g.* $C_5$-$C_{30}$) carbocyclic group or a substituted or unsubstituted $C_1$-$C_{60}$ (*e.g.* $C_1$-$C_{20}$) heterocyclic group,

at least one selected from $R_{10}$ and $R_{20}$ in Formula 1A is a binding site to $L_1$, An, or $Ar_2$,

at least one selected from $R_{30}$(s) in Formula 1B is a binding site to $L_1$, An, or $Ar_2$, and

at least one substituent of the substituted $C_5$-$C_{60}$ (*e.g.* $C_5$-$C_{30}$) carbocyclic group, the substituted $C_1$-$C_{60}$ (*e.g.* $C_1$-$C_{20}$) heterocyclic group, the substituted $C_1$-$C_{60}$ (*e.g.* $C_1$-$C_{20}$) alkyl group, the substituted $C_2$-$C_{60}$ (*e.g.* $C_2$-$C_{20}$) alkenyl group, the substituted $C_2$-$C_{60}$ (*e.g.* $C_2$-$C_{20}$) alkynyl group, the substituted $C_1$-$C_{60}$ (*e.g.* $C_1$-$C_{20}$) alkoxy group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_1$-$C_{10}$ heterocycloalkenyl group, the substituted $C_6$-$C_{60}$ (*e.g.* $C_6$-$C_{30}$) aryl group, the substituted $C_6$-$C_{60}$ (*e.g.* $C_6$-$C_{30}$) aryloxy group, the substituted $C_6$-$C_{60}$ (*e.g.* $C_6$-$C_{30}$) arylthio group, the substituted $C_1$-$C_{60}$ (*e.g.* $C_1$-$C_{20}$) heteroaryl group, the substituted $C_1$-$C_{60}$ (*e.g.* $C_1$-$C_{20}$) heteroaryloxy group, the substituted $C_1$-$C_{60}$ (*e.g.* $C_1$-$C_{20}$) heteroarylthio group, the substituted monovalent non-aromatic condensed polycyclic group, and the substituted monovalent non-aromatic condensed heteropolycyclic group is selected from:

deuterium, -F, -Cl, -Br, -I, a hydroxyl group, a cyano group, a nitro group, an amidino group, a hydrazino group, a hydrazono group, a $C_1$-$C_{60}$ (*e.g.* $C_1$-$C_{20}$) alkyl group, a C2-$C_{60}$ (*e.g.* $C_2$-$C_{20}$) alkenyl group, a $C_2$-$C_{60}$ (*e.g.* $C_2$-$C_{20}$) alkynyl group, and a $C_1$-$C_{60}$ (*e.g.* $C_1$-$C_{20}$) alkoxy group;

a $C_1$-$C_{60}$ (*e.g.* $C_1$-$C_{20}$) alkyl group, a $C_2$-$C_{60}$ (*e.g.* $C_2$-$C_{20}$) alkenyl group, a $C_2$-$C_{60}$ (*e.g.* $C_2$-$C_{20}$) alkynyl group, and a $C_1$-$C_{60}$ (*e.g.* $C_1$-$C_{20}$) alkoxy group, each substituted with at least one selected from deuterium, -F, -Cl, -Br, -I, a hydroxyl group, a cyano group, a nitro group, an amidino group, a hydrazino group, a hydrazono group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ (*e.g.* $C_6$-$C_{30}$) aryl group, a $C_6$-$C_{60}$ (*e.g.* $C_6$-$C_{30}$) aryloxy group, a $C_6$-$C_{60}$ (*e.g.* $C_6$-$C_{30}$) arylthio group, a $C_1$-$C_{60}$ (*e.g.* $C_1$-$C_{20}$) heteroaryl group, a monovalent non-aromatic condensed polycyclic group, a monovalent non-aromatic condensed heteropolycyclic group,-$Si(Q_{11})(Q_{12})(Q_{13})$, -$N(Q_{11})(Q_{12})$, -$B(Q_{11})(Q_{12})$, -$C(=O)(Q_{11})$, -$S(=O)_2(Q_{11})$, and -$P(=O)(Q_{11})(Q_{12})$;

a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ (*e.g.* $C_6$-$C_{30}$) aryl group, a $C_6$-$C_{60}$ (*e.g.* $C_6$-$C_{30}$) aryloxy group, a $C_6$-$C_{60}$ (*e.g.* $C_6$-$C_{30}$) arylthio group, a $C_1$-$C_{60}$ (*e.g.* $C_1$-$C_{20}$) heteroaryl group, a monovalent non-aromatic condensed polycyclic group, and a monovalent non-aromatic condensed heteropolycyclic group;

a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ (*e.g.* $C_6$-$C_{30}$) aryl group, a $C_6$-$C_{60}$ (*e.g.* $C_6$-$C_{30}$) aryloxy group, a $C_6$-$C_{60}$ (*e.g.* $C_6$-$C_{30}$) arylthio group, a $C_1$-$C_{60}$ (*e.g.* $C_1$-$C_{20}$) heteroaryl group, a monovalent non-aromatic condensed polycyclic group, and a monovalent non-aromatic condensed heteropolycyclic group, each substituted with at least one selected from deuterium, -F, -Cl, -Br, -I, a hydroxyl group, a cyano group, a nitro group, an amidino group, a hydrazino group, a hydrazono group, a $C_1$-$C_{60}$ (*e.g.* $C_1$-$C_{20}$) alkyl group, a $C_2$-$C_{60}$ (*e.g.* $C_2$-$C_{20}$) alkenyl group, a $C_2$-$C_{60}$ (*e.g.* $C_2$-$C_{20}$) alkynyl group, a $C_1$-$C_{60}$ (*e.g.* $C_1$-$C_{20}$) alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ (*e.g.* $C_6$-$C_{30}$) aryl group, a $C_6$-$C_{60}$ (*e.g.* $C_6$-$C_{30}$) aryloxy group, a $C_6$-$C_{60}$ (*e.g.* $C_6$-$C_{30}$) arylthio group, a $C_1$-$C_{60}$ (*e.g.* $C_1$-$C_{20}$) heteroaryl group, a monovalent non-aromatic condensed polycyclic group, a monovalent non-aromatic condensed heteropolycyclic group, -$Si(Q_{21})(Q_{22})(Q_{23})$, -$N(Q_{21})(Q_{22})$, -$B(Q_{21})(Q_{22})$, -$C(=O)(Q_{21})$, -$S(=O)_2(Q_{21})$, and-$P(=O)(Q_{21})(Q_{22})$; and

-$Si(Q_{31})(Q_{32})(Q_{33})$, -$N(Q_{31})(Q_{32})$, -$B(Q_{31})(Q_{32})$, -$C(=O)(Q_{31})$, -$S(=O)_2(Q_{31})$, and-$P(=O)(Q_{31})(Q_{32})$,

wherein $Q_1$ to $Q_3$, $Q_{11}$ to $Q_{13}$, $Q_{21}$ to $Q_{23}$, and $Q_{31}$ to $Q_{33}$ are each independently selected from hydrogen, deuterium, -F, -Cl, -Br, -I, a hydroxyl group, a cyano group, a nitro group, an amidino group, a hydrazino group, a hydrazono group, a $C_1$-$C_{60}$ (*e.g.* $C_1$-$C_{20}$) alkyl group, a $C_2$-$C_{60}$ (*e.g.* $C_2$-$C_{20}$) alkenyl group, a $C_2$-$C_{60}$ (*e.g.* $C_2$-$C_{20}$) alkynyl group, a $C_1$-$C_{60}$ (*e.g.* $C_1$-$C_{20}$) alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ (*e.g.* $C_6$-$C_{30}$) aryl group, a $C_1$-$C_{60}$ (*e.g.* $C_1$-$C_{20}$) heteroaryl group, a monovalent non-aromatic condensed polycyclic group, a monovalent non-aromatic condensed heteropolycyclic group, a biphenyl group, and a terphenyl group.

In some embodiments, when m1 is 0, n1 + n2 is at least 2. For example, when m1 is 0, n1 + n2 is 2. In some embodiments, when m1 is 0, n1 is 1 and n2 is 1. In some embodiments, when n1 is 0 and n2 is 1, m1 is not 0. In some embodiments, when n1 is 1 and n2 is 0, m1 is not 0. In some embodiments, when n1 is 0 and n2 is 1, m1 is 1, 2 or 3. In some embodiments, when n1 is 1 and n2 is 0, m1 is 1, 2 or 3. In some embodiments, when n1 is 0 and n2 is 1, m1 is 1. In some embodiments, when n1 is 1 and n2 is 0, m1 is 1.

In some embodiments, in Formula 1, $n1 + n2 \leq 2$.

In some embodiments, in Formula 1A, k1 and k2 may each be 1.

In some embodiments, in Formula 1A, k1 may be 1, and k2 may be 0.

In some embodiments, in Formula 1A, k1 may be 0, and k2 may be 1.

In Formula 1A, when k1 is 0, $-(Y_1)_{k1}-$ is not present, and when k2 is 0, -(Y2)k2- is not present.

In some embodiments, in Formula 1, An and $Ar_2$ may each independently be a group represented by at least one of Formulae 1(1) to 1(4):

Formula 1(1)

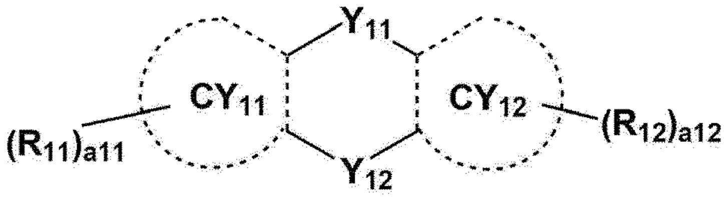

Formula 1(2)

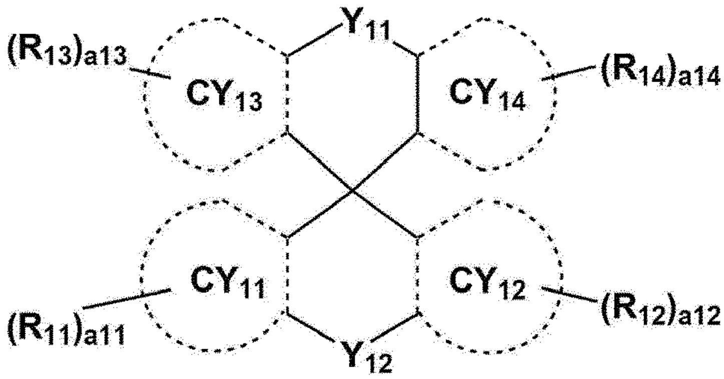

Formula 1(3)

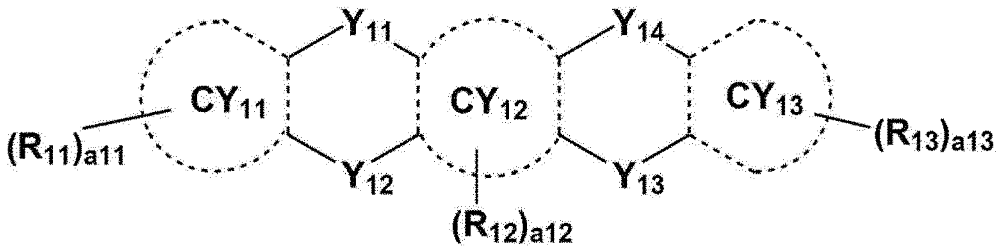

Formula 1(4)

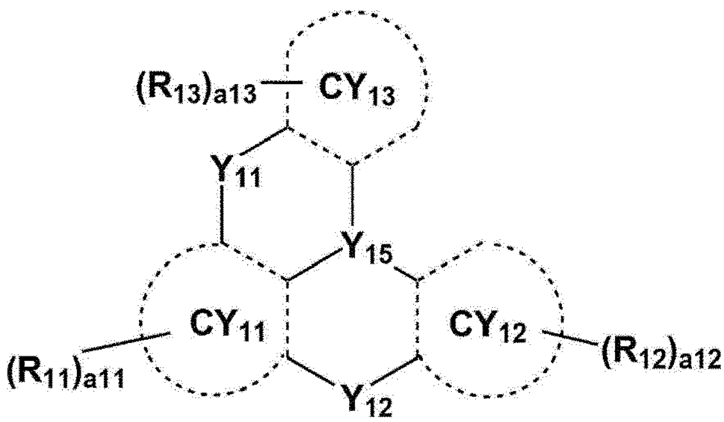

wherein, in Formulae 1(1) to 1(4),

$Y_{11}$ to $Y_{14}$ are each independently selected from a single bond, -O-, -S-,-C($R_{15}$)($R_{16}$)-, -N($R_{15}$)-, -Si($R_{15}$)($R_{16}$)-, -C(=O)-, -S(=O)$_2$-, -B($R_{15}$)-, -P($R_{15}$)-, and-P(=O)($R_{15}$)($R_{16}$)-,
$Y_{15}$ is N, B, or P,
$CY_{11}$ to $CY_{14}$ are each independently selected from a benzene group, a naphthalene group, a carbazole group, a dibenzofuran group, a dibenzothiophene group, and a dibenzosilole group,
$R_{11}$ to $R_{16}$ are each independently a binding site to $L_1$, An, or $Ar_2$, and be selected from hydrogen, deuterium, -F, -Cl, -Br, -I, a hydroxyl group, a cyano group, a nitro group, an amidino group, a hydrazino group, a hydrazono group, a substituted or unsubstituted $C_1$-$C_{60}$ (*e.g.* $C_1$-$C_{20}$) alkyl group, a substituted or unsubstituted $C_2$-$C_{60}$ (*e.g.* $C_2$-$C_{20}$) alkenyl group, a substituted or unsubstituted $C_2$-$C_{60}$ (*e.g.* $C_2$-$C_{20}$) alkynyl group, a substituted or unsubstituted $C_1$-$C_{60}$ (*e.g.* $C_1$-$C_{20}$) alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ (*e.g.* $C_6$-$C_{30}$) aryl group, a substituted or unsubstituted $C_6$-$C_{60}$ (*e.g.* $C_6$-$C_{30}$) aryloxy group, a substituted or unsubstituted $C_6$-$C_{60}$ (*e.g.* $C_6$-$C_{30}$) arylthio group, a substituted or unsubstituted $C_1$-$C_{60}$ (*e.g.* $C_1$-$C_{20}$) heteroaryl group, a substituted or unsubstituted $C_1$-$C_{60}$ (*e.g.* $C_1$-$C_{20}$) heteroaryloxy group, a substituted or unsubstituted $C_1$-$C_{60}$ (*e.g.* $C_1$-$C_{20}$) heteroarylthio group, a substituted or unsubstituted monovalent non-aromatic condensed polycyclic group, a substituted or unsubstituted monovalent non-aromatic condensed heteropolycyclic group, -Si($Q_1$)($Q_2$)($Q_3$), -N($Q_1$)($Q_2$), -B($Q_1$)($Q_2$), -C(=O)($Q_1$), -S(=O)$_2$($Q_1$), and -P(=O)($Q_1$)($Q_2$),
at least one selected from $R_{11}$ to $R_{16}$ is a binding site to $L_1$, An, or $Ar_2$,
wherein $Q_1$ to $Q_3$ are each independently selected from hydrogen, deuterium, -F,-Cl, -Br, -I, a hydroxyl group, a cyano group, a nitro group, an amidino group, a hydrazino group, a hydrazono group, a $C_1$-$C_{60}$ (*e.g.* $C_1$-$C_{20}$) alkyl group, a $C_2$-$C_{60}$ (*e.g.* $C_2$-$C_{20}$) alkenyl group, a $C_2$-$C_{60}$ (*e.g.* $C_2$-$C_{20}$) alkynyl group, a $C_1$-$C_{60}$ (*e.g.* $C_1$-$C_{20}$) alkoxy group, a phenyl group, a biphenyl group, a terphenyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a pyridinyl group, a pyrazinyl group, a pyrimidinyl group, a quinolinyl group, an isoquinolinyl group, a benzoquinolinyl group, a naphthyridinyl group, a quinoxalinyl group, a quinazolinyl group, a carbazolyl group, a phenanthridinyl group, an acridinyl group, a phenanthrolinyl group, a phenazinyl group, a triazinyl group, a dibenzofuranyl group, a dibenzothiophenyl group, a biphenyl group, and a terphenyl group, and
all to a14 are each independently an integer from 1 to 6.

In some embodiments, in Formula 1, $L_1$ may be a single bond, a $C_5$-$C_{30}$ carbocyclic group, or a $C_1$-$C_{20}$ heterocyclic group.
In some embodiments, in Formula 1, $L_1$ may be selected from a single bond, a substituted or unsubstituted $C_3$-$C_{10}$ cycloalkylene group, a substituted or unsubstituted $C_1$-$C_{10}$ heterocycloalkylene group, a substituted or unsubstituted $C_3$-$C_{10}$ cycloalkenylene group, a substituted or unsubstituted $C_1$-$C_{10}$ heterocycloalkenylene group, a substituted or unsubstituted $C_6$-$C_{60}$ (*e.g.* $C_6$-$C_{30}$) arylene group, a substituted or unsubstituted $C_1$-$C_{60}$ (*e.g.* $C_1$-$C_{20}$) heteroarylene group, a substituted or unsubstituted divalent non-aromatic condensed polycyclic group, and a substituted or unsubstituted divalent non-aromatic condensed heteropolycyclic group.
In some embodiments, $L_1$ may be selected from

a single bond, a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an imidazopyridinylene group, an imidazopyrimidinylene group, and an azacarbazolylene group; and
a phenylene group, a naphthylene group, a fluorenylene group, a spiro-bifluorenylene group, a benzofluorenylene

group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an imidazopyridinylene group, an imidazopyrimidinylene group, and an azacarbazolylene group, each substituted with at least one selected from deuterium, -F, -Cl, -Br, -I, a hydroxyl group, a cyano group, a nitro group, an amidino group, a hydrazino group, a hydrazono group, a $C_1$-$C_{20}$ (*e.g.* $C_1$-$C_{10}$) alkyl group, a $C_1$-$C_{20}$ (*e.g.* $C_1$-$C_{10}$) alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, an azacarbazolyl group, $-Si(Q_{31})(Q_{32})(Q_{33})$, $-N(Q_{31})(Q_{32})$, $-B(Q_{31})(Q_{32})$, $-C(=O)(Q_{31})$,$-S(=O)_2(Q_{31})$, and $-P(=O)(Q_{31})(Q_{32})$,

wherein $Q_{31}$ to $Q_{33}$ may each independently be selected from a $C_1$-$C_{10}$ alkyl group, a $C_1$-$C_{10}$ alkoxy group, a phenyl group, a phenyl group substituted with a $C_1$-$C_{10}$ alkyl group, a biphenyl group, a terphenyl group, and a naphthyl group.

In some embodiments, $L_1$ may be a single bond or a group represented by one of Formulae 3-1 to 3-35:

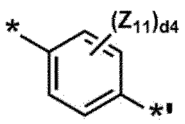
3-1

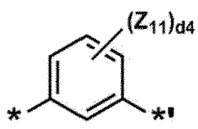
3-2

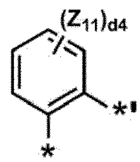
3-3

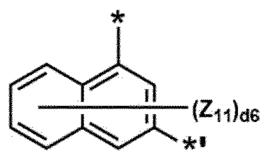
3-4

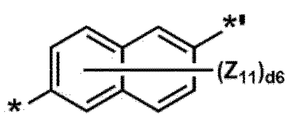
3-5

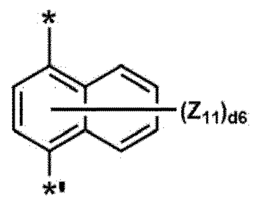
3-6

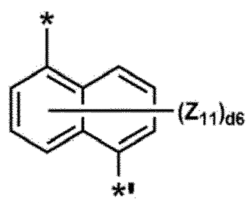
3-7

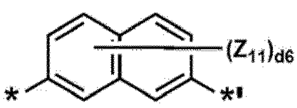
3-8

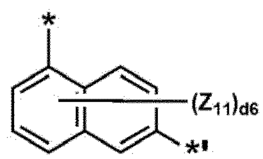
3-9

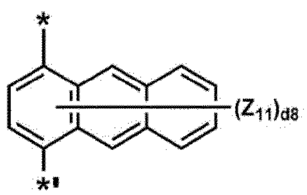
3-10

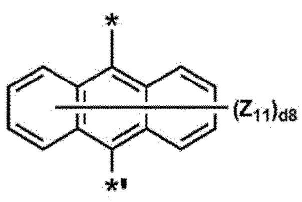
3-11

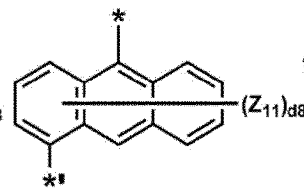
3-12

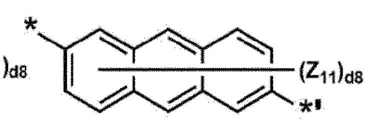
3-13

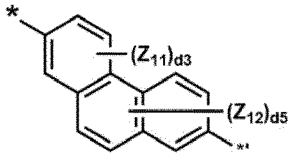
3-14

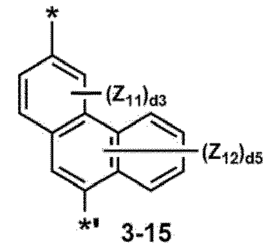
3-15

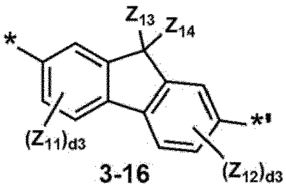
3-16

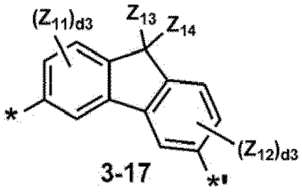
3-17

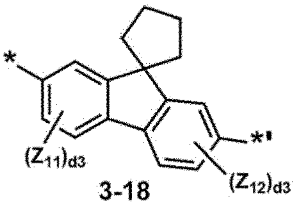
3-18

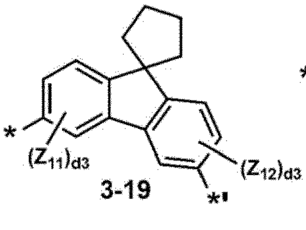
3-19

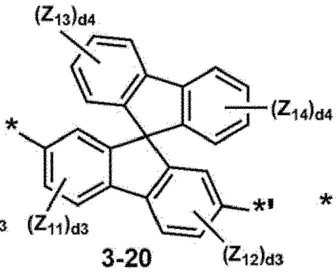
3-20

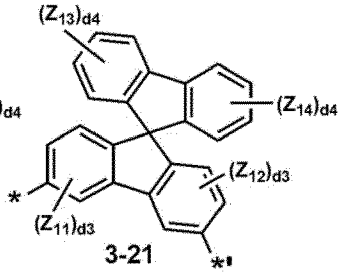
3-21

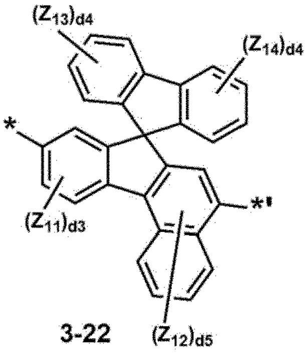
3-22

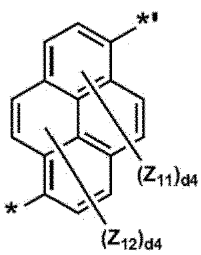
3-23

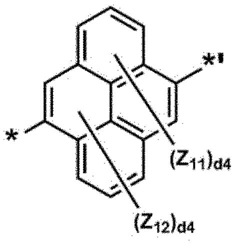
3-24

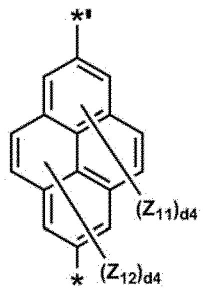
3-25

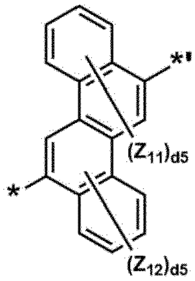
3-26

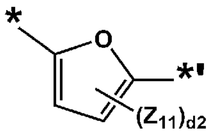
3-27

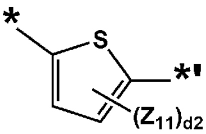
3-28

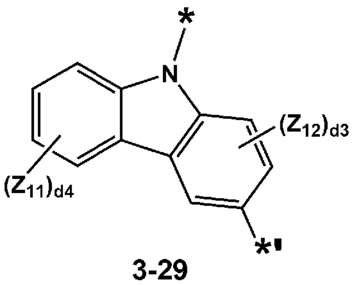
3-29

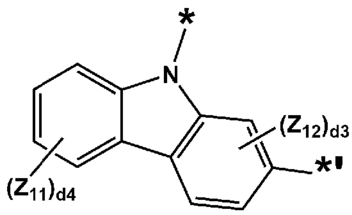
3-30

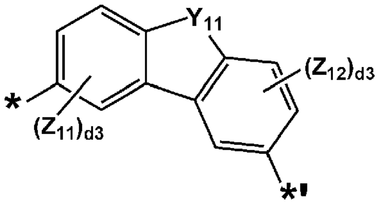
3-31

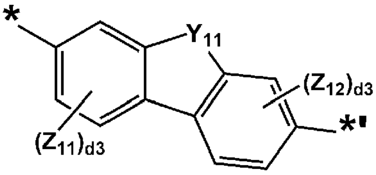
3-32

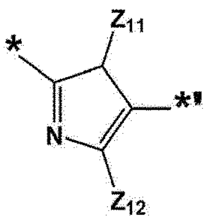

3-33

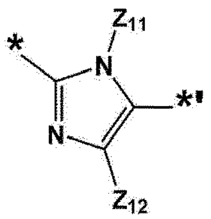

3-34

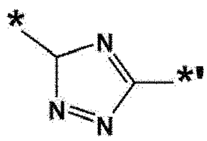

3-35

wherein, in Formulae 3-1 to 3-35,

$Y_{11}$ is *-O-*', *-S-*', or *-N($Z_{15}$)-*',

$Z_{11}$ to $Z_{15}$ are each independently selected from hydrogen, deuterium, -F, -Cl, -Br, -I, a hydroxyl group, a cyano group, a nitro group, an amino group, an amidino group, a hydrazino group, a hydrazono group, a $C_1$-$C_{20}$ (*e.g.* $C_1$-$C_{10}$) alkyl group, a $C_1$-$C_{20}$ (*e.g.* $C_1$-$C_{10}$) alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, a dibenzofuranyl group, a dibenzothiophenyl group, a triazinyl group, a benzimidazolyl group, a phenanthrolinyl group, and -Si($Q_{31}$)($Q_{32}$)($Q_{33}$),

wherein $Q_{31}$ to $Q_{33}$ are each independently selected from a $C_1$-$C_{10}$ alkyl group, a $C_1$-$C_{10}$ alkoxy group, a phenyl group, a biphenyl group, a terphenyl group, and a naphthyl group,

d2 is an integer from 0 to 2,

d3 is an integer from 0 to 3,

d4 is an integer from 0 to 4,

d5 is an integer from 0 to 5,

d6 is an integer from 0 to 6,

d8 is an integer from 0 to 8, and

* and *' each indicate a binding site to an adjacent atom.

In some embodiments, in Formula 1, at least one selected from An and $Ar_2$ may be represented by one of Formulae 4-1 to 4-34:

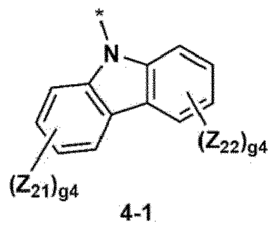

4-1

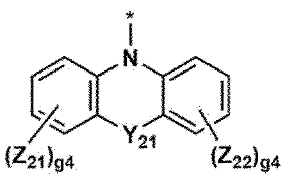

4-2

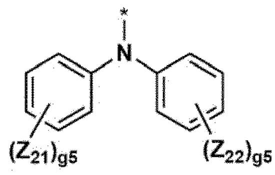

4-3

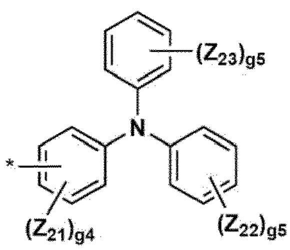

4-4

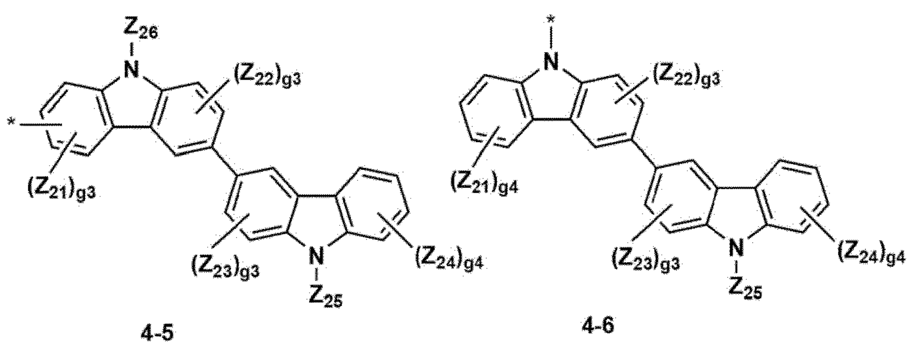

4-5

4-6

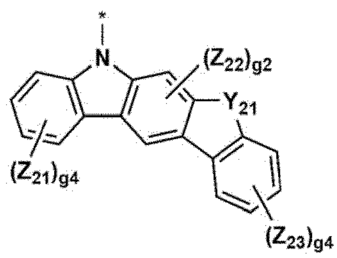

4-7

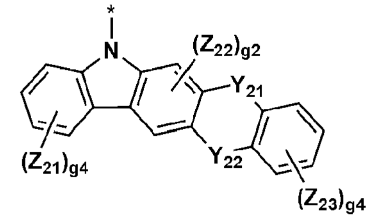

4-8

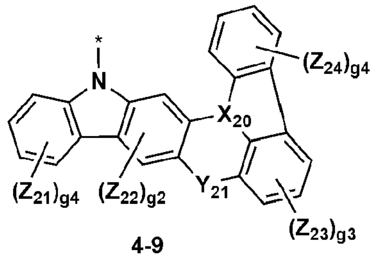

4-9

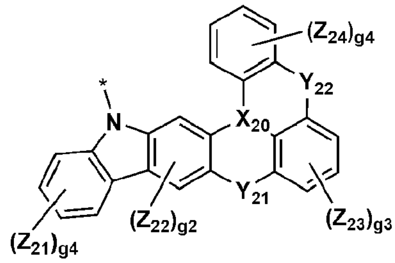

4-10

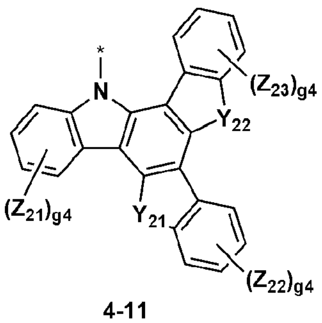

4-11

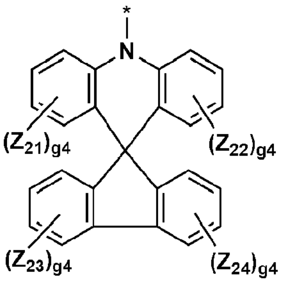

4-12

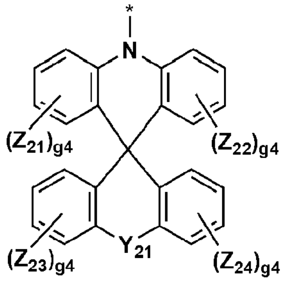

4-13

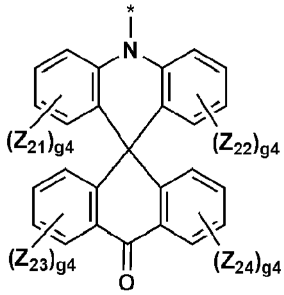

4-14

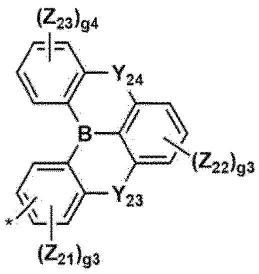

4-15

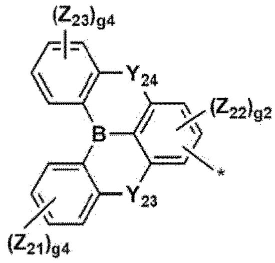

4-16

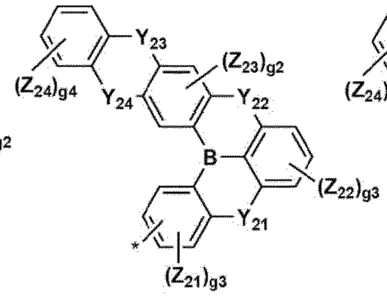

4-17

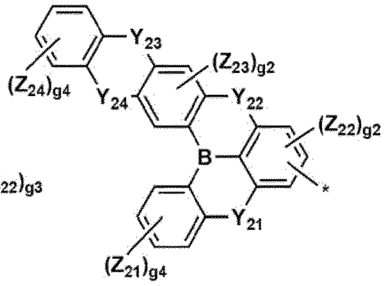

4-18

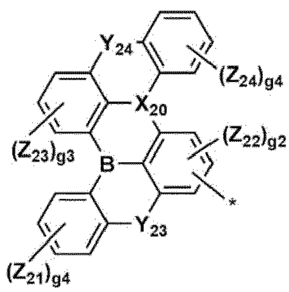

4-19

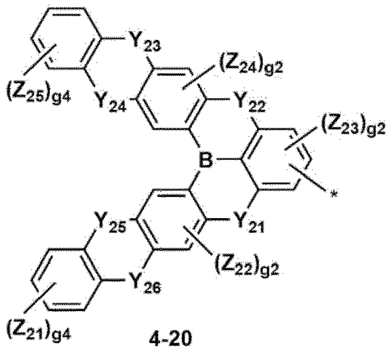

4-20

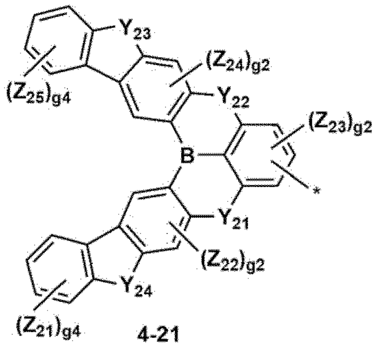

4-21

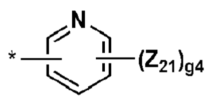

4-22

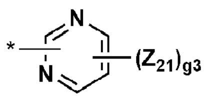

4-23

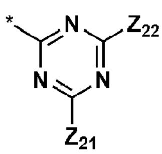

4-24

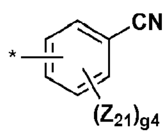

4-25

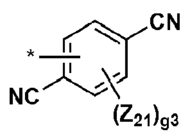

4-26

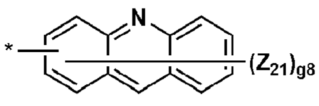

4-27

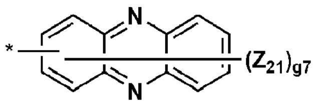

4-28

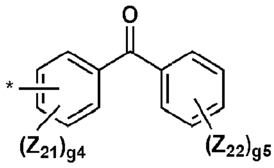

4-29

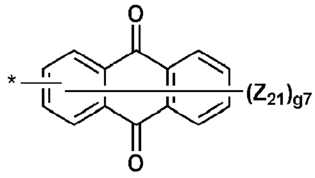

4-30

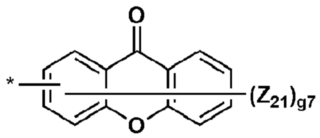

4-31

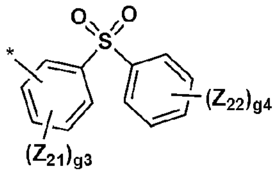

4-32

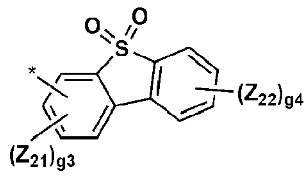

4-33

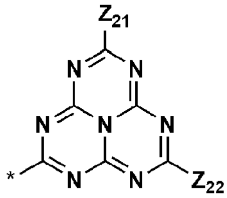

4-34

wherein, in Formulae 4-1 to 4-34,

$X_{20}$ is N, B, or P,

$Y_{21}$ and $Y_{22}$ are each independently O, S, $C(Z_{26})(Z_{27})$, $N(Z_{26})$, or $Si(Z_{26})(Z_{27})$,

$Y_{23}$ to $Y_{26}$ are each independently a single bond, O, S, $C(Z_{28})(Z_{29})$, $N(Z_{28})$, or $Si(Z_{28})(Z_{29})$,

$Z_{21}$ to $Z_{29}$ are each independently selected from hydrogen, deuterium, -F, -Cl, -Br, I, a hydroxyl group, a cyano group, a nitro group, an amino group, an amidino group, a hydrazino group, a hydrazono group, a $C_1$-$C_{20}$ (*e.g.* $C_1$-$C_{10}$) alkyl group, a $C_1$-$C_{20}$ (*e.g.* $C_1$-$C_{10}$) alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, a dibenzofuranyl group, a dibenzothiophenyl group, a triazinyl group, a benzimidazolyl group, a phenanthrolinyl group, and $-Si(Q_{31})(Q_{32})(Q_{33})$,-

wherein $Q_{31}$ to $Q_{33}$ are each independently selected from a $C_1$-$C_{10}$ alkyl group, a $C_1$-$C_{10}$ alkoxy group, a phenyl group, a biphenyl group, a terphenyl group, and a naphthyl group,

g2 is 1 or 2,

g3 is an integer from 1 to 3,

g4 is an integer from 1 to 4,

g5 is an integer from 1 to 5,

g7 is an integer from 1 to 7,

g8 is an integer from 1 to 8, and

* indicates a binding site to an adjacent atom.

In some embodiments, in Formula 1, at least one selected from $Ar_1$ and $Ar_2$ may be represented by one of Formulae 10-1 to 10-45:

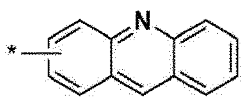

10-1

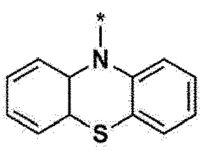

10-2

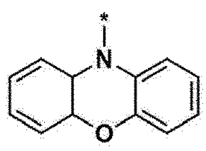

10-3

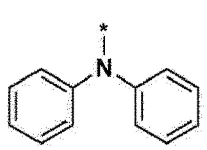

10-4

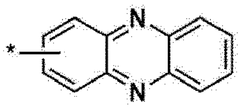
10-5

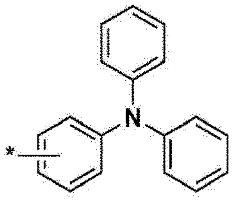
10-6

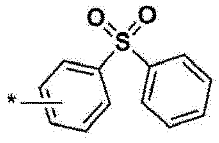
10-7

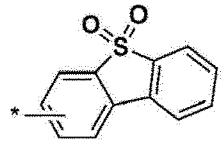
10-8

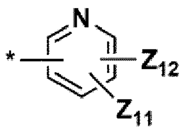
10-9

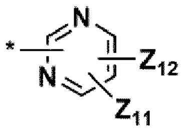
10-10

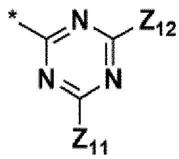
10-11

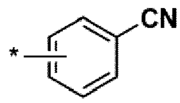
10-12

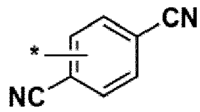
10-13

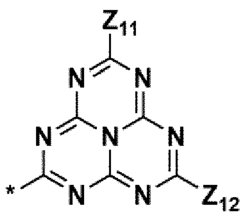
10-14

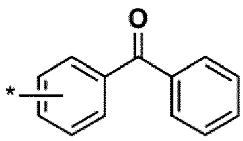
10-15

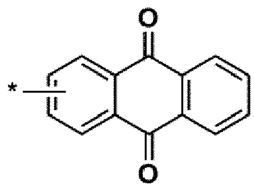
10-16

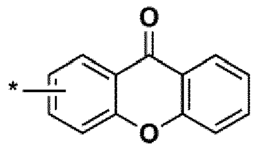
10-17

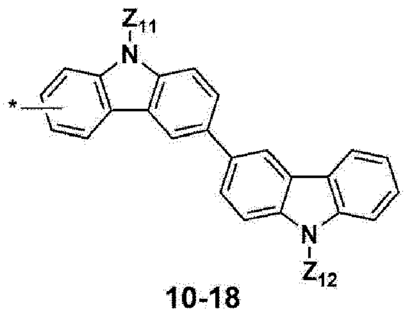
10-18

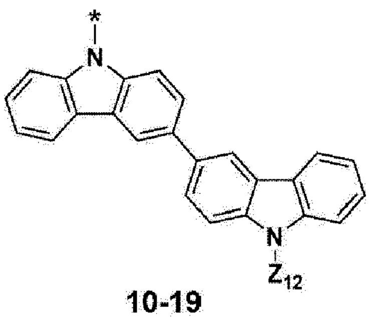
10-19

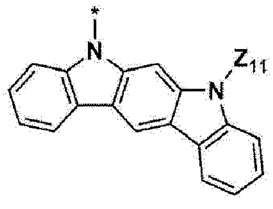
10-20

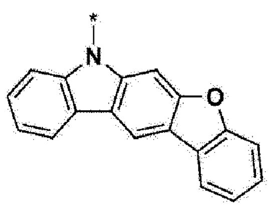
10-21

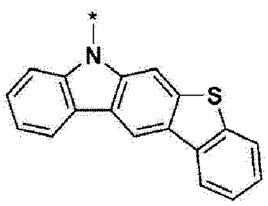
10-22

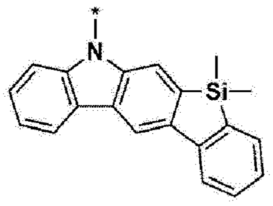

10-23

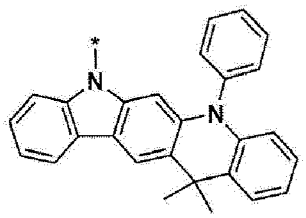

10-24

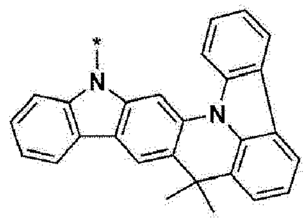

10-25

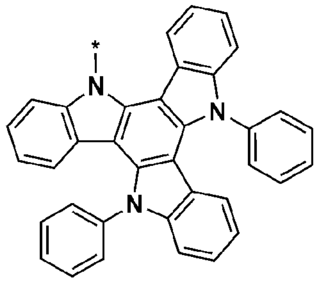

10-26

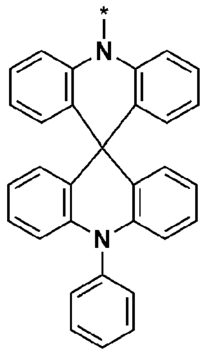

10-27

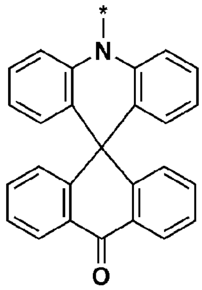

10-28

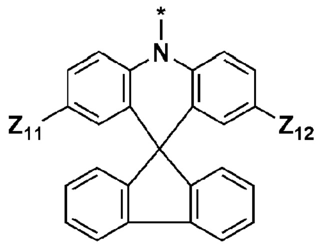

10-29

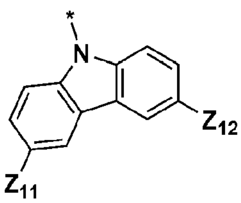

10-30

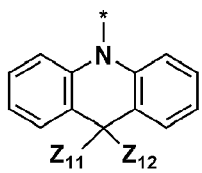

10-31

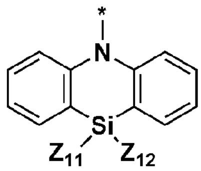

10-32

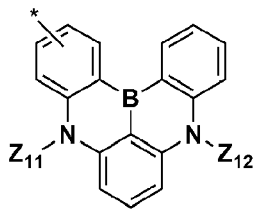

10-33

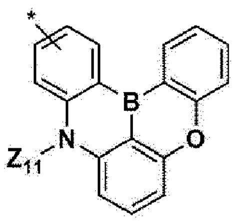

10-34

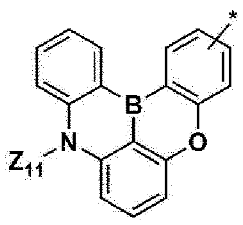

10-35

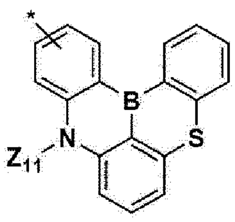

10-36

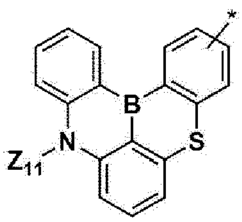

10-37

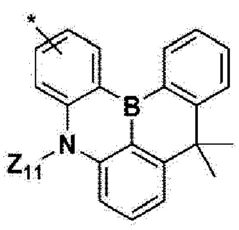

**10-38**

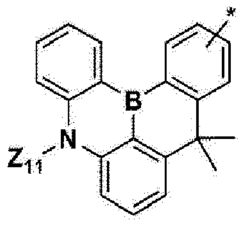

**10-39**

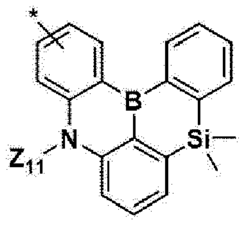

**10-40**

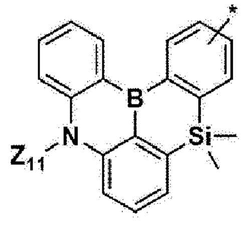

**10-41**

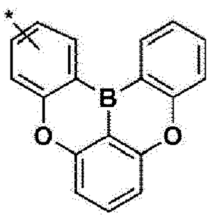

**10-42**

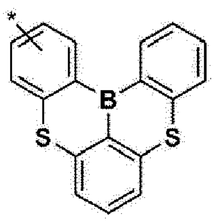

**10-43**

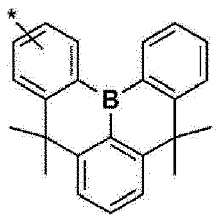

**10-44**

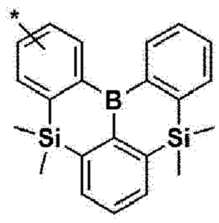

**10-45**

wherein, in Formulae 10-1 to 10-45,

$Z_{11}$ and $Z_{12}$ are each independently selected from hydrogen, deuterium, -F, -Cl, - Br, -I, a hydroxyl group, a cyano group, a nitro group, an amino group, an amidino group, a hydrazino group, a hydrazono group, a $C_1$-$C_{20}$ (*e.g.* $C_1$-$C_{20}$ (*e.g.* $C_1$-$C_{10}$) alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, a dibenzofuranyl group, a dibenzothiophenyl group, a triazinyl group, a benzimidazolyl group, a phenanthrolinyl group, and -Si($Q_{31}$)($Q_{32}$)($Q_{33}$),

wherein $Q_{31}$ to $Q_{33}$ are each independently selected from a $C_1$-$C_{10}$ alkyl group, a $C_1$-$C_{10}$ alkoxy group, a phenyl group, a biphenyl group, a terphenyl group, and a naphthyl group, and

* indicates a binding site to an adjacent atom.

In some embodiments, $R_1$, $R_2$, $R_{10}$, $R_{20}$, and $R_{30}$ may each independently be selected from

hydrogen, deuterium, -F, -Cl, -Br, -I, a methyl group, an ethyl group, an n-propyl group, an iso-propyl group, an n-butyl group, a sec-butyl group, an iso-butyl group, a tert-butyl group, an ethenyl group, a propenyl group, a butenyl group, a methoxy group, an ethoxy group, an n-propoxy group, an iso-propoxy group, an n-butoxy group, a sec-butoxy group, an iso-butoxy group, and a tert-butoxy group;

a methyl group, an ethyl group, an n-propyl group, an iso-propyl group, an n-butyl group, a sec-butyl group, an iso-butyl group, a tert-butyl group, a methoxy group, an ethoxy group, an n-propoxy group, an iso-propoxy group, an n-butoxy group, a sec-butoxy group, an iso-butoxy group, and a tert-butoxy group, each substituted with at least one selected from deuterium, -F, -Cl, -Br, -I, a cyano group, a phenyl group, and a biphenyl group; and

a cyclopentyl group, a cyclohexyl group, a cycloheptyl group, a cyclopentenyl group, a cyclohexenyl group, a phenyl group, a biphenyl group, a terphenyl group, a pentalenyl group, an indenyl group, a naphthyl group, an azulenyl group, an indacenyl group, an acenaphthenyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a perylenyl group, a pentacenyl group, a pyrrolyl group, a thiophenyl group, a furanyl group, a silolyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an indolyl group, an isoindolyl group, an indazolyl group, a purinyl group, a quinolinyl group, an isoquinolinyl group, a benzoquinolinyl group, a benzoisoquinolinyl group, a phthalazinyl group, a naphthyridinyl group, a quinoxalinyl group, a benzoquinoxalinyl group, a quinazolinyl group, a benzoquinazolinyl group,

a cinnolinyl group, a phenanthridinyl group, an acridinyl group, a phenanthrolinyl group, a phenazinyl group, a benzimidazolyl group, a benzofuranyl group, a benzothiophenyl group, a benzosilolyl group, a benzothiazolyl group, a benzoisothiazolyl group, a benzoxazolyl group, a benzoisoxazolyl group, a triazolyl group, a tetrazolyl group, a thiadiazolyl group, an oxadiazolyl group, a triazinyl group, a carbazolyl group, a dibenzofuranyl group, a dibenzothiophenyl group, a dibenzosilolyl group, a benzocarbazolyl group, a naphthobenzofuranyl group, a naphthobenzothiophenyl group, a naphthobenzosilolyl group, a dibenzocarbazolyl group, a dinaphthofuranyl group, a dinaphthothiophenyl group, a dinaphthosilolyl group, an imidazopyridinyl group, an imidazopyrimidinyl group, an oxazolopyridinyl group, a thiazolopyridinyl group, a benzonaphthyridinyl group, an azafluorenyl group, an azaspiro-bifluorenyl group, an azacarbazolyl group, an azadibenzofuranyl group, an azadibenzothiophenyl group, an azadibenzosilolyl group, an indenopyrrolyl group, an indolopyrrolyl group, an indenocarbazolyl group, and an indolocarbazolyl group;

a cyclopentyl group, a cyclohexyl group, a cycloheptyl group, a cyclopentenyl group, a cyclohexenyl group, a phenyl group, a biphenyl group, a terphenyl group, a pentalenyl group, an indenyl group, a naphthyl group, an azulenyl group, an indacenyl group, an acenaphthenyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a perylenyl group, a pentacenyl group, a pyrrolyl group, a thiophenyl group, a furanyl group, a silolyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an indolyl group, an isoindolyl group, an indazolyl group, a purinyl group, a quinolinyl group, an isoquinolinyl group, a benzoquinolinyl group, a benzoisoquinolinyl group, a phthalazinyl group, a naphthyridinyl group, a quinoxalinyl group, a benzoquinoxalinyl group, a quinazolinyl group, a benzoquinazolinyl group, a cinnolinyl group, a phenanthridinyl group, an acridinyl group, a phenanthrolinyl group, a phenazinyl group, a benzimidazolyl group, a benzofuranyl group, a benzothiophenyl group, a benzosilolyl group, a benzothiazolyl group, a benzoisothiazolyl group, a benzoxazolyl group, a benzoisoxazolyl group, a triazolyl group, a tetrazolyl group, a thiadiazolyl group, an oxadiazolyl group, a triazinyl group, a carbazolyl group, a dibenzofuranyl group, a dibenzothiophenyl group, a dibenzosilolyl group, a benzocarbazolyl group, a naphthobenzofuranyl group, a naphthobenzothiophenyl group, a naphthobenzosilolyl group, a dibenzocarbazolyl group, a dinaphthofuranyl group, a dinaphthothiophenyl group, a dinaphthosilolyl group, an imidazopyridinyl group, an imidazopyrimidinyl group, an oxazolopyridinyl group, a thiazolopyridinyl group, a benzonaphthyridinyl group, an azafluorenyl group, an azaspiro-bifluorenyl group, an azacarbazolyl group, an azadibenzofuranyl group, an azadibenzothiophenyl group, an azadibenzosilolyl group, an indenopyrrolyl group, an indolopyrrolyl group, an indenocarbazolyl group, and an indolocarbazolyl group, each substituted with at least one selected from deuterium, -F, -Cl, -Br, -I, a cyano group, a methyl group, an ethyl group, an n-propyl group, an iso-propyl group, an n-butyl group, a sec-butyl group, iso-butyl group, a tert-butyl group, a methoxy group, an ethoxy group, an n-propoxy group, an iso-propoxy group, an n-butoxy group, a sec-butoxy group, an iso-butoxy group, a tert-butoxy group, a cyclopentyl group, a cyclohexyl group, a cycloheptyl group, a cyclopentenyl group, a cyclohexenyl group, a phenyl group, a biphenyl group, a terphenyl group, a pentalenyl group, an indenyl group, a naphthyl group, an azulenyl group, an indacenyl group, an acenaphthenyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a perylenyl group, a pentacenyl group, a pyrrolyl group, a thiophenyl group, a furanyl group, a silolyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an indolyl group, an isoindolyl group, an indazolyl group, a purinyl group, a quinolinyl group, an isoquinolinyl group, a benzoquinolinyl group, a benzoisoquinolinyl group, a phthalazinyl group, a naphthyridinyl group, a quinoxalinyl group, a benzoquinoxalinyl group, a quinazolinyl group, a benzoquinazolinyl group, a cinnolinyl group, a phenanthridinyl group, an acridinyl group, a phenanthrolinyl group, a phenazinyl group, a benzimidazolyl group, a benzofuranyl group, a benzothiophenyl group, a benzosilolyl group, a benzothiazolyl group, a benzoisothiazolyl group, a benzoxazolyl group, a benzoisoxazolyl group, a triazolyl group, a tetrazolyl group, a thiadiazolyl group, an oxadiazolyl group, a triazinyl group, a carbazolyl group, a dibenzofuranyl group, a dibenzothiophenyl group, a dibenzosilolyl group, a benzocarbazolyl group, a naphthobenzofuranyl group, a naphthobenzothiophenyl group, a naphthobenzosilolyl group, a dibenzocarbazolyl group, a dinaphthofuranyl group, a dinaphthothiophenyl group, a dinaphthosilolyl group, an imidazopyridinyl group, an imidazopyrimidinyl group, an oxazolopyridinyl group, a thiazolopyridinyl group, a benzonaphthyridinyl group, an azafluorenyl group, an azaspiro-bifluorenyl group, an azacarbazolyl group, an azadibenzofuranyl group, an azadibenzothiophenyl group, an azadibenzosilolyl group, an indenopyrrolyl group, an indolopyrrolyl group, an indenocarbazolyl group, an indolocarbazolyl group, $-Si(Q_{31})(Q_{32})(Q_{33})$, $-N(Q_{31})(Q_{32})$, $-B(Q_{31})(Q_{32})$, $-C(=O)(Q_{31})$,$-S(=O)(Q_{31})$, $-S(=O)_2(Q_{31})$, $-P(=O)(Q_{31})(Q_{32})$, and $-P(=S)(Q_{31})(Q_{32})$; and

$-N(Q_{11})(Q_{12})$.

In some embodiments, $R_1$, $R_2$, $R_{10}$, $R_{20}$, and $R_{30}$ may each independently be selected from

hydrogen, deuterium, -F, -Cl, -Br, -I, a methyl group, an ethyl group, an n-propyl group, an iso-propyl group, an n-butyl group, a sec-butyl group, an iso-butyl group, a tert-butyl group, an ethenyl group, a propenyl group, a butenyl group, a methoxy group, an ethoxy group, an n-propoxy group, an iso-propoxy group, an n-butoxy group, a sec-butoxy group, an iso-butoxy group, and a tert-butoxy group; and

a group represented by one of Formulae 5-1 to 5-26 and Formulae 6-1 to 6-55:

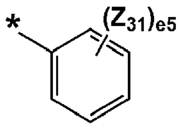

5-1

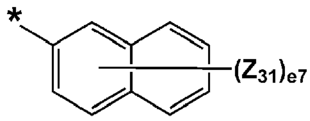

5-2

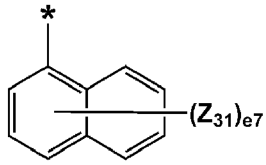

5-3

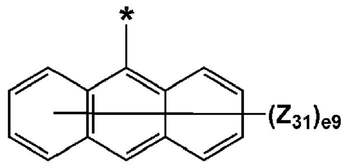

5-4

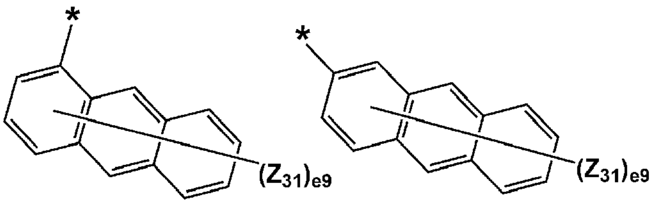

5-5 5-6

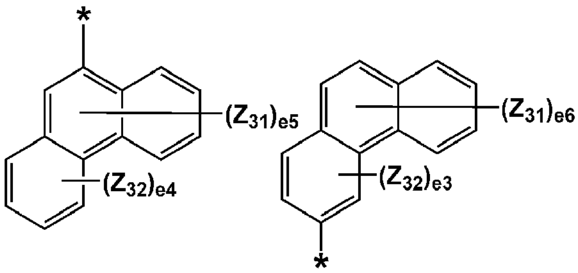

5-7 5-8

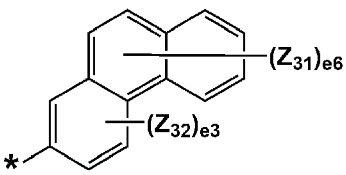

5-9

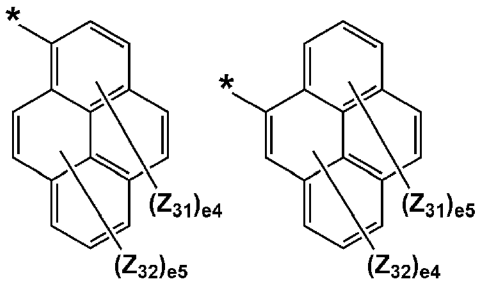

5-10 5-11

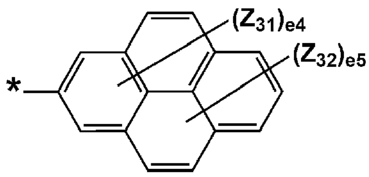

5-12

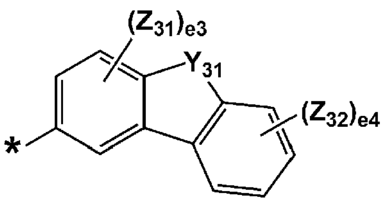

5-13

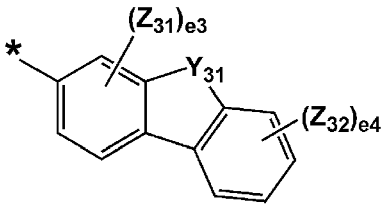

5-14

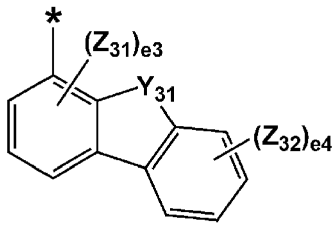

5-15

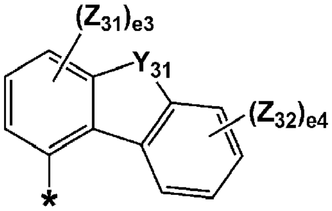

5-16

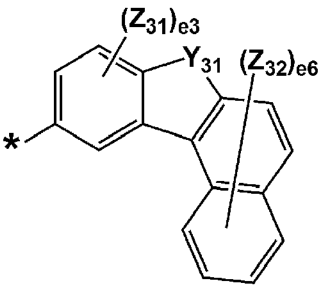

5-17

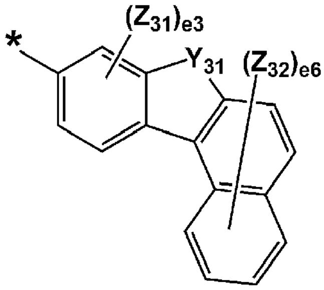

5-18

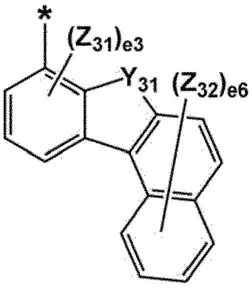

5-19

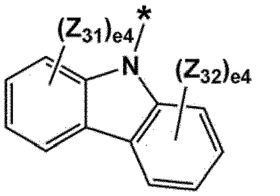

5-20

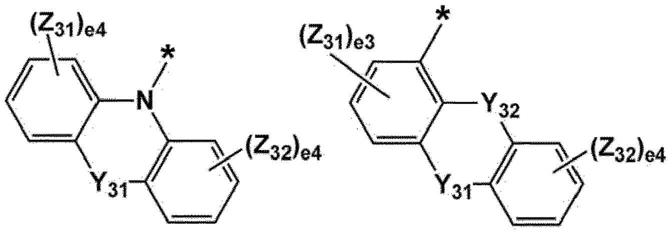

5-21

5-22

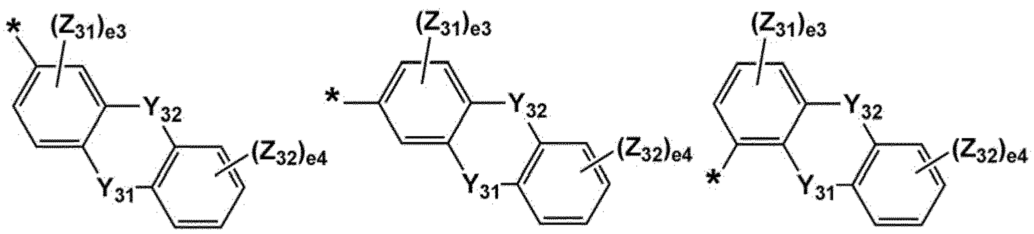

5-23

5-24

5-25

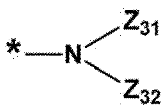

5-26

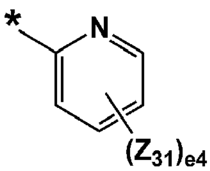

6-1

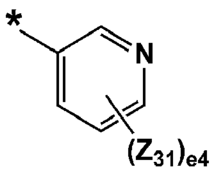

6-2

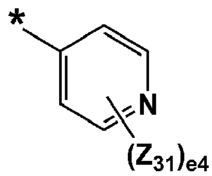

6-3

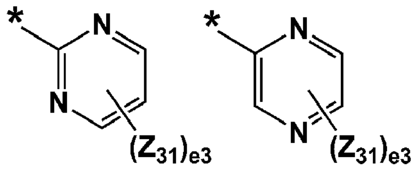

6-4

6-5

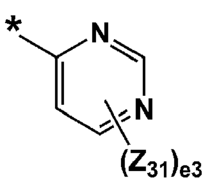

6-6

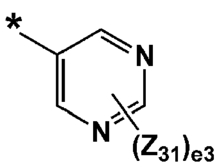

6-7

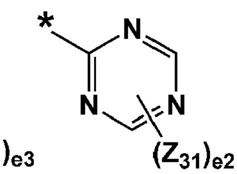

6-8

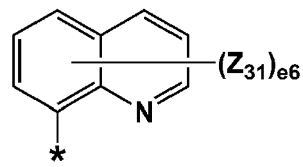

6-9

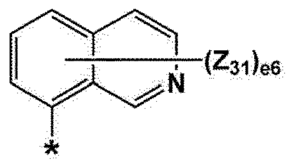
6-10
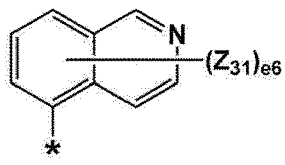
6-11
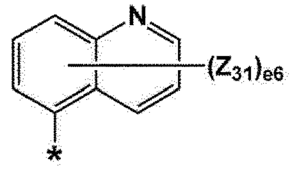
6-12
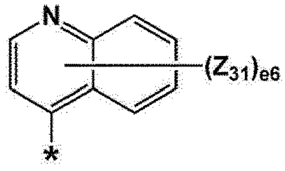
6-13

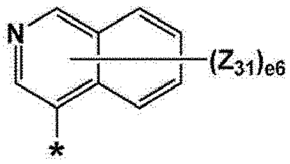
6-14
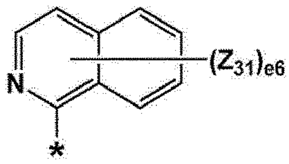
6-15
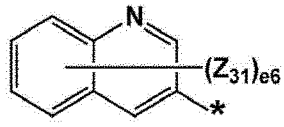
6-16
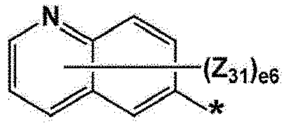
6-17

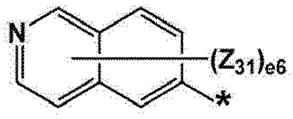
6-18
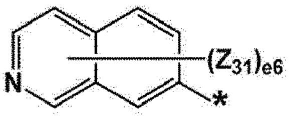
6-19
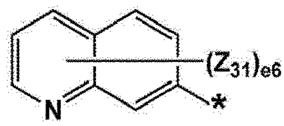
6-20
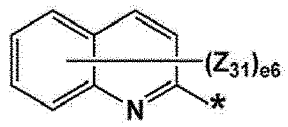
6-21

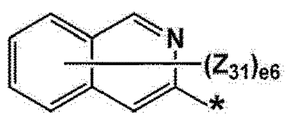
6-22
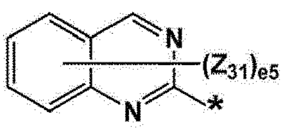
6-23
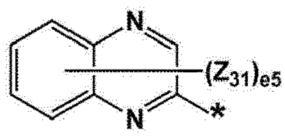
6-24
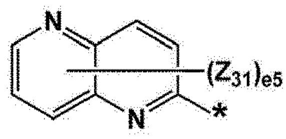
6-25

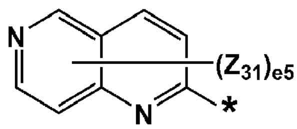
6-26
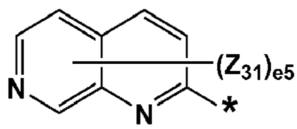
6-27
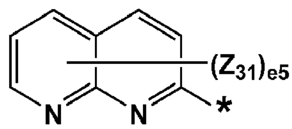
6-28
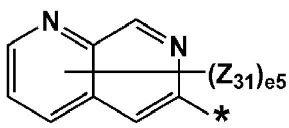
6-29

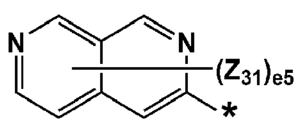
6-30
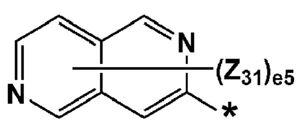
6-31
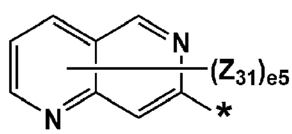
6-32
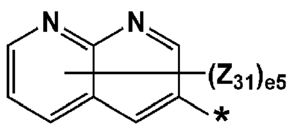
6-33

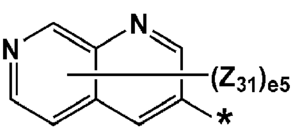
6-34
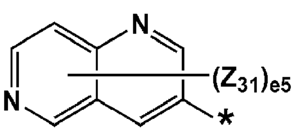
6-35
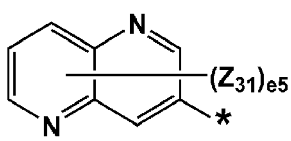
6-36
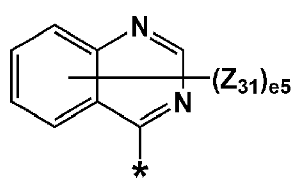
6-37

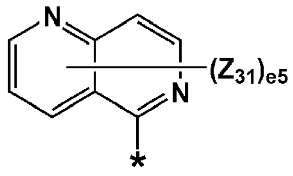

6-38

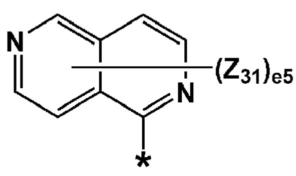

6-39

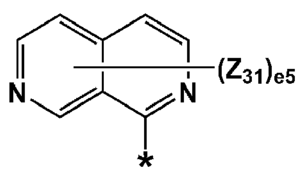

6-40

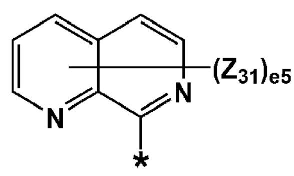

6-41

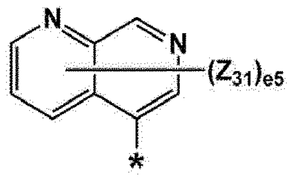

6-42

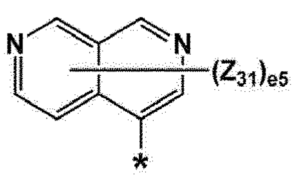

6-43

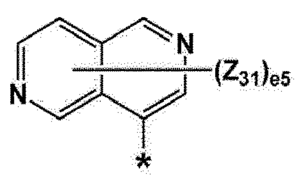

6-44

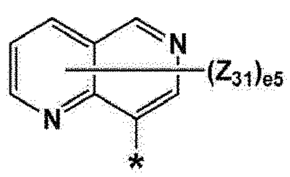

6-45

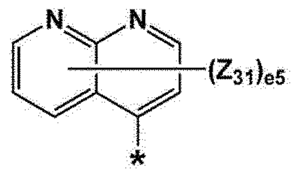

6-46

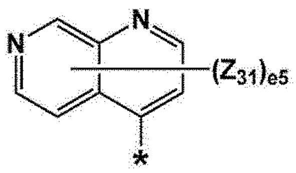

6-47

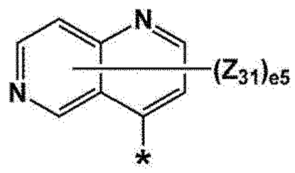

6-48

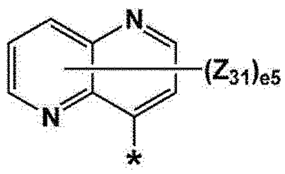

6-49

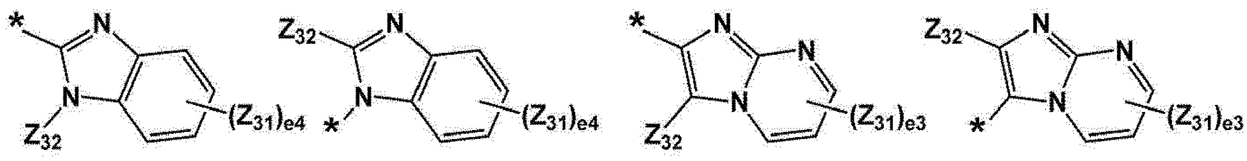

6-50 6-51 6-52 6-53

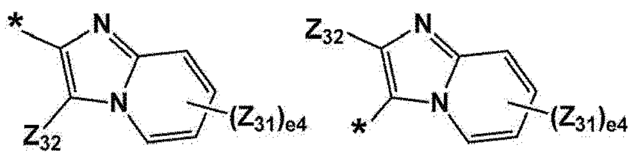

6-54 6-55

wherein, in Formulae 5-1 to 5-26 and Formulae 6-1 to 6-55,

$Y_{31}$ and $Y_{32}$ are each independently O, S, $C(Z_{34})(Z_{35})$, $N(Z_{34})$, or $Si(Z_{34})(Z_{35})$,
$Z_{31}$, $Z_{32}$, $Z_{34}$, and $Z_{35}$ are each independently selected from hydrogen, deuterium,-F, -Cl, -Br, -I, a hydroxyl group, a cyano group, a nitro group, an amidino group, a hydrazino group, a hydrazono group, a $C_1$-$C_{20}$ (*e.g.* $C_1$-$C_{10}$) alkyl group, a $C_1$-$C_{20}$ (*e.g.* $C_1$-$C_{10}$) alkenyl group, a $C_1$-$C_{20}$ (*e.g.* $C_1$-$C_{10}$) alkynyl group, a $C_1$-$C_{20}$ (*e.g.* $C_1$-$C_{10}$) alkoxy group, a phenyl group, a biphenyl group, a terphenyl group, a naphthyl group, a fluorenyl group, a spiro-bifluorenyl group, a phenanthrenyl group, an anthracenyl group, a triphenylenyl group, a pyridinyl group, a pyrimidinyl group, a carbazolyl group, and a triazinyl group,
e2 is 1 or 2,
e3 is an integer from 1 to 3,
e4 is an integer from 1 to 4,
e5 is an integer from 1 to 5,
e6 is an integer from 1 to 6,
e7 is an integer from 1 to 7,
e9 is an integer from 1 to 9, and
* indicates a binding site to an adjacent atom.

In an embodiment, the host may include at least one selected from Compounds 1-1 to 1-17:

Compound 1-1

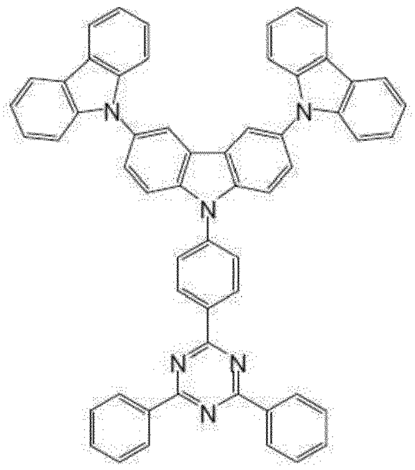

Compound 1-2

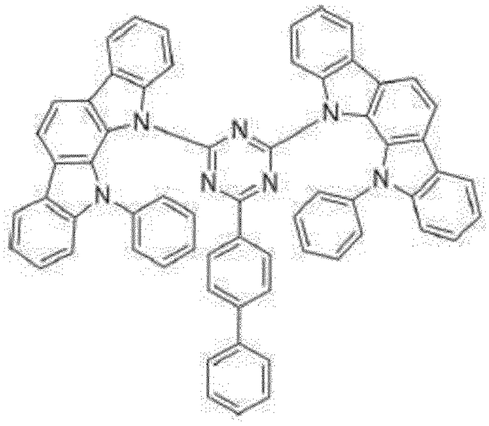

Compound 1-3

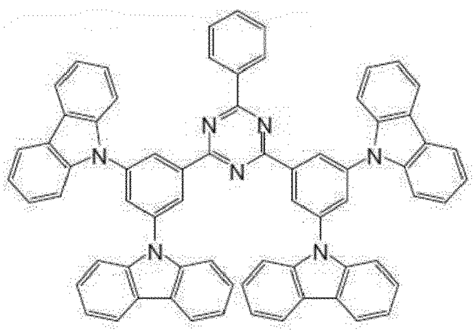

Compound 1-4

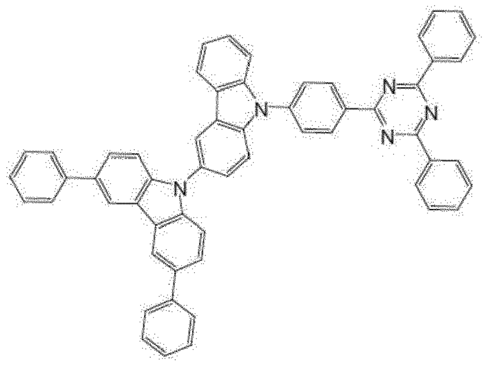

Compound 1-5

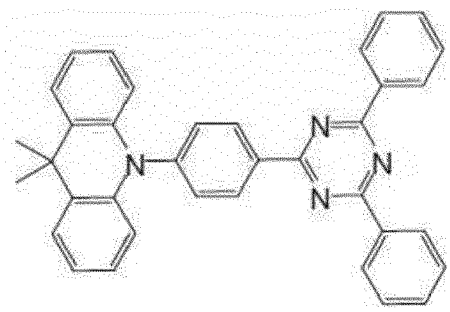

Compound 1-6

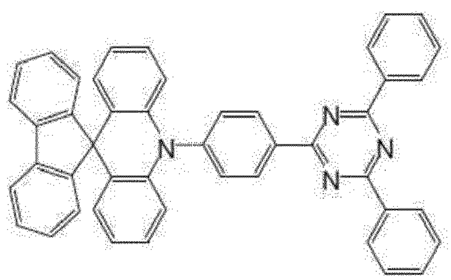

Compound 1-7

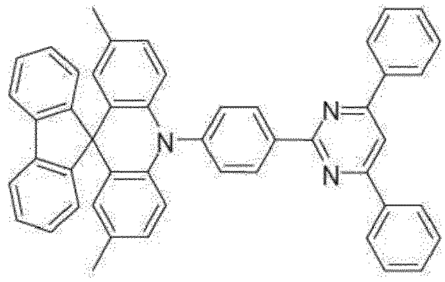

Compound 1-8

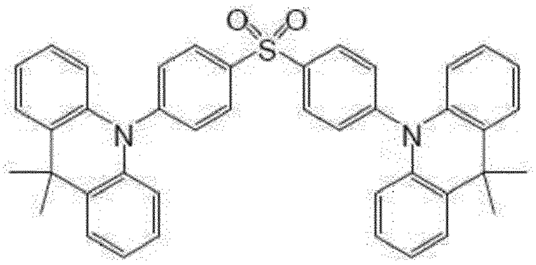

Compound 1-9

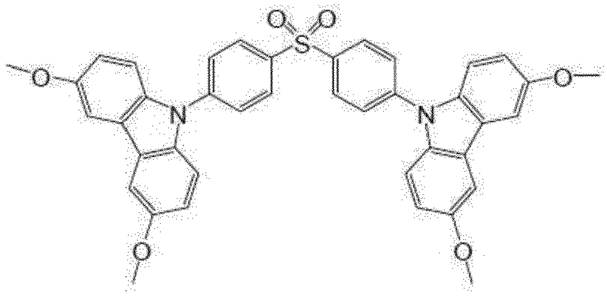

Compound 1-10

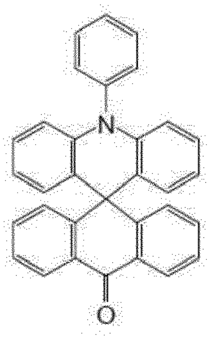

Compound 1-11

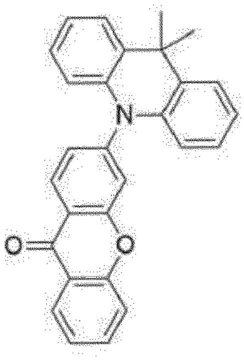

Compound 1-12

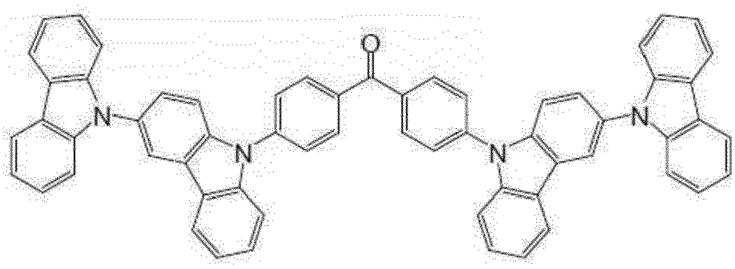

Compound 1-13

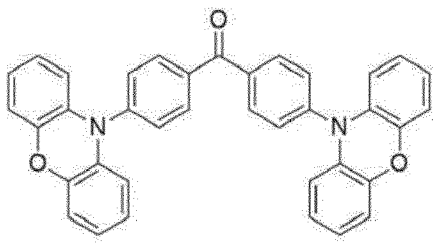

Compound 1-14

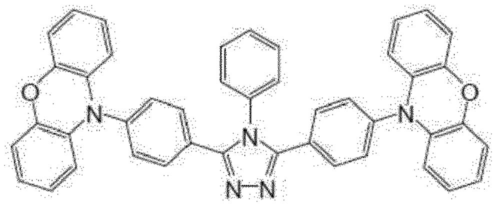

Compound 1-15

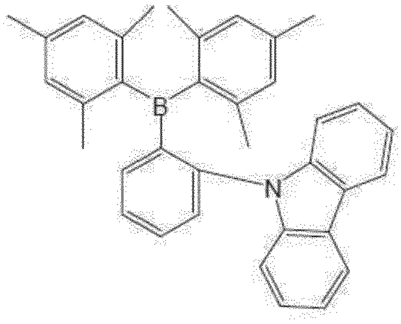

Compound 1-16

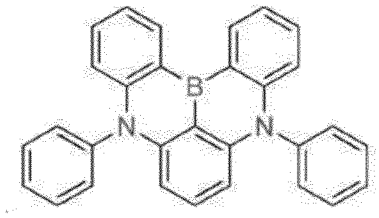

Compound 1-17

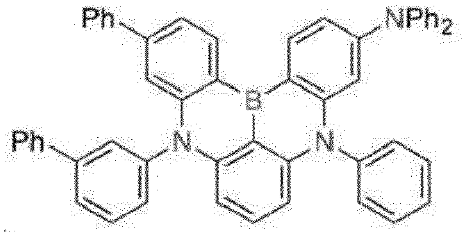

wherein "Ph" in Compounds 1-1 to 1-17 represents a phenyl group.

Since $Ar_1$ and $Ar_2$ in the heterocyclic compound represented by Formula 1 have a structure as described above, the heterocyclic compound may include a substituent having properties of an electron withdrawing group (EWG) and a substituent having properties of an electron donationg group (EDG). By introducing these substituents to proper positions, the energy difference between the singlet state and the triplet state of the overall compound may be properly controlled. By this, thermal activated delayed fluorescence (TADF) may be exhibited.

The energy relationship between a singlet energy (S1) and a triplet energy (T1) of the hetercyclic compound is as follows:

$$\triangle E_{st} = | T1 - S1 | \geq 0.3 \text{ eV}$$

That is, in the heterocyclic compound, as an electron donating moiety is separated from an electron withdrawing moiety, orbital overlap in a molecule may be effectively prevented. Accordingly, the singlet energy level and the triplet energy level of the molecule may not be overlap, thus having a very low $\Delta E_{st}$. Therefore, even at room temperature, RISC from the triplet excited state to the singlet excited state through thermal activation may be feasible, thereby showing TADF. Further, since triplet state excitons may be used in luminescence, the luminescence efficiency may improve.

Furthermore, since the heterocyclic compound has a relatively high hole or electron transportability, an exciton formation rate may increase in an emission layer included in an organic light-emitting device employing the heterocyclic compound represented by Formula 1. Thus, the organic light-emitting device may have a low driving voltage, high efficiency, long lifespan, and high maximum quantum efficiency.

Methods of synthesizing the heterocyclic compound represented by Formula 1 should be readily apparent to those of ordinary skill in the art by referring to Examples described herein. For example, methods of synthesizing the heterocyclic compound are described in documents such as Nature Materials 14, 330-336 (2015), Adv. Mater. (2015), 27(15), 2515-2520, Chem. Sci., 2017, 8, 953-960, ACS Appl. Mater. Interfaces 2017, 9, 24035-24042, and Adv. Mater. (2016), 28, 2777-2781. As used herein, "(for example, the organic layer) including at least one heterocyclic compound" means that "(the organic layer) including a heterocyclic compound of Formula 1, or at least two different heterocyclic compounds of Formula 1".

For example, the organic layer may include Compound 1-1 only as the heterocylic compound. In this embodiment, Compound 1-1 may be included in the emission layer of the organic light-emitting device. In some embodiments, the organic layer may include Compounds 1-1 and 1-2 as the heterocyclic compounds. In this embodiment, Compounds

1-1 and 1-2 may be present in the same layer (for example, Compounds 1-1 and 1-2 may be both present in an emission layer), or in different layers (for example, Compound 1-1 may be present in an emission layer, and Compound 1-2 may be present in an electron transport layer).

In some embodiments, the host may consist of one type of the heterocyclic compound.

In some embodiments, the phosphorescent dopant may include an organometallic compound represented by one of Formulae 4 and 5:

] Formula 4

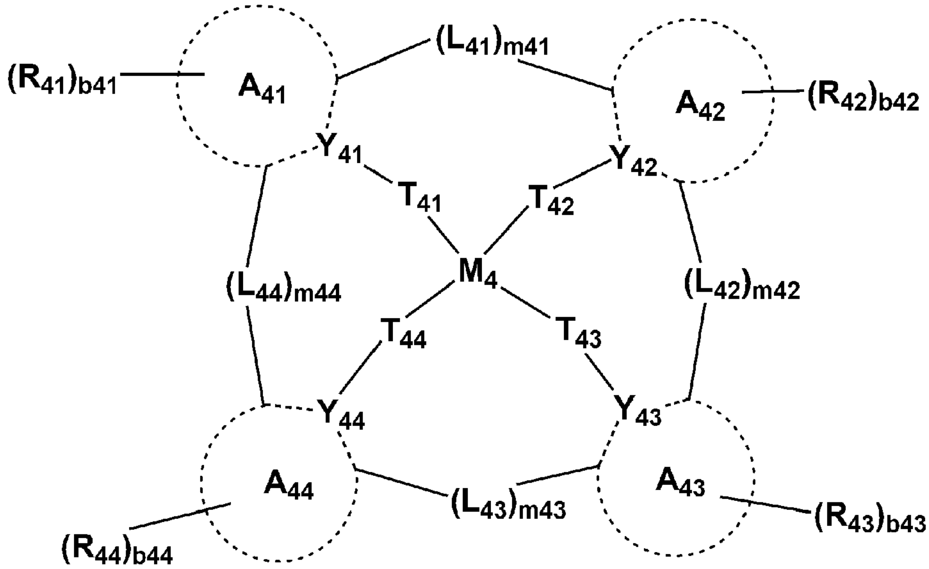

Formula 5

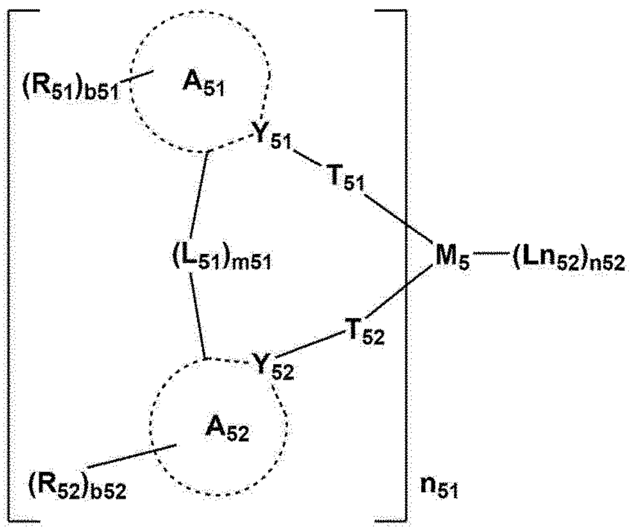

wherein, in Formulae 4 and 5,

$M_4$ and $M_5$ are each independently selected from platinum (Pt), palladium (Pd), copper (Cu), silver (Ag), gold (Au), rhodium (Rh), iridium (Ir), ruthenium (Ru), osmium (Os), titanium (Ti), zirconium (Zr), hafnium (Hf), europium (Eu), terbium (Tb), and thulium (Tm),

n51 is an integer from 1 to 3,

$Ln_{52}$ is an organic ligand, n52 is an integer from 0 to 2,

$Y_{41}$ to $Y_{44}$, $Y_{51}$, and $Y_{52}$ are each independently N or C,

$A_{41}$ to $A_{44}$, $A_{51}$, and $A_{52}$ are each independently selected from a $C_5$-$C_{60}$ (*e.g.* $C_5$-$C_{30}$) carbocyclic group and a $C_1$-$C_{60}$ (*e.g.* $C_1$-$C_{20}$) heterocyclic group,

$T_{41}$ to $T_{44}$, $T_{51}$, and $T_{52}$ are each independently selected from a single bond, *-O-*', and *-S-*',

$L_{41}$ to $L_{44}$ and $L_{51}$ are each independently selected from a single bond, *-O-*', *-S-*', *-C($R_{45}$)($R_{46}$)-*', *-C($R_{45}$)=*', *=C($R_{45}$)-*', *-C($R_{45}$)=C($R_{45}$)-*', *-C(=O)-*', *-C(=S)-*', *-C≡C-*', *-B($R_{45}$)-*', *-N($R_{45}$)-*', *-P($R_{45}$)-*', *-Si($R_{45}$)($R_{46}$)-*', *-P($R_{45}$)($R_{46}$)-*', and *-Ge($R_{45}$)($R_{46}$)-*',

m41 to m44, and m51 are each an integer from 0 to 3,

$R_{41}$ to $R_{46}$, $R_{51}$, and $R_{52}$ are each independently selected from hydrogen, deuterium, -F, -Cl, -Br, -I, a hydroxyl group, a cyano group, a nitro group, an amidino group, a hydrazino group, a hydrazono group, a substituted or unsubstituted $C_1$-$C_{20}$ (*e.g.* $C_1$-$C_{10}$) alkyl group, a substituted or unsubstituted $C_1$-$C_{20}$ (*e.g.* $C_1$-$C_{10}$) alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ (*e.g.* $C_6$-$C_{30}$) aryl group, a substituted or unsubstituted $C_6$-$C_{60}$ (*e.g.* $C_6$-$C_{30}$) aryloxy group, a substituted or unsubstituted C6-$C_{60}$ (*e.g.* $C_6$-$C_{30}$) arylthio group, a substituted or unsubstituted $C_1$-$C_{60}$ (*e.g.* $C_1$-$C_{20}$) heteroaryl group, a substituted or unsubstituted monovalent non-aromatic condensed polycyclic group and a substituted or unsubstituted monovalent non-aromatic condensed heteropolycyclic group,-Si($Q_{41}$)($Q_{42}$)($Q_{43}$), -N($Q_{41}$)($Q_{42}$), -B($Q_{41}$)($Q_{42}$), -C(=O)($Q_{41}$), -S(=O)$_2$($Q_{41}$), and -P(=O)($Q_{41}$)($Q_{42}$),

$R_{45}$ and $R_{41}$; $R_{45}$ and $R_{42}$; $R_{45}$ and $R_{43}$; or $R_{45}$ and $R_{44}$ are optionally bound to form a substituted or unsubstituted $C_5$-$C_{60}$ (*e.g.* $C_5$-$C_{30}$) carbocyclic group or a substituted or unsubstituted $C_1$-$C_{60}$ (*e.g.* $C_1$-$C_{20}$) heterocyclic group,

b41, b42, b43, b44, b51 and b52 are each independently an integer from 1 to 8,

* and *' each indicate a binding site to an adjacent atom, and

at least one substituent of the substituted $C_5$-$C_{60}$ (*e.g.* $C_5$-$C_{30}$) carbocyclic group, the substituted $C_1$-$C_{60}$ (*e.g.* $C_1$-$C_{20}$) heterocyclic group, the substituted $C_1$-$C_{20}$ alkyl group, the substituted $C_2$-$C_{60}$ (*e.g.* $C_2$-$C_{20}$) alkynyl group, the substituted $C_1$-$C_{20}$ (*e.g.* $C_2$-$C_{10}$) alkoxy group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_1$-$C_{10}$ heterocycloalkenyl group, the substituted $C_6$-$C_{60}$ (*e.g.* $C_6$-$C_{30}$) aryl group, the substituted $C_6$-$C_{60}$ (*e.g.* $C_6$-$C_{30}$) aryloxy group, the substituted $C_6$-$C_{60}$ (*e.g.* $C_6$-$C_{30}$) arylthio group, the substituted $C_1$-$C_{60}$ (*e.g.* $C_1$-$C_{20}$) heteroaryl group, the substituted monovalent non-aromatic condensed polycyclic group, and the substituted monovalent non-aromatic condensed heteropolycyclic group may be selected from:

deuterium, -F, -Cl, -Br, -I, a hydroxyl group, a cyano group, a nitro group, an amidino group, a hydrazino group, a hydrazono group, a $C_1$-$C_{60}$ (*e.g.* $C_1$-$C_{20}$) alkyl group, a $C_2$-$C_{60}$ (*e.g.* $C_2$-$C_{20}$) alkenyl group, a $C_2$-$C_{60}$ (*e.g.* $C_2$-$C_{20}$) alkynyl group, and a $C_1$-$C_{60}$ (*e.g.* $C_1$-$C_{20}$) alkoxy group;

a $C_1$-$C_{60}$ (*e.g.* $C_1$-$C_{20}$) alkyl group, a $C_2$-$C_{60}$ (*e.g.* $C_2$-$C_{20}$) alkenyl group, a $C_2$-$C_{60}$ (*e.g.* $C_2$-$C_{20}$) alkynyl group, and a $C_1$-$C_{60}$ (*e.g.* $C_1$-$C_{20}$) alkoxy group, each substituted with at least one selected from deuterium, -F, -Cl, -Br, -I, a hydroxyl group, a cyano group, a nitro group, an amidino group, a hydrazino group, a hydrazono group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ (*e.g.* $C_6$-$C_{30}$) aryl group, a $C_6$-$C_{60}$ (*e.g.* $C_6$-$C_{30}$) aryloxy group, a $C_6$-$C_{60}$ (*e.g.* $C_6$-$C_{30}$) arylthio group, a $C_1$-$C_{60}$ (*e.g.* $C_1$-$C_{20}$) heteroaryl group, a monovalent non-aromatic condensed polycyclic group, a monovalent non-aromatic condensed heteropolycyclic group,-Si($Q_{51}$)($Q_{52}$)($Q_{53}$), -N($Q_{51}$)($Q_{52}$), -B($Q_{51}$)($Q_{52}$), -C(=O)($Q_{51}$), -S(=O)$_2$($Q_{51}$), and -P(=O)($Q_{51}$)($Q_{52}$);

a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ (*e.g.* $C_6$-$C_{30}$) aryl group, a $C_6$-$C_{60}$ (*e.g.* $C_6$-$C_{30}$) aryloxy group, a $C_6$-$C_{60}$ (*e.g.* $C_6$-$C_{30}$) arylthio group, a $C_1$-$C_{60}$ (*e.g.* $C_1$-$C_{20}$) heteroaryl group, a monovalent non-aromatic condensed polycyclic group, and a monovalent non-aromatic condensed heteropolycyclic group;

a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ (*e.g.* $C_6$-$C_{30}$) aryl group, a $C_6$-$C_{60}$ (*e.g.* $C_6$-$C_{30}$) aryloxy group, a $C_6$-$C_{60}$ (*e.g.* $C_6$-$C_{30}$) arylthio group, a $C_1$-$C_{60}$ (*e.g.* $C_1$-$C_{20}$) heteroaryl group, a monovalent non-aromatic condensed polycyclic group, and a monovalent non-aromatic condensed heteropolycyclic group, each substituted with at least one selected from deuterium, -F, -Cl, -Br, -I, a hydroxyl group, a cyano group, a nitro group, an amidino group, a hydrazino group, a hydrazono group, a $C_1$-$C_{60}$ (*e.g.* $C_1$-$C_{20}$) alkyl group, a $C_2$-$C_{60}$ (*e.g.* $C_2$-$C_{20}$) alkenyl group, a $C_2$-$C_{60}$ (*e.g.* $C_2$-$C_{20}$) alkynyl group, a $C_1$-$C_{60}$ (*e.g.* $C_1$-$C_{20}$) alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ (*e.g.* $C_6$-$C_{30}$) aryl group, a $C_6$-$C_{60}$ (*e.g.* $C_6$-$C_{30}$) aryloxy group, a $C_6$-$C_{60}$ (*e.g.* $C_6$-$C_{30}$) arylthio group, a $C_1$-$C_{60}$ (*e.g.* $C_1$-$C_{20}$) heteroaryl group, a monovalent non-aromatic condensed polycyclic group, a monovalent non-aromatic condensed heteropolycyclic group, -Si($Q_{61}$)($Q_{62}$)($Q_{63}$), -N($Q_{61}$)($Q_{62}$), -B($Q_{61}$)($Q_{62}$), -C(=O)($Q_{61}$), -S(=O)$_2$($Q_{61}$), and-P(=O)($Q_{61}$)($Q_{62}$); and

-Si($Q_{71}$)($Q_{72}$)($Q_{73}$), -N($Q_{71}$)($Q_{72}$), -B($Q_{71}$)($Q_{72}$), -C(=O)($Q_{71}$), -S(=O)$_2$($Q_{71}$), and-P(=O)($Q_{71}$)($Q_{72}$),

wherein $Q_{41}$ to $Q_{43}$, $Q_{51}$ to $Q_{53}$, $Q_{61}$ to $Q_{63}$, and $Q_{71}$ to $Q_{73}$ are each independently selected from hydrogen, deuterium, -F, -Cl, -Br, -I, a hydroxyl group, a cyano group, a nitro group, an amidino group, a hydrazino group, a hydrazono group, a $C_1$-$C_{60}$ (*e.g.* $C_1$-$C_{20}$) alkyl group, a $C_2$-$C_{60}$ (*e.g.* $C_2$-$C_{20}$) alkenyl group, a $C_2$-$C_{60}$ (*e.g.* $C_2$-$C_{20}$) alkynyl group, a $C_1$-$C_{60}$

(*e.g.* $C_1$-$C_{20}$) alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ (*e.g.* $C_6$-$C_{30}$) aryl group, a C6-$C_{60}$ (*e.g.* $C_6$-$C_{30}$) aryloxy group, a $C_6$-$C_{60}$ (*e.g.* $C_6$-$C_{30}$) arylthio group, a $C_1$-$C_{60}$ (*e.g.* $C_1$-$C_{20}$) heteroaryl group, a $C_1$-$C_{60}$ (*e.g.* $C_1$-$C_{20}$) heteroaryloxy group, a $C_1$-$C_{60}$ (*e.g.* $C_1$-$C_{20}$) heteroarylthio group, a monovalent non-aromatic condensed polycyclic group, a monovalent non-aromatic condensed heteropolycyclic group, a $C_1$-$C_{60}$ (*e.g.* $C_1$-$C_{20}$) alkyl group substituted with at least one selected from deuterium, -F, and a cyano group, a $C_6$-$C_{60}$ (*e.g.* $C_6$-$C_{30}$) aryl group substituted with at least one selected from deuterium, -F, and a cyano group, a biphenyl group, and a terphenyl group.

In some embodiments, in Formulae 4 and 5, $M_4$ and $M_5$ may each independently be selected from Pt, Pd, Cu, Ag, Au, Ir, and Os.

In some embodiments, in Formulae 4 and 5, $M_4$ and $M_5$ may each independently be Pt or Ir.

In some embodiments, $M_4$ may be Pt, and $M_5$ may be Ir.

In some embodiments, Lns2 in Formula 5 may be any suitable monovalent, divalent, or trivalent organic ligand. For example, Lns2 may be selected from halogen, diketone (e.g., acetylacetonate), a carboxylic acid (e.g., picolinate), -C(=O), isonitrile, -CN, and phosphorus (e.g., phosphine or phosphite), but embodiments are not limited thereto.

In some embodiments, in Formula 4,

$Y_{41}$, $Y_{42}$, and $Y_{43}$ may each be C, and $Y_{44}$ may be N,
$Y_{41}$, $Y_{42}$, and $Y_{44}$ may each be C, and $Y_{43}$ may be N,
$Y_{41}$, $Y_{43}$, and $Y_{44}$ may each be C, and $Y_{42}$ may be N,
$Y_{42}$, $Y_{43}$, and $Y_{44}$ may each be C, and $Y_{41}$ may be N,
$Y_{41}$ and $Y_{44}$ may each be C, and $Y_{42}$ and $Y_{43}$ may each be N,
$Y_{41}$ and $Y_{44}$ may each be N, and $Y_{42}$ and $Y_{43}$ may each be C,
$Y_{41}$ and $Y_{42}$ may each be C, and $Y_{43}$ and $Y_{44}$ may each be N,
$Y_{41}$ and $Y_{42}$ may each be N, and $Y_{43}$ and $Y_{44}$ may each be C,
$Y_{41}$ and $Y_{43}$ may each be C, and $Y_{42}$ and $Y_{44}$ may each be N, or
$Y_{41}$ and $Y_{43}$ may each be N, and $Y_{42}$ and $Y_{44}$ may each be C.
In some embodiments, in Formula 5,
$Y_{51}$ and $Y_{52}$ may each be C,
$Y_{51}$ may be N, and $Y_{52}$ may be C,
$Y_{51}$ may be C, and $Y_{52}$ may be N, or
$Y_{51}$ and $Y_{52}$ may each be N.

In some embodiments, in Formulae 4 and 5, $A_{41}$ to $A_{44}$, $A_{51}$, and $A_{52}$ may each independently be selected from a benzene group, a naphthalene group, an anthracene group, a phenanthrene group, a triphenylene group, a pyrene group, a chrysene group, a cyclopentadiene group, a 1,2,3,4-tetrahydronaphthalene group, a furan group, a thiophene group, a silole group, an indene group, a fluorene group, an indole group, a carbazole group, a benzofuran group, a dibenzofuran group, a benzothiophene group, a dibenzothiophene group, a benzosilole group, a dibenzosilole group, an indenopyridine group, an indolopyridine group, a benzofuropyridine group, a benzothienopyridine group, a benzosilolopyridine group, an indenopyrimidine group, an indolopyrimidine group, a benzofuropyrimidine group, a benzothienopyrimidine group, a benzosilolopyrimidine group, a dihydropyridine group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a 2,3-dihydroimidazole group, a triazole group, a 2,3-dihydrotriazole group, an oxazole group, an iso-oxazole group, a thiazole group, an isothiazole group, an oxadiazole group, a thiadiazole group, a benzopyrazole group, a benzimidazole group, a 2,3-dihydrobenzimidazole group, an imidazopyridine group, a 2,3-dihydroimidazopyridine group, an imidazopyrimidine group, a 2,3-dihydroimidazopyrimidine group, an imidazopyrazine group, a 2,3-dihydroimidazopyrazine group, a benzoxazole group, a benzothiazole group, a benzoxadiazole group, a benzothiadiazole group, a 5,6,7,8-tetrahydroisoquinoline group, and a 5,6,7,8-tetrahydroquinoline group.

In some embodiments, in Formulae 4 and 5, $A_{41}$ to $A_{44}$, $A_{51}$ and $A_{52}$ may each independently be a group represented by one of Formulae 2-1 to 2-43:

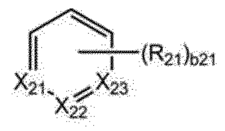
2-1

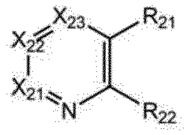
2-2

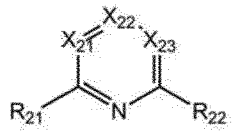
2-3

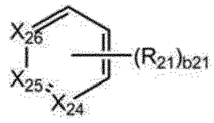
2-4

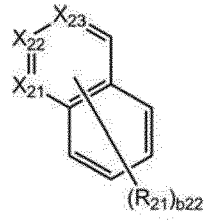
2-5

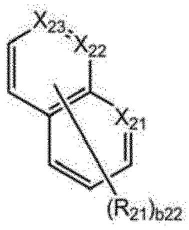
2-6

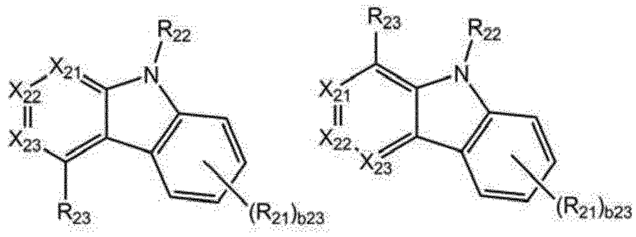
2-7

2-8

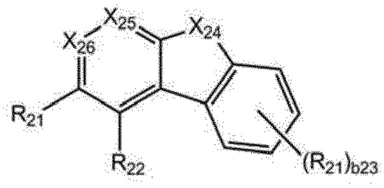
2-9

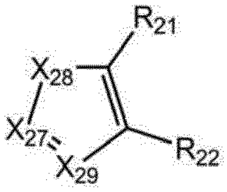
2-10

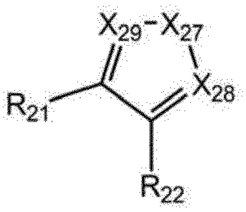
2-11

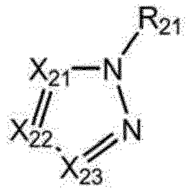
2-12

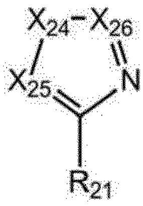
2-13

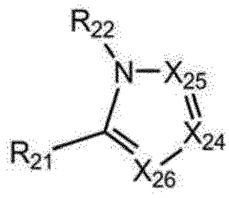
2-14

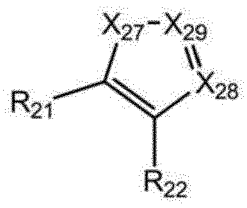
2-15

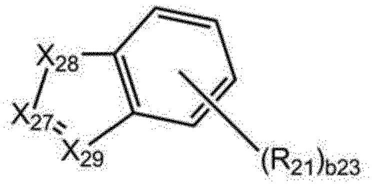
2-16

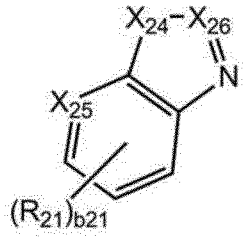
2-17

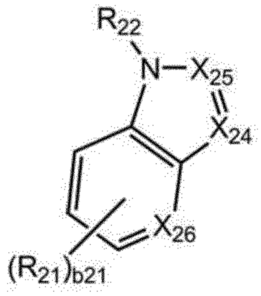
2-18

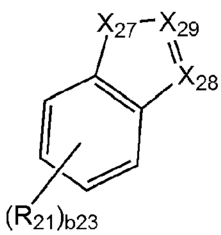
2-19

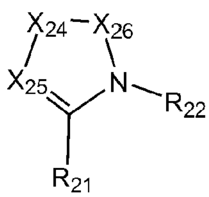
2-20

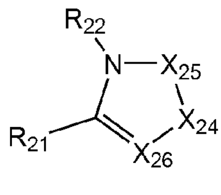
2-21

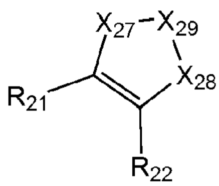
2-22

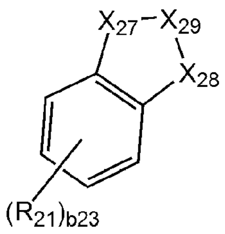

2-23

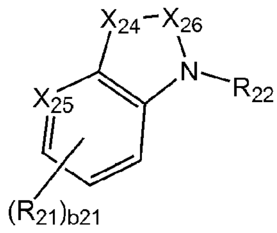

2-24

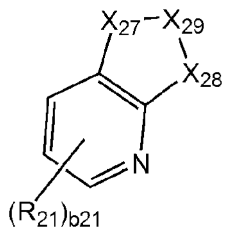

2-25

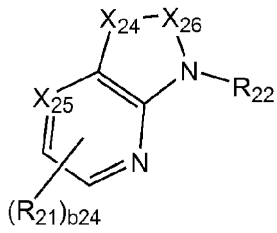

2-26

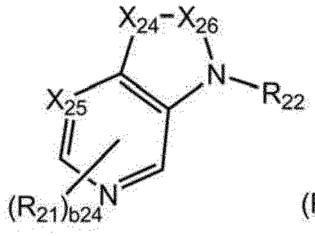

2-27

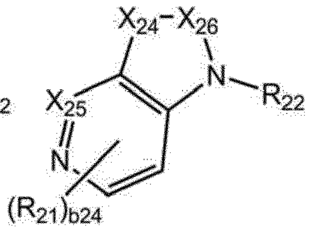

2-28

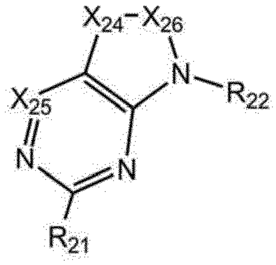

2-29

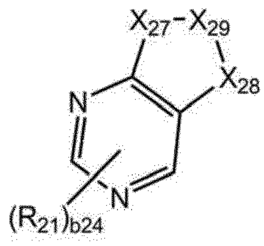

2-30

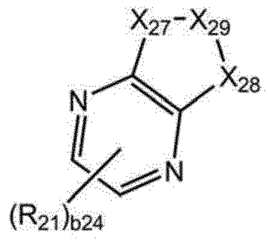

2-31

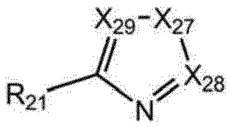

2-32

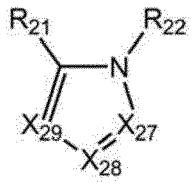

2-33

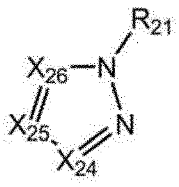

2-34

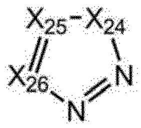

2-35

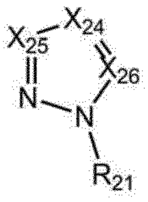

2-36

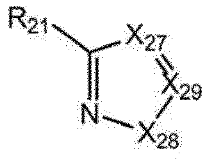

2-37

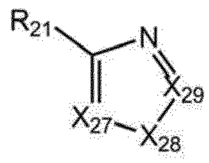

2-38

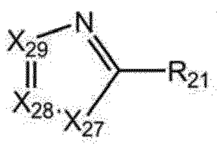

2-39

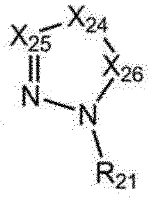

2-40

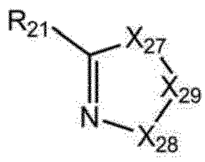

2-41

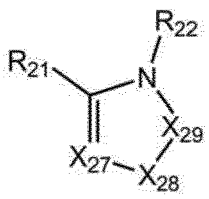

2-42

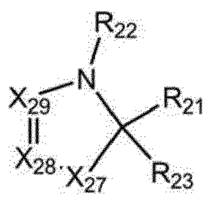

2-43

wherein, in Formulae 2-1 to 2-43,

$X_{21}$ to $X_{23}$ are each independently selected from $C(R_{24})$ and C-*, provided that at least two selected from $X_{21}$ to $X_{23}$ are each C-*,

$X_{24}$ is N-*, $X_{25}$ and $X_{26}$ are each independently selected from $C(R_{24})$ and C-*, provided that at least one selected

from $X_{25}$ and $X_{26}$ is C-*,
$X_{27}$ and $X_{28}$ are each independently selected from N, N($R_{25}$), and N-*, and $X_{29}$ is selected from C($R_{24}$) and C-*, provided that i) at least one selected from $X_{27}$ and $X_{28}$ is N-*, and $X_{29}$ is C-*, or ii) $X_{27}$ and $X_{28}$ are each N-*, and $X_{29}$ is C($R_{24}$),
$R_{21}$ to $R_{25}$ are each independently understood by referring to the descriptions for $R_{10}$ provided herein,
b21 is selected from 1, 2, and 3,
b22 is selected from 1, 2, 3, 4, and 5,
b23 is selected from 1, 2, 3, and 4,
b24 is selected from 1 and 2, and
* indicates a binding site to an adjacent atom.
In some embodiments, in Formula 4, $T_{41}$ to $T_{44}$ may each be a single bond,
$T_{41}$ may be selected from O and S, and $T_{42}$ to $T_{44}$ may each be a single bond,
$T_{42}$ may be selected from O and S, and $T_{41}$, $T_{43}$, and $T_{44}$ may each be a single bond,
$T_{43}$ may be selected from O and S, and $T_{41}$, $T_{42}$, and $T_{44}$ may each be a single bond, or
$T_{44}$ may be selected from O and S, and $T_{41}$, $T_{42}$, and $T_{43}$ may each be a single bond.

In some embodiments, in Formula 4, $T_{41}$ to $T_{44}$ may each be a single bond.
In some embodiments, in Formula 5, $T_{51}$ and $T_{52}$ may each be a single bond.
In some embodiments, a bond between $Y_{41}$ and $T_{41}$ or a bond between $Y_{41}$ and $M_4$ may each be a covalent bond or a coordinate bond.
In some embodiments, a bond between $Y_{42}$ and $T_{42}$ or a bond between $Y_{42}$ and $M_4$ may each be a covalent bond or a coordinate bond.
In some embodiments, a bond between $Y_{43}$ and $T_{43}$ or a bond between $Y_{43}$ and $M_4$ may each be a covalent bond or a coordinate bond.
In some embodiments, a bond between $Y_{44}$ and $T_{44}$ or a bond between $Y_{44}$ and $M_4$ may each be a covalent bond or a coordinate bond.
In some embodiments, a bond between $Y_{51}$ and $T_{51}$ or a bond between $Y_{51}$ and $M_5$ may each be a covalent bond or a coordinate bond.
In some embodiments, a bond between $Y_{52}$ and $T_{52}$ or a bond between $Y_{52}$ and $M_5$ may each be a covalent bond or a coordinate bond.
In some embodiments, $L_{41}$ to $L_{44}$ and $L_{51}$ may each independently be selected from a single bond, *-O-*', *-S-*', *-C($R_{45}$)($R_{46}$)-*', *-C($R_{45}$)=*', *=C($R_{45}$)-*', *-C($R_{45}$)=C($R_{45}$)-*', *-C(=O)-*', and *-N($R_{45}$)-*'.
In some embodiments, in Formulae 4 and 5, $R_{41}$ to $R_{46}$, $R_{51}$, and $R_{52}$ may each independently be selected from
hydrogen, deuterium, -F, -Cl, -Br, -I, a cyano group, a $C_1$-$C_{20}$ (*e.g.* $C_1$-$C_{10}$) alkyl group, and a $C_1$-$C_{20}$ (*e.g.* $C_1$-$C_{10}$) alkoxy group;
a $C_1$-$C_{20}$ (*e.g.* $C_1$-$C_{10}$) alkyl group and a $C_1$-$C_{20}$ (*e.g.* $C_1$-$C_{10}$) alkoxy group, each substituted with at least one selected from deuterium, -F, -Cl, -Br, -I, a cyano group, a phenyl group, and a biphenyl group;
a cyclopentyl group, a cyclohexyl group, a cycloheptyl group, a cyclopentenyl group, a cyclohexenyl group, a phenyl group, a biphenyl group, a terphenyl group, a pentalenyl group, an indenyl group, a naphthyl group, an azulenyl group, an indacenyl group, an acenaphthenyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a perylenyl group, a pentacenyl group, a pyrrolyl group, a thiophenyl group, a furanyl group, a silolyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an indolyl group, an isoindolyl group, an indazolyl group, a purinyl group, a quinolinyl group, an isoquinolinyl group, a benzoquinolinyl group, a benzoisoquinolinyl group, a phthalazinyl group, a naphthyridinyl group, a quinoxalinyl group, a benzoquinoxalinyl group, a quinazolinyl group, a benzoquinazolinyl group, a cinnolinyl group, a phenanthridinyl group, an acridinyl group, a phenanthrolinyl group, a phenazinyl group, a benzimidazolyl group, a benzofuranyl group, a benzothiophenyl group, a benzosilolyl group, a benzothiazolyl group, a benzoisothiazolyl group, a benzoxazolyl group, a benzoisoxazolyl group, a triazolyl group, a tetrazolyl group, a thiadiazolyl group, an oxadiazolyl group, a triazinyl group, a carbazolyl group, a dibenzofuranyl group, a dibenzothiophenyl group, a dibenzosilolyl group, a benzocarbazolyl group, a naphthobenzofuranyl group, a naphthobenzothiophenyl group, a naphthobenzosilolyl group, a dibenzocarbazolyl group, a dinaphthofuranyl group, a dinaphthothiophenyl group, a dinaphthosilolyl group, an imidazopyridinyl group, an imidazopyrimidinyl group, an oxazolopyridinyl group, a thiazolopyridinyl group, a benzonaphthyridinyl group, an azafluorenyl group, an azaspiro-bifluorenyl group, an azacarbazolyl group, an azadibenzofuranyl group, an azadibenzothiophenyl group, an azadibenzosilolyl group, an indenopyrrolyl group, an indolopyrrolyl group, an indenocarbazolyl group, and an indolocarbazolyl group;
a cyclopentyl group, a cyclohexyl group, a cycloheptyl group, a cyclopentenyl group, a cyclohexenyl group, a

phenyl group, a biphenyl group, a terphenyl group, a pentalenyl group, an indenyl group, a naphthyl group, an azulenyl group, an indacenyl group, an acenaphthenyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a perylenyl group, a pentacenyl group, a pyrrolyl group, a thiophenyl group, a furanyl group, a silolyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an indolyl group, an isoindolyl group, an indazolyl group, a purinyl group, a quinolinyl group, an isoquinolinyl group, a benzoquinolinyl group, a benzoisoquinolinyl group, a phthalazinyl group, a naphthyridinyl group, a quinoxalinyl group, a benzoquinoxalinyl group, a quinazolinyl group, a benzoquinazolinyl group, a cinnolinyl group, a phenanthridinyl group, an acridinyl group, a phenanthrolinyl group, a phenazinyl group, a benzimidazolyl group, a benzofuranyl group, a benzothiophenyl group, a benzosilolyl group, a benzothiazolyl group, a benzoisothiazolyl group, a benzoxazolyl group, a benzoisoxazolyl group, a triazolyl group, a tetrazolyl group, a thiadiazolyl group, an oxadiazolyl group, a triazinyl group, a carbazolyl group, a dibenzofuranyl group, a dibenzothiophenyl group, a dibenzosilolyl group, a benzocarbazolyl group, a naphthobenzofuranyl group, a naphthobenzothiophenyl group, a naphthobenzosilolyl group, a dibenzocarbazolyl group, a dinaphthofuranyl group, a dinaphthothiophenyl group, a dinaphthosilolyl group, an imidazopyridinyl group, an imidazopyrimidinyl group, an oxazolopyridinyl group, a thiazolopyridinyl group, a benzonaphthyridinyl group, an azafluorenyl group, an azaspiro-bifluorenyl group, an azacarbazolyl group, an azadibenzofuranyl group, an azadibenzothiophenyl group, an azadibenzosilolyl group, an indenopyrrolyl group, an indolopyrrolyl group, an indenocarbazolyl group, and an indolocarbazolyl group, each substituted with at least one selected from deuterium, -F, -Cl, -Br, -I, a cyano group, a $C_1$-$C_{20}$ (*e.g.* $C_1$-$C_{10}$) alkyl group, a $C_1$-$C_{20}$ (*e.g.* $C_1$-$C_{10}$) alkoxy group, a cyclopentyl group, a cyclohexyl group, a cycloheptyl group, a cyclopentenyl group, a cyclohexenyl group, a phenyl group, a biphenyl group, a terphenyl group, a pentalenyl group, an indenyl group, a naphthyl group, an azulenyl group, an indacenyl group, an acenaphthenyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a perylenyl group, a pentacenyl group, a pyrrolyl group, a thiophenyl group, a furanyl group, a silolyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an indolyl group, an isoindolyl group, an indazolyl group, a purinyl group, a quinolinyl group, an isoquinolinyl group, a benzoquinolinyl group, a benzoisoquinolinyl group, a phthalazinyl group, a naphthyridinyl group, a quinoxalinyl group, a benzoquinoxalinyl group, a quinazolinyl group, a benzoquinazolinyl group, a cinnolinyl group, a phenanthridinyl group, an acridinyl group, a phenanthrolinyl group, a phenazinyl group, a benzimidazolyl group, a benzofuranyl group, a benzothiophenyl group, a benzosilolyl group, a benzothiazolyl group, a benzoisothiazolyl group, a benzoxazolyl group, a benzoisoxazolyl group, a triazolyl group, a tetrazolyl group, a thiadiazolyl group, an oxadiazolyl group, a triazinyl group, a carbazolyl group, a dibenzofuranyl group, a dibenzothiophenyl group, a dibenzosilolyl group, a benzocarbazolyl group, a naphthobenzofuranyl group, a naphthobenzothiophenyl group, a naphthobenzosilolyl group, a dibenzocarbazolyl group, a dinaphthofuranyl group, a dinaphthothiophenyl group, a dinaphthosilolyl group, an imidazopyridinyl group, an imidazopyrimidinyl group, an oxazolopyridinyl group, a thiazolopyridinyl group, a benzonaphthyridinyl group, an azafluorenyl group, an azaspiro-bifluorenyl group, an azacarbazolyl group, an azadibenzofuranyl group, an azadibenzothiophenyl group, an azadibenzosilolyl group, an indenopyrrolyl group, an indolopyrrolyl group, an indenocarbazolyl group, an indolocarbazolyl group, $-Si(Q_{31})(Q_{32})(Q_{33})$, $-N(Q_{31})(Q_{32})$, $-B(Q_{31})(Q_{32})$, $-C(=O)(Q_{31})$, $-S(=O)(Q_{31})$, $-S(=O)_2(Q_{31})$, $-P(=O)(Q_{31})(Q_{32})$, and-$P(=S)(Q_{31})(Q_{32})$; and

$-Si(Q_1)(Q_2)(Q_3)$, $-B(Q_1)(Q_2)$, $-N(Q_1)(Q_2)$, $-P(Q_1)(Q_2)$, $-C(=O)(Q_1)$, $-S(=O)(Q_1)$,$-S(=O)_2(Q_1)$, $-P(=O)(Q_1)(Q_2)$, and $-P(=S)(Q_1)(Q_2)$,

wherein $Q_1$ to $Q_3$ and $Q_{31}$ to $Q_{33}$ may each independently be selected from hydrogen, deuterium, -F, -Cl, -Br, -I, a hydroxyl group, a cyano group, a nitro group, an amidino group, a hydrazino group, a hydrazono group, a $C_1$-$C_{60}$ (*e.g.* $C_1$-$C_{20}$) alkyl group, a $C_2$-$C_{60}$ (*e.g.* $C_2$-$C_{20}$) alkenyl group, a $C_2$-$C_{60}$ (*e.g.* $C_2$-$C_{20}$) alkynyl group, a $C_1$-$C_{60}$ (*e.g.* $C_1$-$C_{20}$) alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ (*e.g.* $C_6$-$C_{30}$) aryl group, a $C_6$-$C_{60}$ (*e.g.* $C_6$-$C_{30}$) aryloxy group, a $C_6$-$C_{60}$ (*e.g.* $C_6$-$C_{30}$) arylthio group, a $C_1$-$C_{60}$ (*e.g.* $C_1$-$C_{20}$) heteroaryl group, a $C_1$-$C_{60}$ (*e.g.* $C_1$-$C_{20}$) heteroaryloxy group, a $C_1$-$C_{60}$ (*e.g.* $C_1$-$C_{20}$) heteroarylthio group, a monovalent non-aromatic condensed polycyclic group, a monovalent non-aromatic condensed heteropolycyclic group, a $C_1$-$C_{60}$ (*e.g.* $C_1$-$C_{20}$) alkyl group substituted with at least one selected from deuterium,-F, and a cyano group, a $C_1$-$C_{60}$ (e.g. $C_1$-$C_{20}$) aryl group substituted with at least one selected from deuterium, -F, and a cyano group, a biphenyl group, and a terphenyl group.

In some embodiments, $R_{41}$ to $R_{46}$, $R_{51}$, and $R_{52}$ may each independently be selected from

hydrogen, deuterium, -F, -Cl, -Br, -I, a methyl group, an ethyl group, an n-propyl group, an iso-propyl group, an n-butyl group, a sec-butyl group, an iso-butyl group, a tert-butyl group, an ethenyl group, a propenyl group, a butenyl group, a methoxy group, an ethoxy group, an n-propoxy group, an iso-propoxy group, an n-butoxy group, a sec-butoxy group, an iso-butoxy group, and a tert-butoxy group; and

a group represented by one of Formulae 5-1 to 5-26 and Formulae 6-1 to 6-55.

In some embodiments, in Formulae 4 and 5, $R_{41}$ to $R_{46}$, $R_{51}$, and $R_{52}$ may each independently be selected from

hydrogen, deuterium, -F, -Cl, -Br, -I, a cyano group, a $C_1$-$C_{20}$ (*e.g.* $C_1$-$C_{10}$) alkyl group, and a $C_1$-$C_{20}$ (*e.g.* $C_1$-$C_{10}$) alkoxy group;

a $C_1$-$C_{20}$ (*e.g.* $C_1$-$C_{10}$) alkyl group and a $C_1$-$C_{20}$ (*e.g.* $C_1$-$C_{10}$) alkoxy group, each substituted with at least one selected from deuterium, -F, -Cl, -Br, -I, a cyano group, a phenyl group, and a biphenyl group; and

a phenyl group, a biphenyl group, a terphenyl group, a naphthyl group, a fluorenyl group, a spiro-bifluorenyl group, a carbazolyl group, a dibenzofuranyl group, a dibenzothiophenyl group, and a dibenzosilolyl group; and

a phenyl group, a biphenyl group, a terphenyl group, a naphthyl group, a fluorenyl group, a spiro-bifluorenyl group, a carbazolyl group, a dibenzofuranyl group, a dibenzothiophenyl group, and a dibenzosilolyl group, each substituted with at least one selected from deuterium, -F, -Cl, -Br, -I, a cyano group, a $C_1$-$C_{20}$ (*e.g.* $C_1$-$C_{10}$) alkyl group, a $C_1$-$C_{20}$ (*e.g.* $C_1$-$C_{10}$) alkoxy group, a phenyl group, and a biphenyl group.

In some embodiments, (i) in Formula 4, in a case where $Y_{41}$ and $Y_{42}$ are each N, $Y_{43}$ and $Y_{44}$ are each C, and m43 is 0, $A_{43}$ may be a 6-membered heterocyclic group.

For example, the 6-membered heterocyclic group may be selected from a benzene group, a pyridine group, a pyrimidine group, a pyrazine group, a pyridazine group, and a triazine group.

In some embodiments, (ii) in Formula 5, in a case where $A_{51}$ is a pyridine group, and $A_{52}$ is a benzene group, at least one selected from $R_{51}$ and $R_{52}$ may not be hydrogen.

In some embodiments, the phosphorescent dopant may include at least one selected from Compounds 2-1 to 2-45:

Compound 2-1

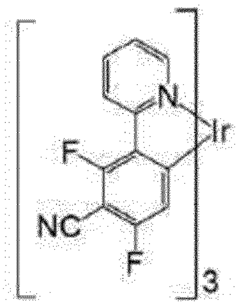

Compound 2-2

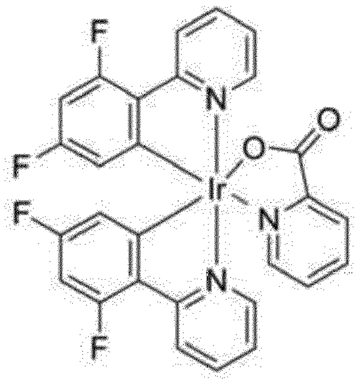

Compound 2-3

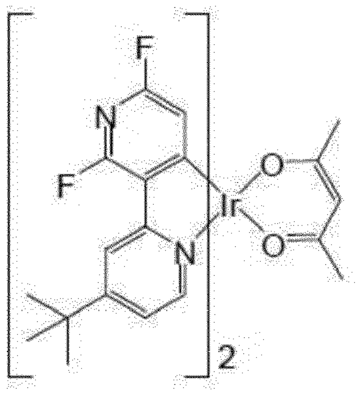

Compound 2-4

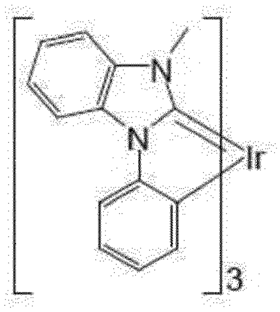

Compound 2-5

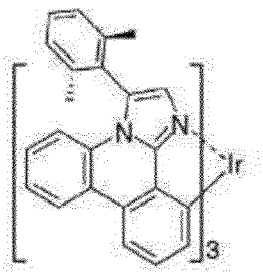

Compound 2-6

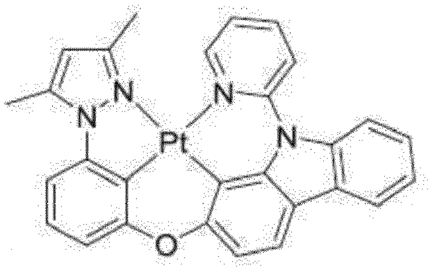

Compound 2-7

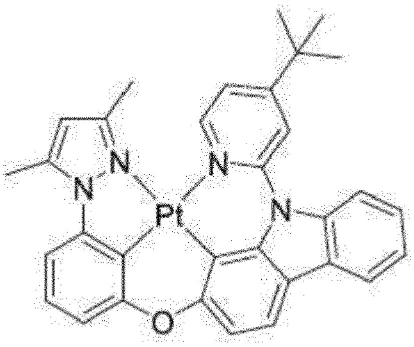

Compound 2-8

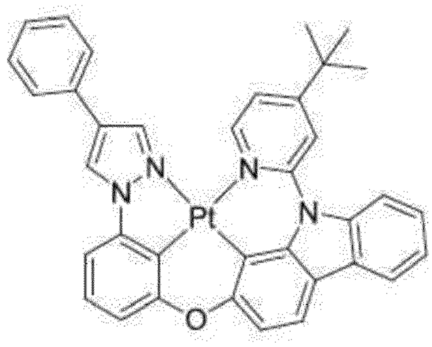

Compound 2-9

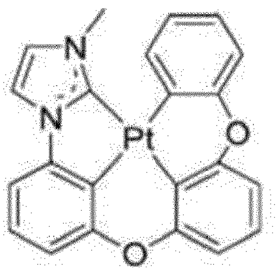

Compound 2-10

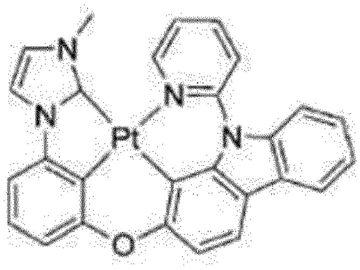

Compound 2-11

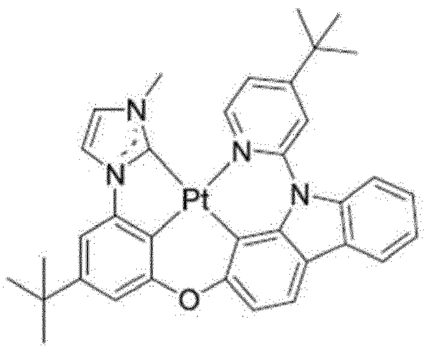

Compound 2-12

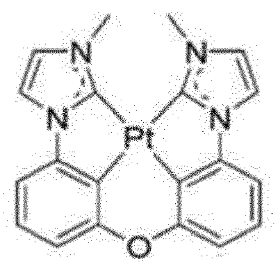

Compound 2-13

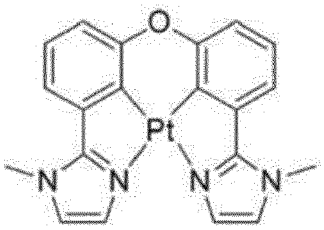

Compound 2-14

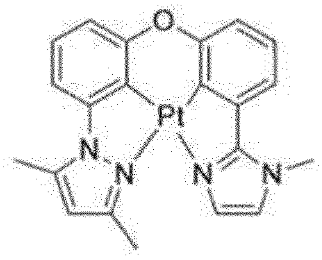

Compound 2-15

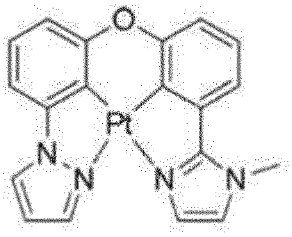

Compound 2-16

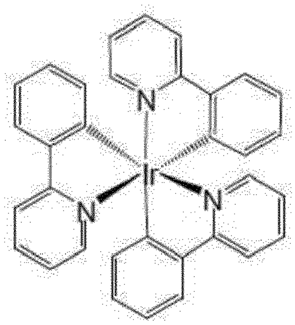

Compound 2-17

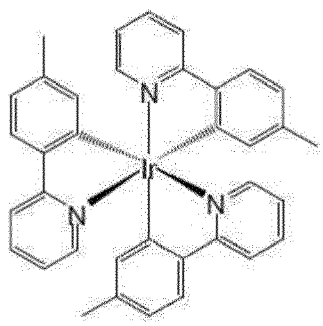

Compound 2-18

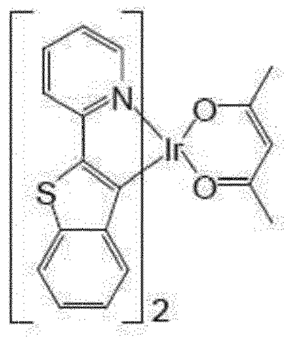

Compound 2-19

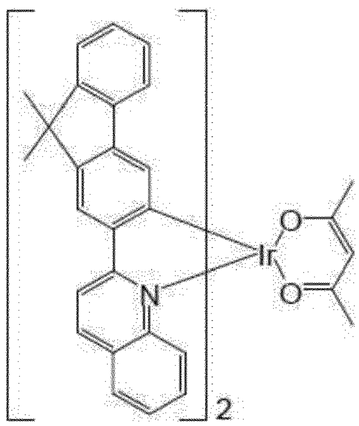

Compound 2-20

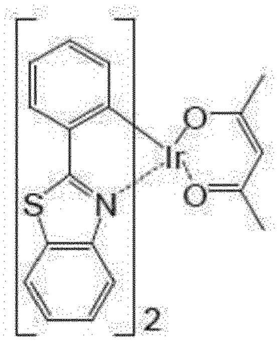

Compound 2-21

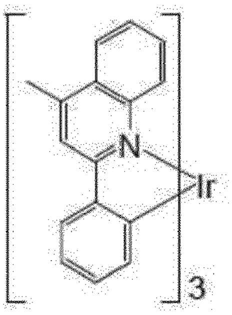

Compound 2-22

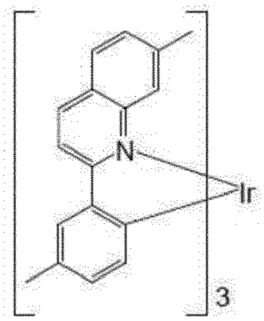

Compound 2-23

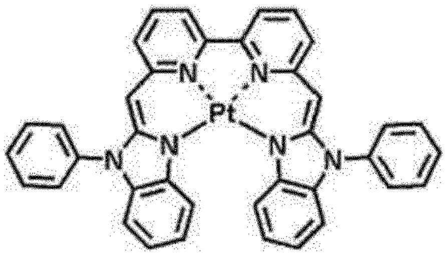

Compound 2-24

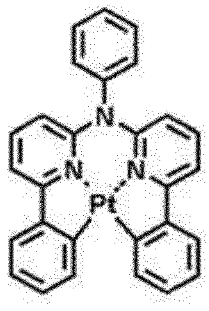

Compound 2-25

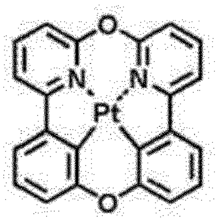

Compound 2-26

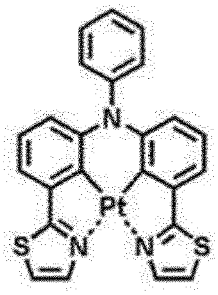

Compound 2-27

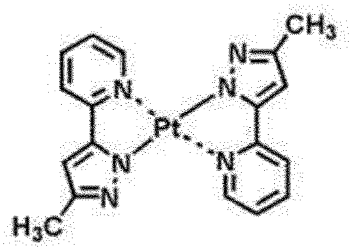

Compound 2-28

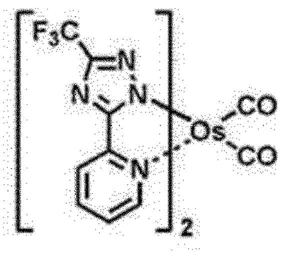

Compound 2-29

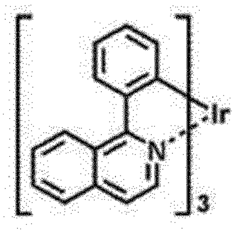

Compound 2-30

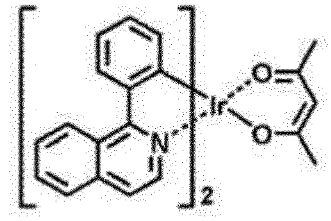

Compound 2-31

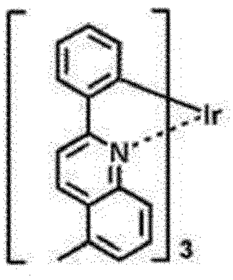

Compound 2-32

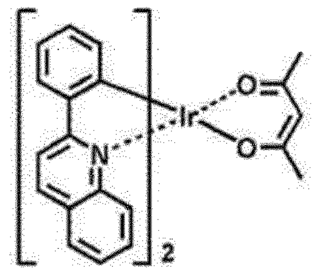

Compound 2-33

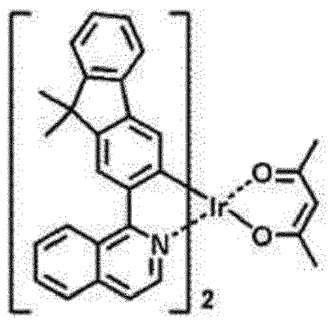

Compound 2-34

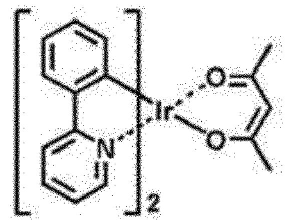

Compound 2-35

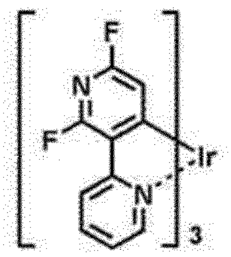

Compound 2-36

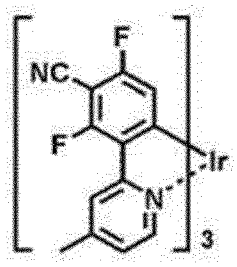

Compound 2-37

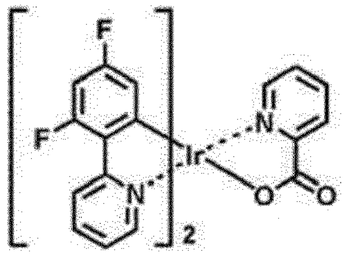

Compound 2-38

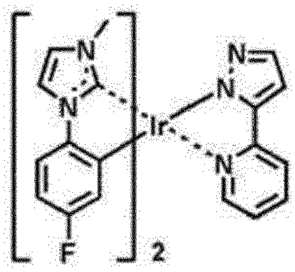

Compound 2-39

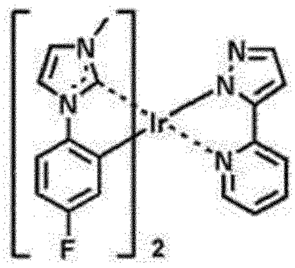

Compound 2-40

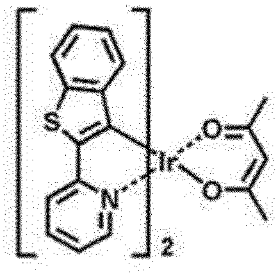

Compound 2-41

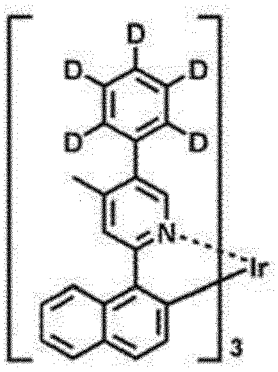

Compound 2-42

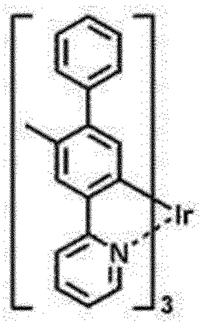

Compound 2-43

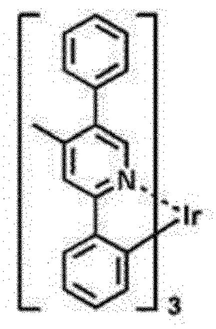

Compound 2-44

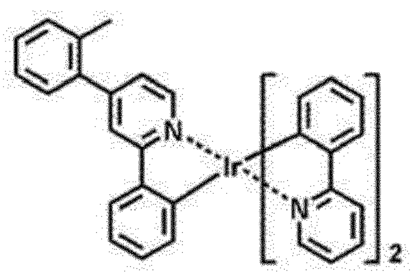

Compound 2-45

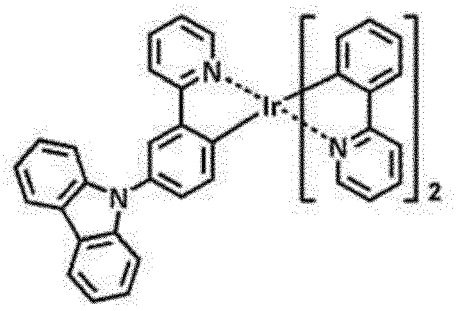

.

In addition, in the organic light-emitting device, as the host and the phosphorescent dopant satisfy Equation 3, the triplet energy of the host may be equal to or greater than the triplet energy of the phosphorescent dopant. Accordingly, excitons in a triplet state may be used in light emission. Thus, the organic light-emitting device may have a low driving voltage, improved luminescence efficiency, and high external quantum efficiency.

In some embodiments, a weight percentage of the host may be greater than a weight percentage of the phosphorescent dopant in the emission layer.

In some embodiments, the emission layer may emit blue light having a maximum emission wavelength of 420 nanometers (nm) or greater and 475 nm or lower.

In some embodiments, the first electrode may be an anode, the second electrode may be a cathode, and the organic layer may further include a hole transport region between the first electrode and the emission layer and an electron transport region between the emission layer and the second electrode, wherein the hole transport region may include a hole injection layer, a hole transport layer, an emission auxiliary layer, an electron blocking layer, or a combination thereof, and

the electron transport region may include a hole blocking layer, an electron transport layer, an electron injection layer, or a combination thereof.

In some embodiments, a hole transport region of the organic light-emitting device may include a p-dopant, wherein the p-dopant may have the lowest unoccupied molecular orbital (LUMO) level of -3.5 electron Volts (eV) or less.

In some embodiments, the hole transport region may include an electron blocking layer, and the electron blocking layer may include a carbazole-containing compound. In some embodiments, the electron blocking layer may be in a direct contact with the emission layer.

In an embodiment, the electron transport region of the organic light-emitting device may further include a metal-containing material, e.g., an alkali metal, an alkaline earth metal, a rare earth metal, an alkali metal compound, an alkaline earth metal compound, a rare earth metal compound, an alkali metal complex, an alkaline earth metal complex, a rare earth metal complex, or a combination thereof.

In some embodiments, the electron transport region may include a hole blocking layer, and the hole blocking layer may include a dibenzothiophene-containing compound. In some embodiments, the hole blocking layer may be in a direct contact with the emission layer.

The term "organic layer" as used herein refers to a single and/or a plurality of layers between the first electrode and the second electrode in an organic light-emitting device. A material included in the "organic layer" is not limited to an organic material.

Description of FIG. 1

FIG. 1 illustrates a schematic cross-sectional view of an organic light-emitting device 10 according to an embodiment. The organic light-emitting device 10 includes a first electrode 110, an organic layer 150, and a second electrode 190.

Hereinafter, the structure of the organic light-emitting device 10 according to an embodiment and a method of manufacturing an organic light-emitting device according to an embodiment will be described in connection with FIG. 1.

First electrode 110

In FIG. 1, a substrate may be additionally located under the first electrode 110 or above the second electrode 190. The substrate may be a glass substrate or a plastic substrate, each having excellent mechanical strength, thermal stability, transparency, surface smoothness, ease of handling, and water resistance.

The first electrode 110 may be formed by depositing or sputtering, onto the substrate, a material for forming the first electrode 110. When the first electrode 110 is an anode, the material for forming the first electrode 11 may be selected from materials with a high work function that facilitate hole injection.

The first electrode 110 may be a reflective electrode, a semi-transmissive electrode, or a transmissive electrode. When the first electrode 110 is a transmissive electrode, a material for forming the first electrode 110 may be selected from indium tin oxide (ITO), indium zinc oxide (IZO), tin oxide ($SnO_2$), zinc oxide (ZnO), and any combinations thereof, but embodiments are not limited thereto. In some embodiments, when the first electrode 110 is a semi-transmissive electrode or a reflective electrode, as a material for forming the first electrode 110, at least one of magnesium (Mg), silver (Ag), aluminium (Al), aluminium-lithium (Al-Li), calcium (Ca), magnesium-indium (Mg-In), magnesium-silver (Mg-Ag), and any combination thereof may be used, but embodiments are not limited thereto.

The first electrode 110 may have a single-layered structure, or a multi-layered structure including two or more layers. In some embodiments, the first electrode 110 may have a triple-layered structure of ITO/Ag/ITO, but embodiments are not limited thereto.

Organic layer 150

The organic layer 150 may be on the first electrode 110. The organic layer 150 may include an emission layer.

The organic layer 150 may further include a hole transport region between the first electrode 110 and the emission layer and an electron transport region between the emission layer and the second electrode 190.

Hole transport region in organic layer 150

The hole transport region may have i) a single-layered structure consisting of a single layer consisting of a single material, ii) a single-layered structure consisting of a single layer including a plurality of different materials, or iii) a multi-layered structure having a plurality of layers including a plurality of different materials.

The hole transport region may include at least one selected from a hole injection layer, a hole transport layer, an emission auxiliary layer, and an electron blocking layer.

For example, the hole transport region may have a single-layered structure including a single layer including a plurality of different materials or a multi-layered structure, e.g., a hole injection layer/hole transport layer structure, a hole injection layer/hole transport layer/emission auxiliary layer structure, a hole injection layer/emission auxiliary layer structure, a hole transport layer/emission auxiliary layer structure, or a hole injection layer/hole transport layer/electron blocking layer structure, wherein layers of each structure are sequentially stacked on the first electrode 110 in each stated order, but embodiments are not limited thereto.

The hole transport region may include at least one selected from m-MTDATA, TDATA, 2-TNATA, NPB (NPD), β-NPB, TPD, a spiro-TPD, a spiro-NPB, methylated-NPB, TAPC, HMTPD, 4,4',4"-tris(N-carbazolyl)triphenylamine (TCTA), polyaniline/dodecylbenzenesulfonic acid (PANI/DBSA), poly(3,4-ethylenedioxythiophene)/poly(4-styrenesulfonate) (PEDOT/PSS), polyaniline/camphor sulfonic acid (PANI/CSA), polyaniline/poly(4-styrenesulfonate) (PANI/PSS), a compound represented by Formula 201, and a compound represented by Formula 202:

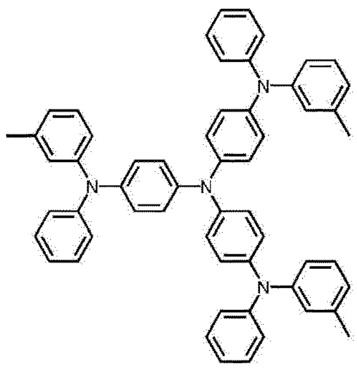

m-MTDATA

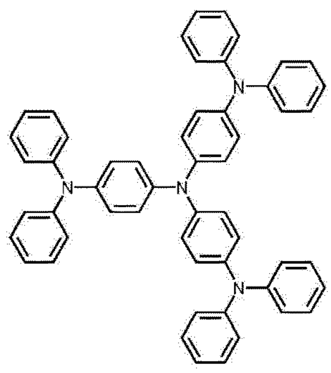

TDATA

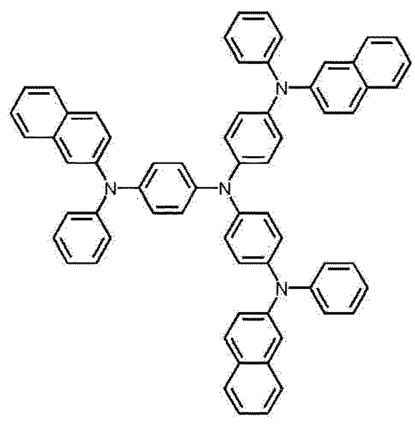

2-TNATA

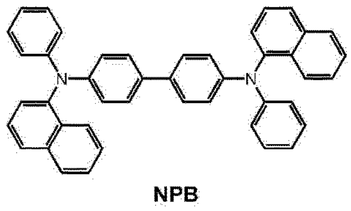

NPB

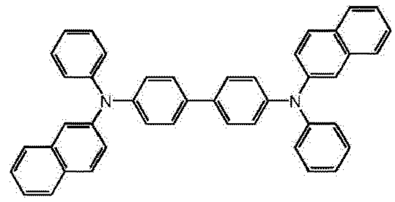

β-NPB

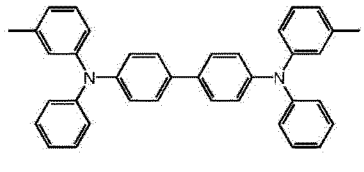

TPD

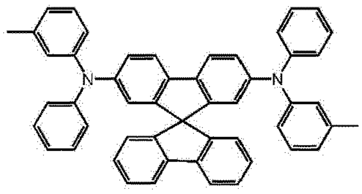

Spiro-TPD

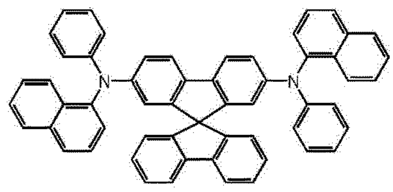

Spiro-NPB

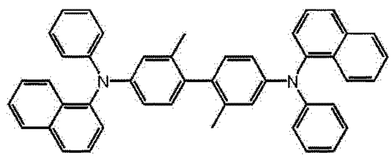

methylated NPB

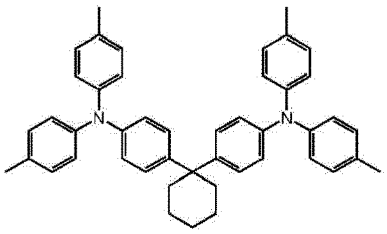

TAPC

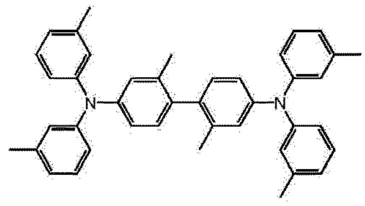

HMTPD

Formula 201

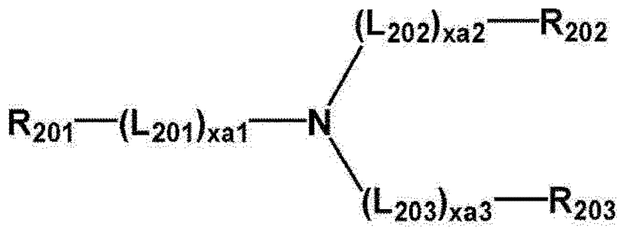

Formula 202

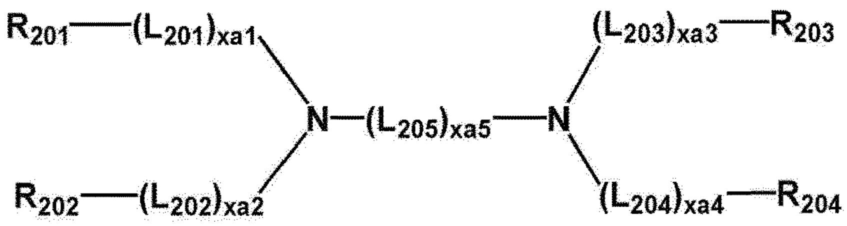

wherein, in Formulae 201 and 202,

$L_{201}$ to $L_{204}$ may each independently be selected from a substituted or unsubstituted $C_3$-$C_{10}$ cycloalkylene group, a substituted or unsubstituted $C_1$-$C_{10}$ heterocycloalkylene group, a substituted or unsubstituted $C_3$-$C_{10}$ cycloalkenylene group, a substituted or unsubstituted $C_1$-$C_{10}$ heterocycloalkenylene group, a substituted or unsubstituted $C_6$-$C_{60}$ (*e.g.* $C_6$-$C_{32}$) arylene group, a substituted or unsubstituted $C_1$-$C_{60}$ (*e.g.* $C_1$-$C_{20}$) heteroarylene group, a substituted or unsubstituted divalent non-aromatic condensed polycyclic group, and a substituted or unsubstituted divalent non-aromatic condensed heteropolycyclic group,

$L_{205}$ may be selected from *-O-*', *-S-*', *-N($Q_{201}$)-*', a substituted or unsubstituted $C_1$-$C_{20}$ alkylene group, a substituted or unsubstituted $C_2$-$C_{20}$ alkenylene group, a substituted or unsubstituted $C_3$-$C_{10}$ cycloalkylene group, a substituted or unsubstituted $C_1$-$C_{10}$ heterocycloalkylene group, a substituted or unsubstituted $C_3$-$C_{10}$ cycloalkenylene group, a substituted or unsubstituted $C_1$-$C_{10}$ heterocycloalkenylene group, a substituted or unsubstituted $C_6$-$C_{60}$ (*e.g.* $C_6$-$C_{32}$) arylene group, a substituted or unsubstituted $C_1$-$C_{60}$ (*e.g.* $C_1$-$C_{20}$) heteroarylene group, a substituted or unsubstituted divalent non-aromatic condensed polycyclic group, and a substituted or unsubstituted divalent non-aromatic condensed heteropolycyclic group,

xa1 to xa4 may each independently be an integer from 0 to 3,

xa5 may be an integer from 1 to 10, and

$R_{201}$ to $R_{204}$ and $Q_{201}$ may each independently be selected from a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ (*e.g.* $C_6$-$C_{32}$) aryl group, a substituted or unsubstituted $C_6$-$C_{60}$ (*e.g.* $C_6$-$C_{32}$) aryloxy group, a substituted or unsubstituted $C_6$-$C_{60}$ (*e.g.* $C_6$-$C_{32}$) arylthio group, a substituted or unsubstituted $C_1$-$C_{60}$ (*e.g.* $C_1$-$C_{20}$) heteroaryl group, a substituted or unsubstituted monovalent non-aromatic condensed polycyclic group, and a substituted or unsubstituted monovalent non-aromatic condensed heteropolycyclic group.

In some embodiments, in Formula 202, $R_{201}$ and $R_{202}$ may optionally be bound via a single bond, a dimethyl-methylene group, or a diphenyl-methylene group, and $R_{203}$ and $R_{204}$ may optionally be bound via a single bond, a dimethyl-methylene group, or a diphenyl-methylene group.

In an embodiment, in Formulae 201 and 202,

$L_{201}$ to $L_{205}$ may each independently be selected from

a phenylene group, a pentalenylene group, an indenylene group, a naphthylene group, an azulenylene group, a heptalenylene group, an indacenylene group, an acenaphthenylene group, a fluorenylene group, a spiro-bifluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, and a pyridinylene group; and

a phenylene group, a pentalenylene group, an indenylene group, a naphthylene group, an azulenylene group, a heptalenylene group, an indacenylene group, an acenaphthenylene group, a fluorenylene group, a spiro-bifluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, and a pyridinylene group, each substituted with at least one selected from deuterium, -F, -Cl, -Br, -I, a hydroxyl group, a cyano group, a nitro group, an amidino group, a hydrazino group, a hydrazono group, a $C_1$-$C_{20}$ alkyl group, a $C_1$-$C_{20}$ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a $C_1$-$C_{10}$ alkyl group, a phenyl group substituted with -F, a pentalenyl group, an indenyl group, a naphthyl group, an azulenyl group, a heptalenyl group, an indacenyl group, an acenaphthenyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, -Si($Q_{31}$)($Q_{32}$)($Q_{33}$), and -N($Q_{31}$)($Q_{32}$),

wherein $Q_{31}$ to $Q_{33}$ may each independently be selected from a $C_1$-$C_{10}$ alkyl group, a $C_1$-$C_{10}$ alkoxy group, a phenyl group, a biphenyl group, a terphenyl group, and a naphthyl group.

In one or more embodiments, xa1 to xa4 may each independently be 0, 1, or 2.

In one or more embodiments, xa5 may be 1, 2, 3, or 4.

In one or more embodiments, $R_{201}$ to $R_{204}$ and $Q_{201}$ may each independently be selected from a phenyl group, a biphenyl group, a terphenyl group, a pentalenyl group, an indenyl group, a naphthyl group, an azulenyl group, a heptalenyl group, an indacenyl group, an acenaphthenyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group; and

a phenyl group, a biphenyl group, a terphenyl group, a pentalenyl group, an indenyl group, a naphthyl group, an azulenyl group, a heptalenyl group, an indacenyl group, an acenaphthenyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group, each substituted with at least one selected from deuterium, -F, -Cl, -Br, -I, a hydroxyl group, a cyano group, a nitro group, an amidino group, a hydrazino group, a hydrazono group, a $C_1$-$C_{20}$ alkyl group, a $C_1$-$C_{20}$ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a $C_1$-$C_{10}$ alkyl group, a phenyl group substituted with -F, a pentalenyl group, an indenyl group, a naphthyl group, an azulenyl group, a heptalenyl group, an indacenyl group, an acenaphthenyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, $-Si(Q_{31})(Q_{32})(Q_{33})$, and $-N(Q_{31})(Q_{32})$,

wherein $Q_{31}$ to $Q_{33}$ may respectively be understood by referring to the descriptions therefor provided herein.

In one or more embodiments, in Formula 201, at least one of $R_{201}$ to $R_{203}$ may be selected from

a fluorenyl group, a spiro-bifluorenyl group, a carbazolyl group, a dibenzofuranyl group, and a dibenzothiophenyl group; and

a fluorenyl group, a spiro-bifluorenyl group, a carbazolyl group, a dibenzofuranyl group, and a dibenzothiophenyl group, each substituted with at least one selected from deuterium, -F, -Cl, -Br, -I, a hydroxyl group, a cyano group, a nitro group, an amidino group, a hydrazino group, a hydrazono group, a $C_1$-$C_{20}$ alkyl group, a $C_1$-$C_{20}$ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a $C_1$-$C_{10}$ alkyl group, a phenyl group substituted with -F, a naphthyl group, a fluorenyl group, a spiro-bifluorenyl group, a carbazolyl group, a dibenzofuranyl group, and a dibenzothiophenyl group,

but embodiments are not limited thereto.

In one or more embodiments, in Formula 202, i) $R_{201}$ and $R_{202}$ may be bound via a single bond, and/or ii) $R_{203}$ and $R_{204}$ may be bound via a single bond.

In one or more embodiments, in Formula 202, at least one of $R_{201}$ to $R_{204}$ may be selected from

a carbazolyl group; and

a carbazolyl group substituted with at least one selected from deuterium, -F, -Cl,-Br, -I, a hydroxyl group, a cyano group, a nitro group, an amidino group, a hydrazino group, a hydrazono group, a $C_1$-$C_{20}$ alkyl group, a $C_1$-$C_{20}$ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a $C_1$-$C_{10}$ alkyl group, a phenyl group substituted with -F, a naphthyl group, a fluorenyl group, a spiro-bifluorenyl group, a carbazolyl group, a dibenzofuranyl group, and a dibenzothiophenyl group, but embodiments are not limited thereto.

The compound represented by Formula 201 may be represented by Formula 201A:

Formula 201A

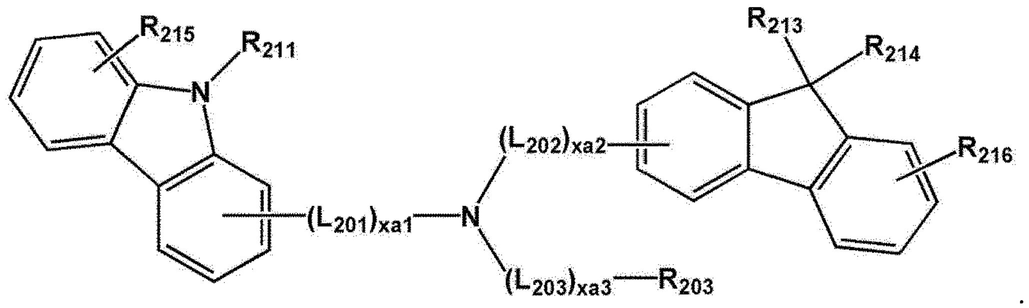

In some embodiments, the compound represented by Formula 201 may be represented by Formula 201A(1), but embodiments are not limited thereto:

Formula 201A(1)

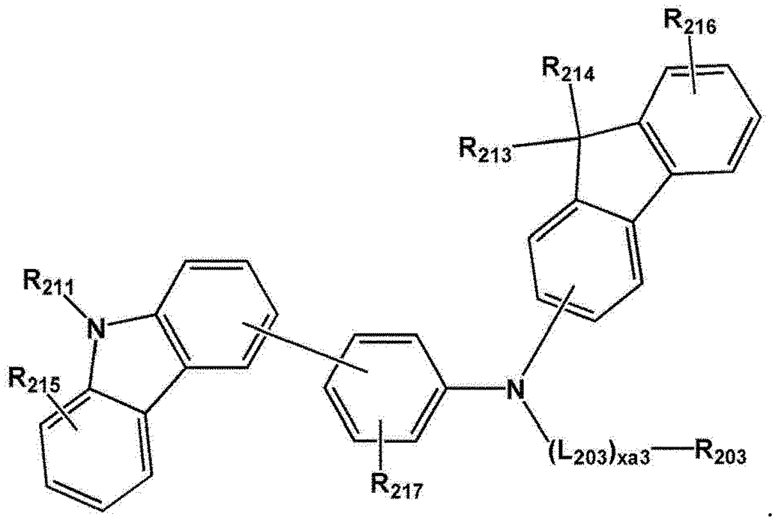

In some embodiments, the compound represented by Formula 201 may be represented by Formula 201A-1, but embodiments are not limited thereto:

Formula 201A-1

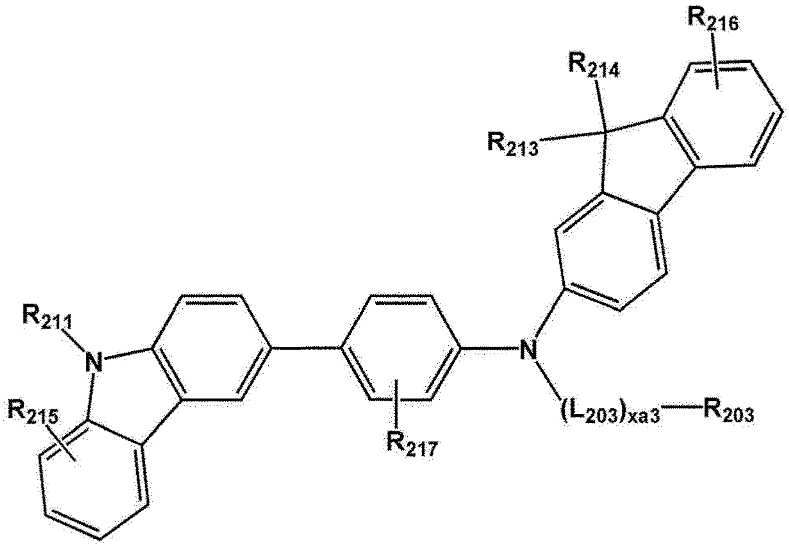

.

In some embodiments, the compound represented by Formula 202 may be represented by Formula 202A:

Formula 202A

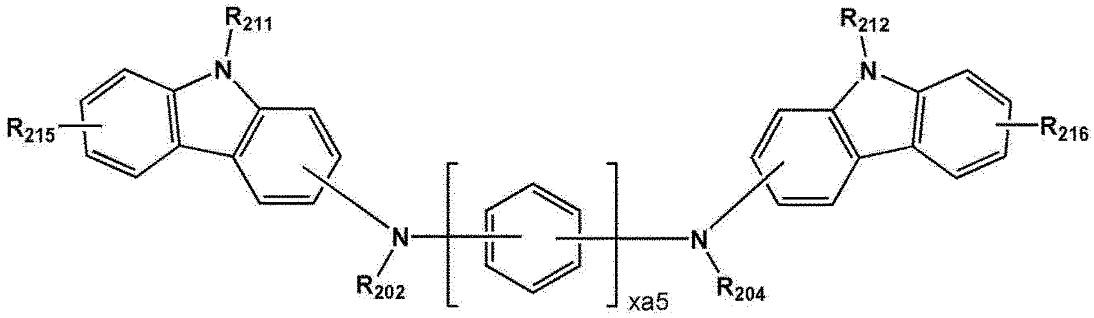

.

In some embodiments, the compound represented by Formula 202 may be represented by Formula 202A-1:

| Formula 202A-1

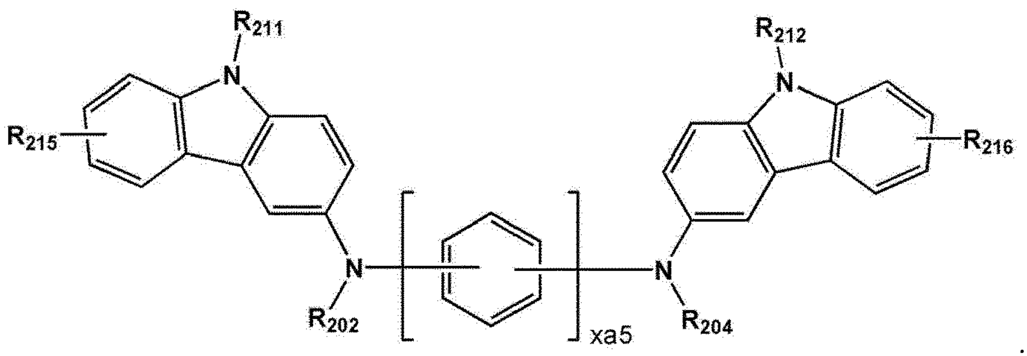

.

In Formulae 201A, 201A(1), 201A-1, 202A, and 202A-1,

$L_{201}$ to $L_{203}$, xa1 to xa3, xa5, and $R_{202}$ to $R_{204}$ may respectively be understood by referring to the descriptions therefor provided herein,

$R_{211}$ and $R_{212}$ may each be understood by referring to the descriptions for $R_{203}$ provided herein, and

$R_{213}$ to $R_{217}$ may each independently be selected from hydrogen, deuterium, -F,-Cl, -Br, -I, a hydroxyl group, a

cyano group, a nitro group, an amidino group, a hydrazino group, a hydrazono group, a $C_1$-$C_{20}$ alkyl group, a $C_1$-$C_{20}$ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a $C_1$-$C_{10}$ alkyl group, a phenyl group substituted with -F, a pentalenyl group, an indenyl group, a naphthyl group, an azulenyl group, a heptalenyl group, an indacenyl group, an acenaphthenyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group.

The hole transport region may include at least one compound selected from Compounds HT1 to HT39, but embodiments are not limited thereto:

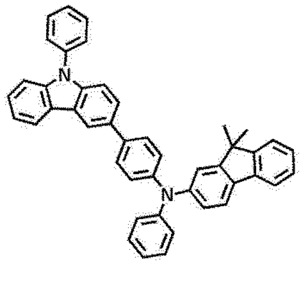

HT1

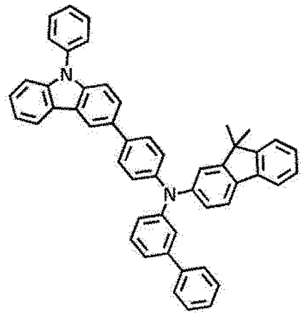

HT2

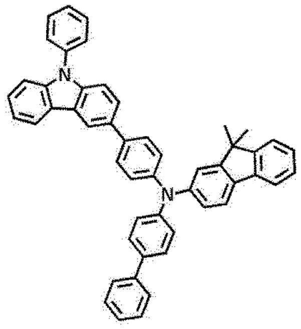

HT3

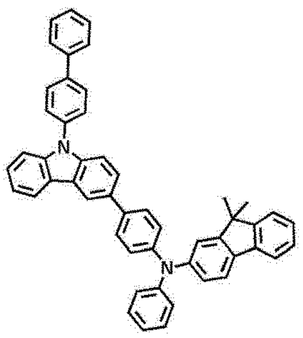

HT4

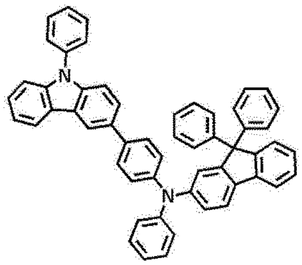

HT5

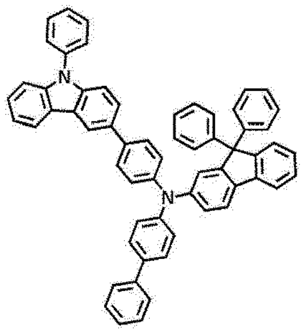

HT6

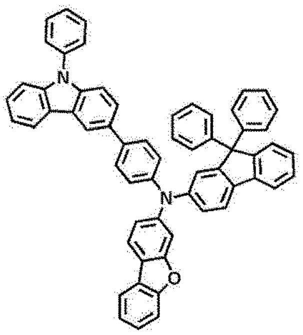

HT7

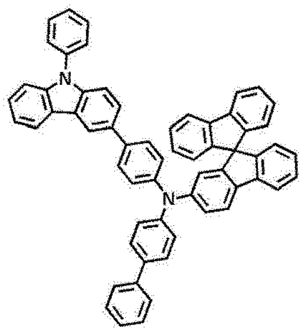

HT8

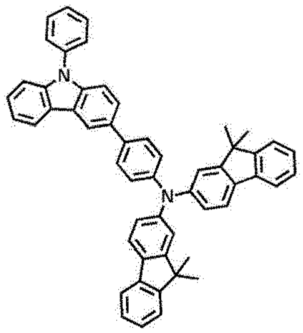

HT9

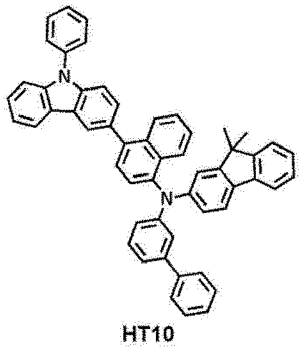
HT10

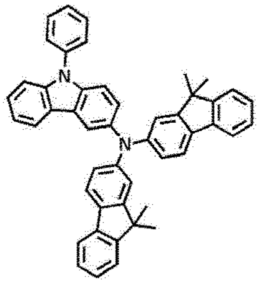
HT11

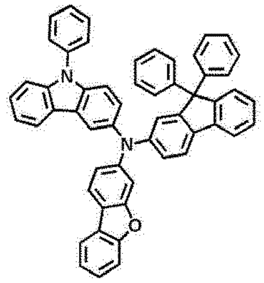
HT12

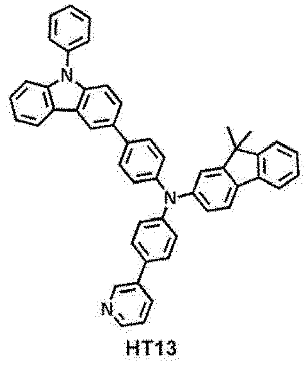
HT13

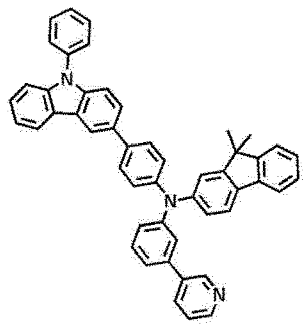
HT14

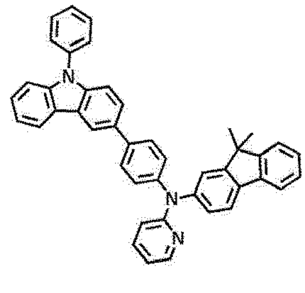
HT15

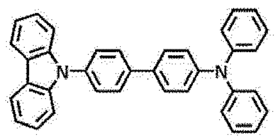
HT16

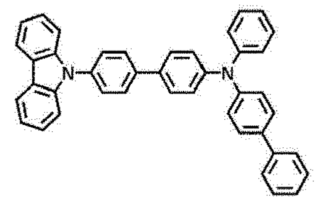
HT17

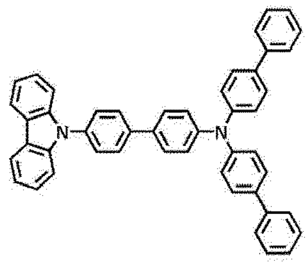
HT18

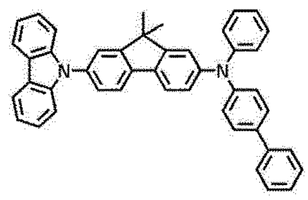
HT19

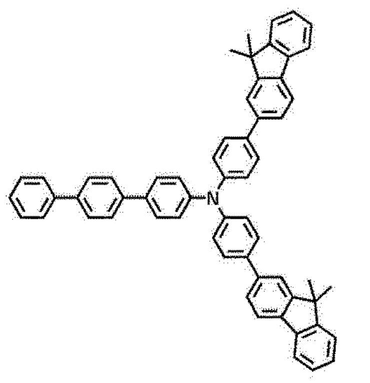
HT20

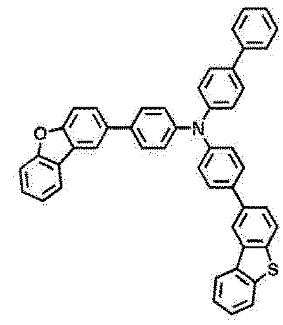
HT21

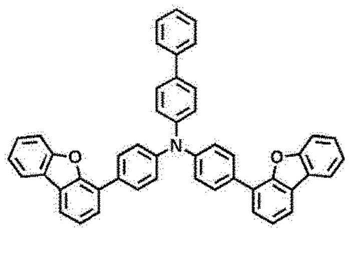

HT22

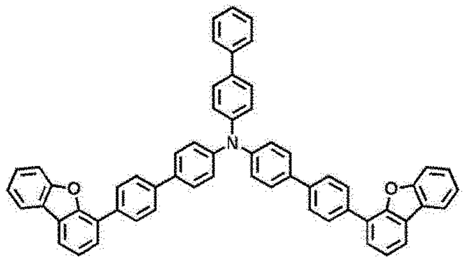

HT23

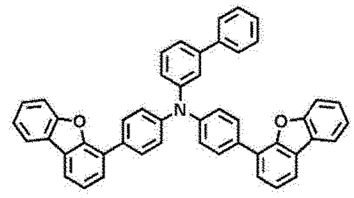

HT24

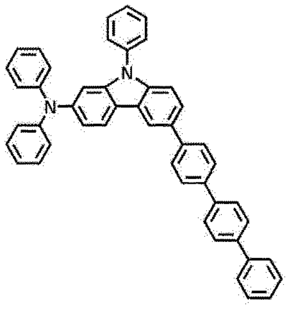

HT25

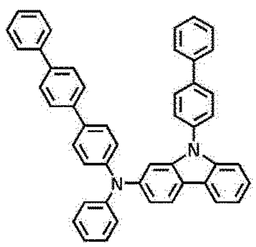

HT26

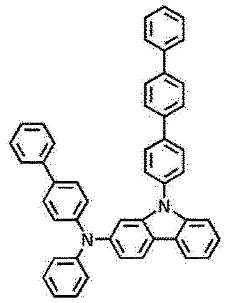

HT27

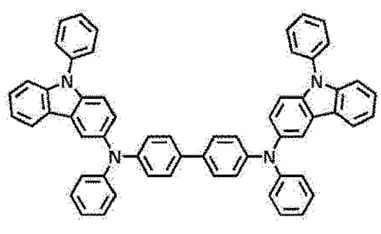

HT28

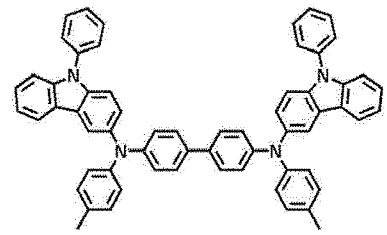

HT29

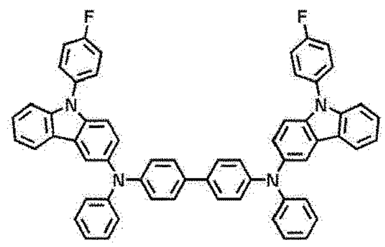

HT30

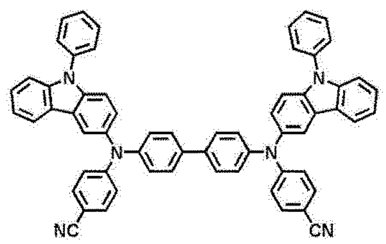

HT31

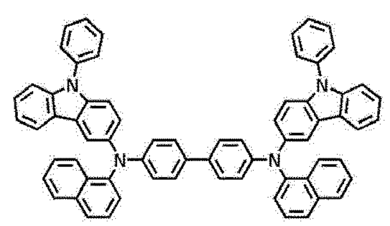

HT32

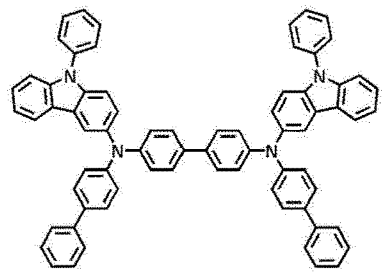

HT33

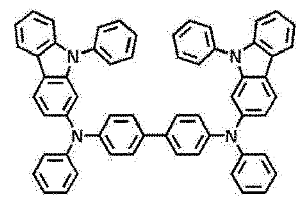

HT34

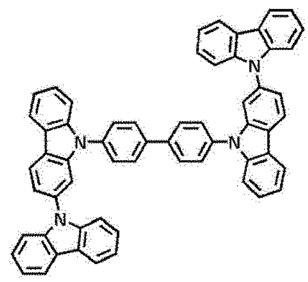

HT35

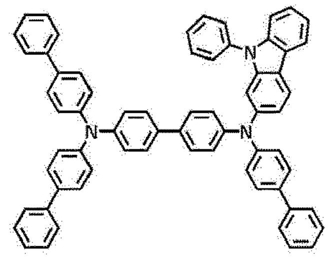

HT36

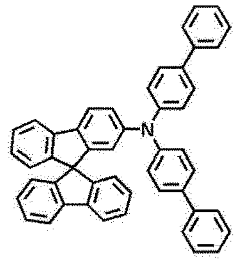

HT37

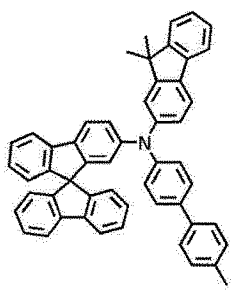

HT38

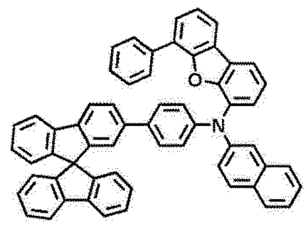

HT39 .

The thickness of the hole transport region may be in a range of about 100 (Angstroms) Å to about 10,000 Å, and in some embodiments, about 100 A to about 7000 Å, about 100 Å to about 5000 Å, about 100 Å to about 3000 Å, or about 100 Å to about 1,000 Å. When the hole transport region includes at least one selected from a hole injection layer and a hole transport layer, the thickness of the hole injection layer may be in a range of about 100 Å to about 9,000 Å, and in some embodiments, about 100 Å to about 7,000 Å, about 100 Å to about 5,000 Å, about 100 Å to about 3,000 Å, about 100 Å to about 2,000 Å, about 100 Å to about 1,000 Å or about 100 Å to about 200 Å, and the thickness of the hole transport layer may be in a range of about 50 Å to about 2,000 Å, and in some embodiments, about 100 Å to about 1,500 Å about 100 Å to about 1,000 Å, about 200 Å to about 800 Å, about 200 Å to about 600 Å, or about 300 Å to about 500 Å. When the thicknesses of the hole transport region, the hole injection layer, and the hole transport layer are within any of these ranges, excellent hole transport characteristics may be obtained without a substantial increase in driving voltage.

The emission auxiliary layer may increase light emission efficiency by compensating for an optical resonance distance according to the wavelength of light emitted by an emission layer. The electron blocking layer may reduce or eliminate the flow of electrons from an electron transport region. The emission auxiliary layer and the electron blocking layer may include the aforementioned materials.

p-dopant

The hole transport region may include a charge generating material as well as the aforementioned materials, to improve conductive properties of the hole transport region. The charge generating material may be substantially homogeneously or non-homogeneously dispersed in the hole transport region.

The charge generating material may include, for example, a p-dopant.

In some embodiments, the lowest unoccupied molecular orbital (LUMO) energy level of the p-dopant may be -3.5 eV or less.

The p-dopant may include at least one selected from a quinone derivative, a metal oxide, and a cyano group-containing compound, but embodiments are not limited thereto.

In some embodiments, the p-dopant may include at least one selected from

a quinone derivative, such as tetracyanoquinodimethane (TCNQ) or 2,3,5,6-tetrafluoro-7,7,8,8-tetracyanoquinodimethane (F4-TCNQ);
a metal oxide, such as tungsten oxide or molybdenum oxide;
1,4,5,8,9,11-hexaazatriphenylene-hexacarbonitrile (HAT-CN); and
a compound represented by Formula 221,

but embodiments are not limited thereto:

HAT-CN

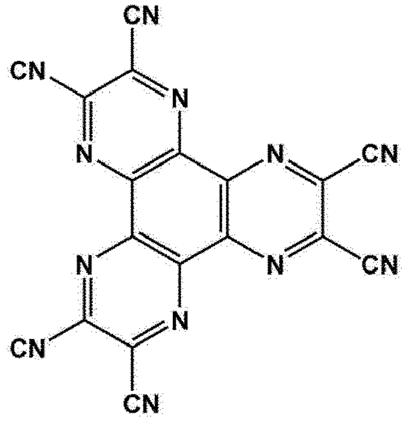

F4-TCNQ

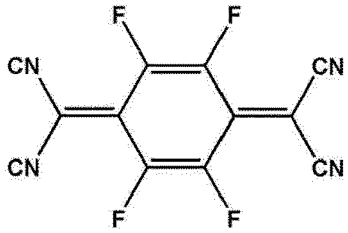

Formula 221

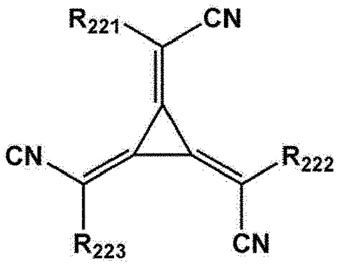

wherein, in Formula 221,

$R_{221}$ to $R_{223}$ may each independently be selected from a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ (*e.g.* $C_6$-$C_{32}$) aryl group, a substituted or unsubstituted $C_1$-$C_{60}$ (*e.g.* $C_1$-$C_{20}$) heteroaryl group, a substituted or unsubstituted monovalent non-aromatic condensed polycyclic group, and a substituted or unsubstituted monovalent non-aromatic condensed heteropolycyclic group, wherein at least one selected from $R_{221}$ to $R_{223}$ may include at least one substituent selected from a cyano group, -F, -Cl, -Br, -I, a $C_1$-$C_{20}$ alkyl group substituted with -F, a $C_1$-$C_{20}$ alkyl group substituted with -Cl, a $C_1$-$C_{20}$ alkyl group substituted with -Br, and a $C_1$-$C_{20}$ alkyl group substituted with -I.

Emission layer in organic layer 150

When the organic light-emitting device 10 is a full color organic light-emitting device, the emission layer may be patterned into a red emission layer, a green emission layer, or a blue emission layer, according to a sub-pixel. In one or more embodiments, the emission layer may have a stacked structure. The stacked structure may include two or more layers selected from a red emission layer, a green emission layer, and a blue emission layer. The two or more layers may be in direct contact with each other. In some embodiments, the two or more layers may be separated from each other. In one or more embodiments, the emission layer may include two or more materials. The two or more materials may include a red light-emitting material, a green light-emitting material, or a blue light-emitting material. The two or more materials may be mixed with each other in a single layer. The two or more materials mixed with each other in the single layer may emit white light.

The emission layer may include a host and a dopant.

The amount of the dopant in the emission layer may be, in general, in a range of about 0.01 parts to about 40

parts by weight, for example, about 0.01 parts to about 15 parts by weight, based on 100 parts by weight of the host, but embodiments are not limited thereto.

The thickness of the emission layer may be in a range of about 100 Å to about 1,000 Å, and in some embodiments, about 200 Å to about 600 Å or about 200 Å to about 300 Å. When the thickness of the emission layer is within any of these ranges, improved luminescence characteristics may be obtained without a substantial increase in driving voltage.

Host in emission layer

The host may include the heterocyclic compound described above.

In some embodiments, the host may further include a compound represented by Formula 301:

Formula 301 $[Ar_{301}]_{xb11}-[(L_{301})_{xb1}-R_{301}]_{xb21}$

wherein, in Formula 301,

$Ar_{301}$ may be selected from a substituted or unsubstituted $C_5$-$C_{60}$ carbocyclic group and a substituted or unsubstituted $C_1$-$C_{60}$ heterocyclic group,
xb11 may be 1, 2, or 3,
$L_{301}$ may be selected from a substituted or unsubstituted $C_3$-$C_{10}$ cycloalkylene group, a substituted or unsubstituted $C_1$-$C_{10}$ heterocycloalkylene group, a substituted or unsubstituted $C_3$-$C_{10}$ cycloalkenylene group, a substituted or unsubstituted $C_1$-$C_{10}$ heterocycloalkenylene group, a substituted or unsubstituted $C_6$-$C_{60}$ arylene group, a substituted or unsubstituted $C_1$-$C_{60}$ heteroarylene group, a substituted or unsubstituted divalent non-aromatic condensed polycyclic group, and a substituted or unsubstituted divalent non-aromatic condensed heteropolycyclic group,
xb1 may be an integer from 0 to 5,
$R_{301}$ may be selected from deuterium, -F, -Cl, -Br, -I, a hydroxyl group, a cyano group, a nitro group, an amidino group, a hydrazino group, a hydrazono group, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted C6-$C_{60}$ aryl group, a substituted or unsubstituted $C_6$-$C_{60}$ aryloxy group, a substituted or unsubstituted C6-C60 arylthio group, a substituted or unsubstituted $C_1$-$C_{60}$ heteroaryl group, a substituted or unsubstituted monovalent non-aromatic condensed polycyclic group, a substituted or unsubstituted monovalent non-aromatic condensed heteropolycyclic group, $-Si(Q_{301})(Q_{302})(Q_{303})$, $-N(Q_{301})(Q_{302})$, $-B(Q_{301})(Q_{302})$, $-C(=O)(Q_{301})$, $-S(=O)_2(Q_{301})$, and $-P(=O)(Q_{301})(Q_{302})$, and
xb21 may be an integer from 1 to 5,

wherein $Q_{301}$ to $Q_{303}$ may each independently be selected from a $C_1$-$C_{10}$ alkyl group, a $C_1$-$C_{10}$ alkoxy group, a phenyl group, a biphenyl group, a terphenyl group, and a naphthyl group, but embodiments are not limited thereto.

For example, $Ar_{301}$ may be a substituted or unsubstituted $C_6$-$C_{32}$ carbocyclic group or a substituted or unsubstituted $C_1$-$C_{12}$ heterocyclic group, but embodiments are not limited thereto.

In an embodiment, in Formula 301, $Ar_{301}$ may be selected from

a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, and a dibenzothiophene group; and
a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, and a dibenzothiophene group, each substituted with at least one selected from deuterium, -F, -Cl, -Br, -I, a hydroxyl group, a cyano group, a nitro group, an amidino group, a hydrazino group, a hydrazono group, a $C_1$-$C_{20}$ alkyl group, a $C_1$-$C_{20}$ alkoxy group, a phenyl group, a biphenyl group, a terphenyl group, a naphthyl group, $-Si(Q_{31})(Q_{32})(Q_{33})$,$-N(Q_{31})(Q_{32})$, $-B(Q_{31})(Q_{32})$, $-C(=O)(Q_{31})$, $-S(=O)_2(Q_{31})$, and $-P(=O)(Q_{31})(Q_{32})$,

wherein $Q_{31}$ to $Q_{33}$ may each independently be selected from a $C_1$-$C_{10}$ alkyl group, a $C_1$-$C_{10}$ alkoxy group, a phenyl group, a biphenyl group, a terphenyl group, and a naphthyl group, but embodiments are not limited thereto.

When xb11 in Formula 301 is 2 or greater, at least two $Ar_{301}$(s) may be bound via a single bond.

In one or more embodiments, the compound represented by Formula 301 may be represented by Formula 301-1 or 301-2:

Formula 301-1

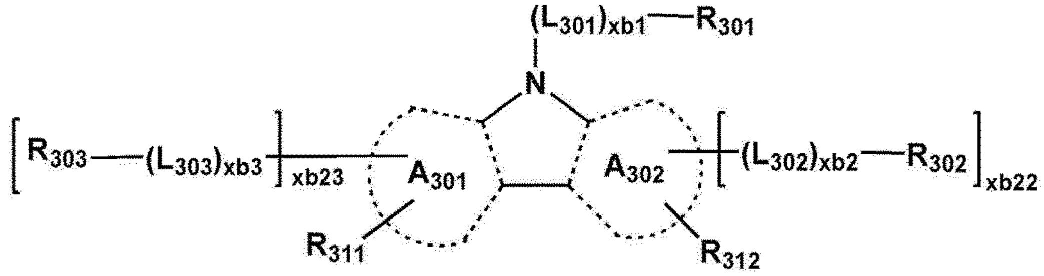

Formula 301-2

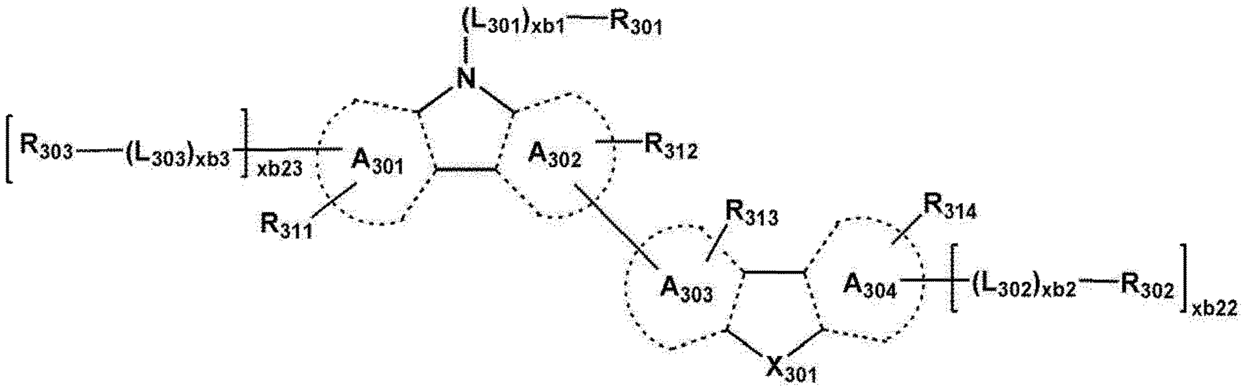

wherein, in Formulae 301-1 to 301-2,

$A_{301}$ to A304 may each independently be selected from a benzene group, a naphthalene group, a phenanthrene group, a fluoranthene group, a triphenylene group, a pyrene group, a chrysene group, a pyridine group, a pyrimidine group, an indene group, a fluorene group, a spiro-bifluorene group, a benzofluorene group, a dibenzofluorene group, an indole group, a carbazole group, a benzocarbazole group, a dibenzocarbazole group, a furan group, a benzofuran group, a dibenzofuran group, a naphthofuran group, a benzonaphthofuran group, a dinaphthofuran group, a thiophene group, a benzothiophene group, a dibenzothiophene group, a naphthothiophene group, a benzonapthothiophene group, and a dinaphthothiophene group,

$X_{301}$ may be O, S, or N-[$(L_{304})_{xb4}$-$R_{304}$],

$R_{311}$ to $R_{314}$ may each independently be selected from hydrogen, deuterium, -F,-Cl, -Br, -I, a hydroxyl group, a cyano group, a nitro group, an amidino group, a hydrazino group, a hydrazono group, a $C_1$-$C_{20}$ alkyl group, a $C_1$-$C_{20}$ alkoxy group, a phenyl group, a biphenyl group, a terphenyl group, a naphthyl group -Si($Q_{31}$)($Q_{32}$)($Q_{33}$), -N($Q_{31}$)($Q_{32}$), -B($Q_{31}$)($Q_{32}$),-C(=O)($Q_{31}$), -S(=O)$_2$($Q_{31}$), and -P(=O)($Q_{31}$)($Q_{32}$),

xb22 and xb23 may each independently be 0, 1, or 2,

$L_{301}$, xbl, $R_{301}$, and $Q_{31}$ to $Q_{33}$ may respectively be understood by referring to the descriptions therefor provided herein,

$L_{302}$ to $L_{304}$ may each be understood by referring to the descriptions for $L_{301}$ provided herein,

xb2 to xb4 may each be understood by referring to the descriptions for xb1 provided herein, and

$R_{302}$ to $R_{304}$ may each be understood by referring to the descriptions for $R_{301}$ provided herein.

For example, $L_{301}$ to $L_{304}$ in Formulae 301, 301-1, and 301-2 may each independently be selected from: a substituted or unsubstituted $C_3$-$C_{10}$ cycloalkylene group, a substituted or unsubstituted $C_1$-$C_{10}$ heterocycloalkylene group, a substituted or unsubstituted C3-$C_{10}$ cycloalkenylene group, a substituted or unsubstituted $C_1$-$C_{10}$ heterocycloalkenylene group, a substituted or unsubstituted $C_6$-$C_{30}$ arylene group, a substituted or unsubstituted $C_1$-$C_{20}$ heteroarylene group, a substituted or unsubstituted divalent non-aromatic condensed polycyclic group, and a substituted or unsubstituted divalent non-aromatic condensed heteropolycyclic group, but embodiments are not limited thereto.

In some embodiments, in Formulae 301, 301-1, and 301-2, $L_{301}$ to $L_{304}$ may each independently be selected from

a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an imidazopyridinylene group, an imidazopyrimidinylene group, and an azacarbazolylene group; and

a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an imidazopyridinylene group, an imidazopyrimidinylene group, and an azacarbazolylene group, each substituted with at least one selected from deuterium, -F, -Cl, -Br, -I, a hydroxyl group, a cyano group, a nitro group, an amidino group, a hydrazino group, a hydrazono group, a $C_1$-$C_{20}$ alkyl group, a $C_1$-$C_{20}$ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, an azacarbazolyl group, $-Si(Q_{31})(Q_{32})(Q_{33})$, $-N(Q_{31})(Q_{32})$, $-B(Q_{31})(Q_{32})$, $-C(=O)(Q_{31})$, $-S(=O)_2(Q_{31})$, and $-P(=O)(Q_{31})(Q_{32})$,

wherein $Q_{31}$ to $Q_{33}$ may respectively be understood by referring to the descriptions therefor provided herein.

As another example, $R_{301}$ to $R_{304}$ in Formulae 301, 301-1, and 301-2 may each independently be selected from: deuterium, -F, -Cl, -Br, -I, a hydroxyl group, a cyano group, a nitro group, an amidino group, a hydrazino group, a hydrazono group, a substituted or unsubstituted $C_1$-$C_{60}$ (*e.g.* $C_1$-$C_{10}$) alkyl group, a substituted or unsubstituted $C_2$-$C_{60}$ (*e.g.* $C_2$-$C_{10}$) alkenyl group, a substituted or unsubstituted $C_2$-$C_{60}$ (*e.g.* $C_2$-$C_{10}$) alkynyl group, a substituted or unsubstituted $C_1$-$C_{60}$ (*e.g.* $C_1$-$C_{10}$) alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{30}$ aryl group, a substituted or unsubstituted $C_6$-$C_{30}$ aryloxy group, a substituted or unsubstituted $C_6$-$C_{30}$ arylthio group, a substituted or unsubstituted $C_1$-$C_{20}$ heteroaryl group, a substituted or unsubstituted monovalent non-aromatic condensed polycyclic group, a substituted or unsubstituted monovalent non-aromatic condensed heteropolycyclic group, $-Si(Q_{301})(Q_{302})(Q_{303})$, $-N(Q_{301})(Q_{302})$, $-B(Q_{301})(Q_{302})$, $-C(=O)(Q_{301})$, $-S(=O)_2(Q_{301})$, and $-P(=O)(Q_{301})(Q_{302})$, but embodiments are not limited

thereto.

In some embodiments, in Formulae 301, 301-1, and 301-2, $R_{301}$ to $R_{304}$ may each independently be selected from

a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group; and

a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group, each substituted with at least one selected from deuterium,-F, -Cl, -Br, -I, a hydroxyl group, a cyano group, a nitro group, an amidino group, a hydrazino group, a hydrazono group, a $C_1$-$C_{20}$ alkyl group, a $C_1$-$C_{20}$ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, an azacarbazolyl group,-$Si(Q_{31})(Q_{32})(Q_{33})$, -$N(Q_{31})(Q_{32})$, -$B(Q_{31})(Q_{32})$, -$C(=O)(Q_{31})$, -$S(=O)_2(Q_{31})$, and -$P(=O)(Q_{31})(Q_{32})$,

wherein $Q_{31}$ to $Q_{33}$ may respectively be understood by referring to the descriptions therefor provided herein.

In some embodiments, the host may further include an alkaline earth metal complex. For example, the host may include a complex selected from a Be complex (for example, Compound H55), a Mg complex, and a Zn complex. For example, the host may include a beryllium (Be) complex, e.g., Compound H55, a magnesium (Mg) complex, or a zinc (Zn) complex.

The host may further include at least one selected from 9,10-di(2-naphthyl)anthracene (ADN), 2-methyl-9,10-bis(naphthalen-2-yl)anthracene (MADN), 9,10-di-(2-naphthyl)-2-t-butyl-anthracene (TBADN), 4,4'-bis(N-carbazolyl)-1,1'-biphenyl (CBP), 1,3-di-9-carbazolylbenzene (mCP), and 1,3,5-tri(carbazol-9-yl)benzene (TCP), but embodiments are not limited thereto:

Phosphorescent dopant included in emission layer of organic layer 150

The phosphorescent dopant may include the organometallic compound described above.

In some embodiments, the phosphorescent dopant may further include a compound represented by Formula 401:

Formula 401 $M(L_{401})_{xc1}(L_{402})_{xc2}$

Formula 402

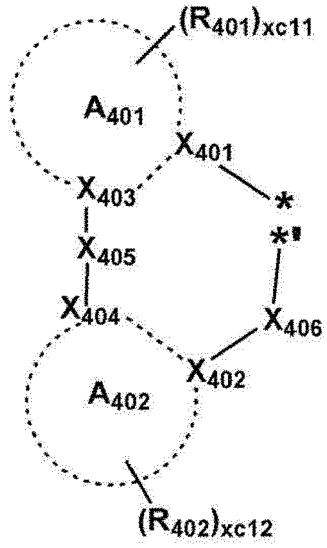

wherein, in Formulae 401 and 402,

M may be selected from iridium (Ir), platinum (Pt), palladium (Pd), osmium (Os), titanium (Ti), zirconium (Zr), hafnium (Hf), europium (Eu), terbium (Tb), rhodium (Rh), and thulium (Tm),

$L_{401}$ may be selected from ligands represented by Formula 402, and xc1 may be 1, 2, or 3; when xc1 is 2 or greater, at least two $L_{401}$(s) may be identical to or different from each other,

$L_{402}$ may be an organic ligand, and xc2 may be an integer selected from 0 to 4; when xc2 is 2 or greater, at least two $L_{402}$(s) may be identical to or different from each other,

$X_{401}$ to $X_{404}$ may each independently be a nitrogen or a carbon,

$X_{401}$ and $X_{403}$ may be bound to each other via a single bond or a double bond, $X_{402}$ and $X_{404}$ may be bound to each other via a single bond or a double bond,

$A_{401}$ and A402 may each independently be a $C_5$-$C_{60}$ (*e.g.* $C_5$-$C_{30}$) carbocyclic group or a $C_1$-$C_{60}$ (*e.g.* $C_1$-$C_{20}$) heterocyclic group,

$X_{405}$ may be selected from a single bond, *-O-*', *-S-*', *-C(=O)-*', *-N($Q_{411}$)-*', *-C($Q_{411}$)($Q_{412}$)-*', *-C($Q_{411}$)=C($Q_{412}$)-*', *-C($Q_{411}$)=*', and *=C=*', wherein $Q_{411}$ and $Q_{412}$ may be selected from hydrogen, deuterium, a $C_1$-$C_{20}$ alkyl group, a $C_1$-$C_{20}$ alkoxy group, a phenyl group, a biphenyl group, a terphenyl group, and a naphthyl group,

$X_{406}$ may be a single bond, O, or S,

$R_{401}$ and $R_{402}$ may each independently be selected from hydrogen, deuterium, -F, - Cl, -Br, -I, a hydroxyl group, a cyano group, a nitro group, an amidino group, a hydrazino group, a hydrazono group, a substituted or unsubstituted $C_1$-$C_{20}$ alkyl group, a substituted or unsubstituted $C_1$-$C_{20}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ (*e.g.* $C_6$-$C_{30}$) aryl group, a substituted or unsubstituted $C_6$-$C_{60}$ (*e.g.* $C_6$-$C_{30}$) aryloxy group, a substituted or unsubstituted $C_6$-$C_{60}$ (*e.g.* $C_6$-$C_{30}$) arylthio group, a substituted or unsubstituted $C_1$-$C_{60}$ (*e.g.* $C_1$-$C_{20}$) heteroaryl group, a substituted or unsubstituted monovalent non-aromatic condensed polycyclic group, a substituted or unsubstituted monovalent non-aromatic condensed heteropolycyclic group, -Si($Q_{401}$)($Q_{402}$)($Q_{403}$), -N($Q_{401}$)($Q_{402}$),-B($Q_{401}$)($Q_{402}$), -C(=O)($Q_{401}$), -S(=O)$_2$($Q_{401}$), and -P(=O)($Q_{401}$)($Q_{402}$), wherein $Q_{401}$ to $Q_{403}$ may each independently be selected from a $C_1$-$C_{10}$ alkyl group, a $C_1$-$C_{10}$ alkoxy group, a $C_6$-$C_{20}$ aryl group, and a $C_1$-$C_{20}$ heteroaryl group,

xc11 and xc12 may each independently be an integer from 0 to 10, and

* and *' in Formula 402 each indicate a binding site to M in Formula 401.

In an embodiment, in Formula 402, $A_{401}$ and $A_{402}$ may each independently be selected from a benzene group, a naphthalene group, a fluorene group, a spiro-bifluorene group, an indene group, a pyrrole group, a thiophene group, a furan group, an imidazole group, a pyrazole group, a thiazole group, an isothiazole group, an oxazole group, an isoxazole group, a pyridine group, a pyrazine group, a pyrimidine group, a pyridazine group, a quinoline group, an isoquinoline group, a benzoquinoline group, a quinoxaline group, a quinazoline group, a carbazole group, a benzimidazole group, a benzofuran group, a benzothiophene group, an isobenzothiophene group, a benzoxazole group, an isobenzoxazole group, a triazole group, a tetrazole group, an oxadiazole group, a triazine group, a dibenzofuran group, and a dibenzothiophene group.

In one or more embodiments, in Formula 402, i) $X_{401}$ may be nitrogen, and $X_{402}$ may be carbon, or ii) $X_{401}$ and $X_{402}$ may each be nitrogen.

In one embodiment, in Formula 402, $R_{401}$ and $R_{402}$ may each independently be selected from

hydrogen, deuterium, -F, -Cl, -Br, -I, a hydroxyl group, a cyano group, a nitro group, an amidino group, a hydrazino group, a hydrazono group, a $C_1$-$C_{20}$ alkyl group, and a $C_1$-C20 alkoxy group;

a $C_1$-$C_{20}$ alkyl group and a $C_1$-$C_{20}$ alkoxy group, each substituted with at least one selected from deuterium, -F, -Cl, -Br, -I, a hydroxyl group, a cyano group, a nitro group, an amidino group, a hydrazino group, a hydrazono group, a phenyl group, a naphthyl group, a cyclopentyl group, a cyclohexyl group, an adamantyl group, a norbornanyl group, and a norbornenyl group;

a cyclopentyl group, a cyclohexyl group, an adamantyl group, a norbornanyl group, a norbornenyl group, a phenyl group, a biphenyl group, a terphenyl group, a naphthyl group, a fluorenyl group, a pyridinyl group, a pyrazinyl group, a pyrimidinyl group, a pyridazinyl group, a triazinyl group, a quinolinyl group, an isoquinolinyl group, a quinoxalinyl group, a quinazolinyl group, a carbazolyl group, a dibenzofuranyl group, and a dibenzothiophenyl group;

a cyclopentyl group, a cyclohexyl group, an adamantyl group, a norbornanyl group, a norbornenyl group, a phenyl group, a biphenyl group, a terphenyl group, a naphthyl group, a fluorenyl group, a pyridinyl group, a pyrazinyl group, a pyrimidinyl group, a pyridazinyl group, a triazinyl group, a quinolinyl group, an isoquinolinyl group, a quinoxalinyl group, a quinazolinyl group, a carbazolyl group, a dibenzofuranyl group, and a dibenzothiophenyl group, each substituted with at least one selected from deuterium, -F, -Cl, -Br, -I, a hydroxyl group, a cyano group, a nitro group, an amidino group, a hydrazino group, a hydrazono group, a $C_1$-$C_{20}$ alkyl group, a $C_1$-$C_{20}$ alkoxy group, a cyclopentyl group, a cyclohexyl group, an adamantyl group, a norbornanyl group, a norbornenyl group, a phenyl group, a biphenyl group, a terphenyl group, a naphthyl group, a fluorenyl group, a pyridinyl group, a pyrazinyl group, a pyrimidinyl group, a pyridazinyl group, a triazinyl group, a quinolinyl group, an isoquinolinyl group, a quinoxalinyl group, a quinazolinyl group, a carbazolyl group, a dibenzofuranyl group, and a dibenzothiophenyl group; and

$-Si(Q_{401})(Q_{402})(Q_{403})$, $-N(Q_{401})(Q_{402})$, $-B(Q_{401})(Q_{402})$, $-C(=O)(Q_{401})$,$-S(=O)_2(Q_{401})$, and $-P(=O)(Q_{401})(Q_{402})$,

wherein $Q_{401}$ to $Q_{403}$ may each independently be selected from a $C_1$-$C_{10}$ alkyl group, a $C_1$-$C_{10}$ alkoxy group, a phenyl group, a biphenyl group, and a naphthyl group, but embodiments are not limited thereto.

In one or more embodiments, when xc1 in Formula 401 is 2 or greater, two $A_{401}$(s) of at least two $L_{401}$(s) may optionally be linked via $X_{407}$ as a linking group; or two $A_{402}$(s) may optionally be linked via $X_{408}$ as a linking group (see Compounds PD1 to PD4 and PD7). $X_{407}$ and $X_{408}$ may each independently be selected from a single bond, *-O-*', *-S-*', *-C(=O)-*', $*-N(Q_{413})-*'$, $*-C(Q_{413})(Q_{414})-*'$, and $*-C(Q_{413})=C(Q_{414})-*'$, wherein $Q_{413}$ and $Q_{414}$ may each independently be hydrogen, deuterium, a $C_1$-$C_{20}$ alkyl group, a $C_1$-$C_{20}$ alkoxy group, a phenyl group, a biphenyl group, a terphenyl group, or a naphthyl group, but embodiments are not limited thereto.

$L_{402}$ in Formula 401 may be any suitable monovalent, divalent, or trivalent organic ligand. For example, $L_{402}$ may be selected from halogen, diketone (e.g., acetylacetonate), a carboxylic acid (e.g., picolinate), -C(=O), isonitrile, -CN, and phosphorus (e.g., phosphine or phosphite), but embodiments are not limited thereto.

In some embodiments, the phosphorescent dopant may further include, for example, at least one selected from Compounds PD1 to PD25, but embodiments are not limited thereto:

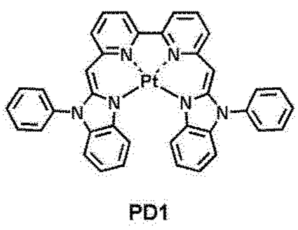

PD1

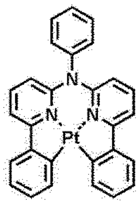

PD2

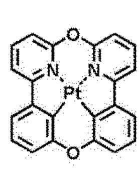

PD3

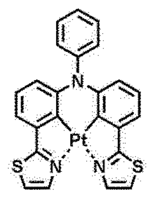

PD4

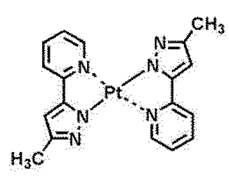

PD5

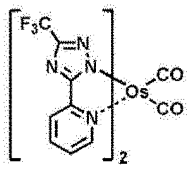
PD6

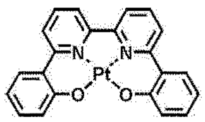
PD7

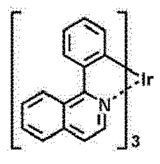
PD8

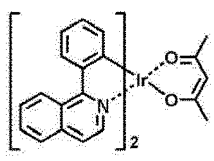
PD9

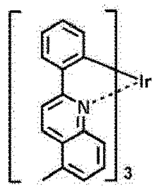
PD10

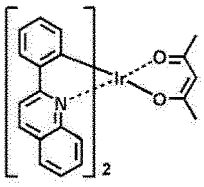
PD11

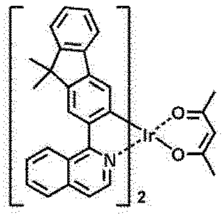
PD12

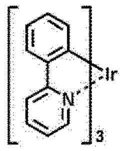
PD13

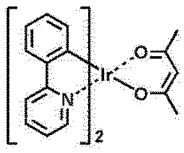
PD14

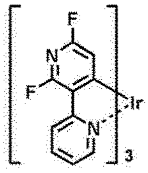
PD15

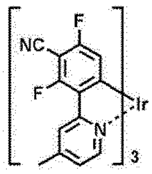
PD16

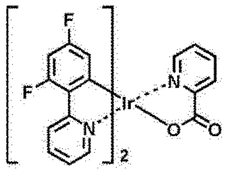
PD17

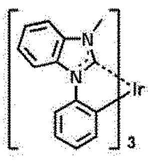
PD18

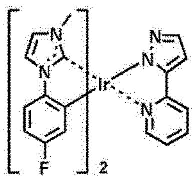
PD19

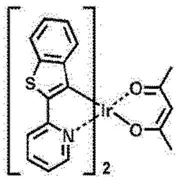
PD20

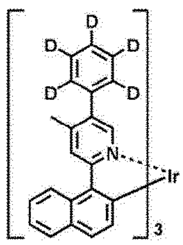
PD21

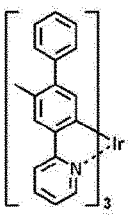
PD22

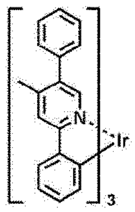
PD23

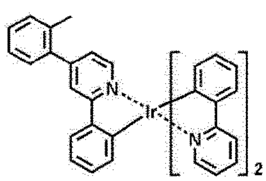
PD24

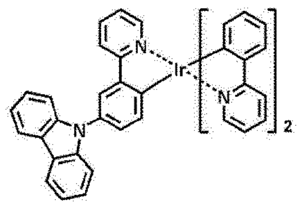
PD25

.

Electron transport region in organic layer 150

The electron transport region may have i) a single-layered structure consisting of a single layer consisting of a single material, ii) a single-layered structure consisting of a single layer including a plurality of different materials, or iii) a multi-layered structure each having a plurality of layers, each having a plurality of different materials.

The electron transport region may include at least one selected from a buffer layer, a hole blocking layer, an electron control layer, an electron transport layer, and an electron injection layer, but embodiments are not limited thereto.

In some embodiments, the electron transport region may have an electron transport layer/electron injection layer structure, a hole blocking layer/electron transport layer/electron injection layer structure, an electron control layer/electron transport layer/electron injection layer structure, or a buffer layer/electron transport layer/electron injection layer structure, wherein layers of each structure are sequentially stacked on the emission layer in each stated order, but embodiments are not limited thereto.

The electron transport region (for example, the buffer layer, the hole blocking layer, the electron control layer, or the electron transport layer in the electron transport region) may include a metal-free compound including at least one $\pi$ electron-depleted nitrogen-containing ring.

The term "$\pi$ electron-depleted nitrogen-containing ring" as used herein refers to a $C_1$-$C_{60}$ (*e.g.* a $C_1$-$C_{30}$) heterocyclic group having at least one *-N=*' moiety as a ring-forming moiety.

For example, the "$\pi$ electron-depleted nitrogen-containing ring" may be i) a 5-membered to 7-membered heteromonocyclic group having at least one *-N=*' moiety, ii) a heteropolycyclic group in which at least two 5-membered

to 7-membered heteromonocyclic groups, each having at least one *-N=*' moiety, are condensed, or iii) a heteropolycyclic group in which at least one of a 5-membered to 7-membered heteromonocyclic group, each having at least one *-N=*' moiety, is condensed with at least one $C_5$-$C_{60}$ (*e.g.* a $C_5$-$C_{30}$) carbocyclic group.

Examples of the $\pi$ electron-depleted nitrogen-containing ring may include imidazole, pyrazole, thiazole, isothiazole, oxazole, isoxazole, pyridine, pyrazine, pyrimidine, pyridazine, indazole, purine, quinoline, isoquinoline, benzoquinoline, phthalazine, naphthyridine, quinoxaline, quinazoline, cinnoline, phenanthridine, acridine, phenanthroline, phenazine, benzimidazole, isobenzothiazole, benzoxazole, isobenzoxazole, triazole, tetrazole, oxadiazole, triazine, thiadiazole, imidazopyridine, imidazopyrimidine, and azacarbazole, but embodiments are not limited thereto.

In some embodiments, the electron transport region may include a compound represented by Formula 601:

Formula 601 $[Ar_{601}]_{xe11}$-$[(L_{601})_{xe1}$-$R_{601}]_{xe21}$

wherein, in Formula 601,

$Ar_{601}$ may be selected from a substituted or unsubstituted $C_5$-$C_{60}$ carbocyclic group and a substituted or unsubstituted $C_1$-$C_{60}$ heterocyclic group,
xe11 may be 1, 2, or 3,
$L_{601}$ may be selected from a substituted or unsubstituted $C_3$-$C_{10}$ cycloalkylene group, a substituted or unsubstituted $C_1$-$C_{10}$ heterocycloalkylene group, a substituted or unsubstituted $C_3$-$C_{10}$ cycloalkenylene group, a substituted or unsubstituted $C_1$-$C_{10}$ heterocycloalkenylene group, a substituted or unsubstituted $C_6$-$C_{60}$ arylene group, a substituted or unsubstituted $C_1$-$C_{60}$ heteroarylene group, a substituted or unsubstituted divalent non-aromatic condensed polycyclic group, and a substituted or unsubstituted divalent non-aromatic condensed heteropolycyclic group,
xe1 may be an integer from 0 to 5,
$R_{601}$ may be selected from a substituted or unsubstituted $C_3$-$C_{10}$ cycloalkyl group, a substituted or unsubstituted $C_1$-$C_{10}$ heterocycloalkyl group, a substituted or unsubstituted C3-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted monovalent non-aromatic condensed polycyclic group, a substituted or unsubstituted monovalent non-aromatic condensed heteropolycyclic group, $-Si(Q_{601})(Q_{602})(Q_{603})$, $-C(=O)(Q_{601})$, $-S(=O)_2(Q_{601})$, and $-P(=O)(Q_{601})(Q_{602})$,
wherein $Q_{601}$ to $Q_{603}$ may each independently be a $C_1$-$C_{10}$ alkyl group, a $C_1$-$C_{10}$ alkoxy group, a phenyl group, a biphenyl group, a terphenyl group, or a naphthyl group, and
xe21 may be an integer from 1 to 5.

In an embodiment, at least one selected from $Ar_{601}$(s) in the number of xe11 and $R_{601}$(s) in the number of xe21 may include the $\pi$ electron-depleted nitrogen-containing ring.

For example, $Ar_{601}$ may be a substituted or unsubstituted $C_5$-$C_{30}$ carbocyclic group or a substituted or unsubstituted $C_1$-$C_{20}$ heterocyclic group, but embodiments are not limited thereto.

In an embodiment, in Formula 601, $Ar_{601}$ may be selected from

a benzene group, a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, a dibenzothiophene group, a carbazole group, an imidazole group, a pyrazole group, a thiazole group, an isothiazole group, an oxazole group, an isoxazole group, a pyridine group, a pyrazine group, a pyrimidine group, a pyridazine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, an isobenzothiazole group, a benzoxazole group, an isobenzoxazole group, a triazole group, a tetrazole group, an oxadiazole group, a triazine group, a thiadiazole group, an imidazopyridine group, an imidazopyrimidine group, and an azacarbazole group; and
a benzene group, a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, a dibenzothiophene group, a carbazole group, an imidazole group, a pyrazole group, a thiazole group, an isothiazole group, an oxazole group, an isoxazole group, a pyridine group, a pyrazine group, a pyrimidine group, a pyridazine group, an indazole group, a purine group, a quinoline group, an isoquinoline group, a benzoquinoline group, a phthalazine group, a naphthyridine group, a

quinoxaline group, a quinazoline group, a cinnoline group, a phenanthridine group, an acridine group, a phenanthroline group, a phenazine group, a benzimidazole group, an isobenzothiazole group, a benzoxazole group, an isobenzoxazole group, a triazole group, a tetrazole group, an oxadiazole group, a triazine group, a thiadiazole group, an imidazopyridine group, an imidazopyrimidine group, and an azacarbazole group, each substituted with at least one selected from deuterium, -F, -Cl, -Br, -I, a hydroxyl group, a cyano group, a nitro group, an amidino group, a hydrazino group, a hydrazono group, a $C_1$-$C_{20}$ alkyl group, a $C_1$-$C_{20}$ alkoxy group, a phenyl group, a biphenyl group, a terphenyl group, a naphthyl group, -Si($Q_{31}$)($Q_{32}$)($Q_{33}$), -S(=O)$_2$($Q_{31}$), and -P(=O)($Q_{31}$)($Q_{32}$),

wherein $Q_{31}$ to $Q_{33}$ may each independently be selected from a $C_1$-$C_{10}$ alkyl group, a $C_1$-$C_{10}$ alkoxy group, a phenyl group, a biphenyl group, a terphenyl group, and a naphthyl group.

When xe11 in Formula 601 is 2 or greater, at least two $Ar_{601}$(s) may be bound via a single bond.

In one or more embodiments, $Ar_{601}$ in Formula 601 may be an anthracene group.

In some embodiments, the compound represented by Formula 601 may be represented by Formula 601-1:

Formula 601-1

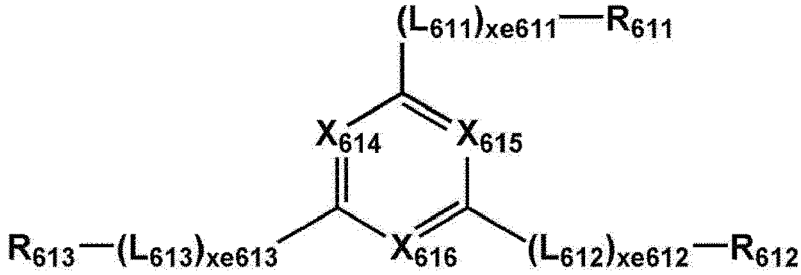

wherein, in Formula 601-1,

$X_{614}$ may be N or C($R_{614}$), $X_{615}$ may be N or C($R_{615}$), $X_{616}$ may be N or C($R_{616}$), at least one selected from $X_{614}$ to $X_{616}$ may be N,
$L_{611}$ to $L_{613}$ may each independently be understood by referring to the descriptions for $L_{601}$ provided herein,
xe611 to xe613 may each independently be understood by referring to the descriptions for xe1 provided herein,
$R_{611}$ to $R_{613}$ may each independently be understood by referring to the descriptions for $R_{601}$ provided herein, and
$R_{614}$ to $R_{616}$ may each independently be selected from hydrogen, deuterium, -F, - Cl, -Br, -I, a hydroxyl group, a cyano group, a nitro group, an amidino group, a hydrazino group, a hydrazono group, a $C_1$-$C_{20}$ alkyl group, a $C_1$-$C_{20}$ alkoxy group, a phenyl group, a biphenyl group, a terphenyl group, and a naphthyl group.

For example, $L_{601}$ and $L_{611}$ to $L_{613}$ in Formulae 601 and 601-1 may each independently be selected from: a substituted or unsubstituted $C_3$-$C_{10}$ cycloalkylene group, a substituted or unsubstituted $C_1$-$C_{10}$ heterocycloalkylene group, a substituted or unsubstituted $C_3$-$C_{10}$ cycloalkenylene group, a substituted or unsubstituted $C_1$-$C_{10}$ heterocycloalkenylene group, a substituted or unsubstituted $C_6$-$C_{30}$ arylene group, a substituted or unsubstituted $C_1$-$C_{20}$ heteroarylene group, a substituted or unsubstituted divalent non-aromatic condensed polycyclic group, and a substituted or unsubstituted divalent non-aromatic condensed heteropolycyclic group, but embodiments are not limited thereto.

In an embodiment, in Formulae 601 and 601-1, $L_{601}$ and $L_{611}$ to $L_{613}$ may each independently be selected from

a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an

imidazopyridinylene group, an imidazopyrimidinylene group, and an azacarbazolylene group; and
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an imidazopyridinylene group, an imidazopyrimidinylene group, and an azacarbazolylene group, each substituted with at least one selected from deuterium, -F, -Cl, -Br, -I, a hydroxyl group, a cyano group, a nitro group, an amidino group, a hydrazino group, a hydrazono group, a $C_1$-$C_{20}$ alkyl group, a $C_1$-$C_{20}$ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group,

but embodiments are not limited thereto.

In one or more embodiments, in Formulae 601 and 601-1, xe1 and xe611 to xe613 may each independently be 0, 1, or 2.

For example, $R_{601}$ and $R_{611}$ to $R_{613}$ in Formulae 601 and 601-1 may each independently be selected from: a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{30}$ aryl group, a substituted or unsubstituted $C_6$-$C_{30}$ aryloxy group, a substituted or unsubstituted $C_6$-$C_{30}$ arylthio group, a substituted or unsubstituted $C_1$-$C_{20}$ heteroaryl group, a substituted or unsubstituted monovalent non-aromatic condensed polycyclic group, a substituted or unsubstituted monovalent non-aromatic condensed heteropolycyclic group, $-Si(Q_{601})(Q_{602})(Q_{603})$, $-C(=O)(Q_{601})$, $-S(=O)_2(Q_{601})$, and $-P(=O)(Q_{601})(Q_{602})$.

In one or more embodiments, in Formulae 601 and 601-1, $R_{601}$ and $R_{611}$ to $R_{613}$ may each independently be selected from

a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group;

a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group,

a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group, each substituted with at least one selected from deuterium, - F, -Cl, -Br, -I, a hydroxyl group, a cyano group, a nitro group, an amidino group, a hydrazino group, a hydrazono group, a $C_1$-$C_{20}$ alkyl group, a $C_1$-$C_{20}$ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group; and

$-S(=O)_2(Q_{601})$ and $-P(=O)(Q_{601})(Q_{602})$,

wherein $Q_{601}$ and $Q_{602}$ may respectively be understood by referring to the descriptions therefor provided herein.

The electron transport region may include at least one compound selected from Compounds ET1 to ET36, but embodiments are not limited thereto:

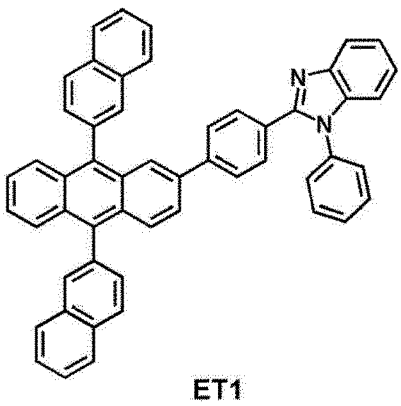

ET1

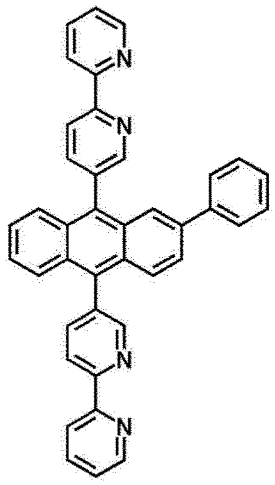

ET2

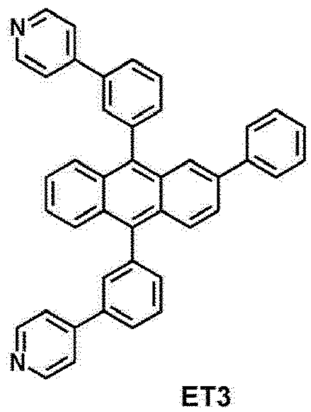

ET3

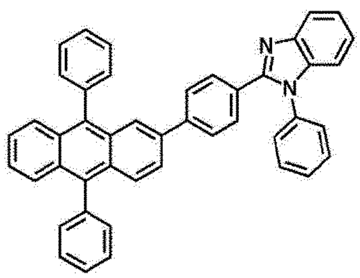

ET4

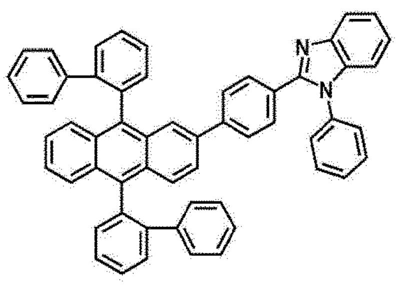

ET5

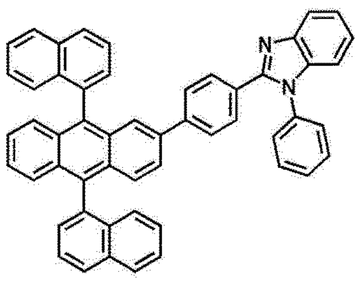

ET6

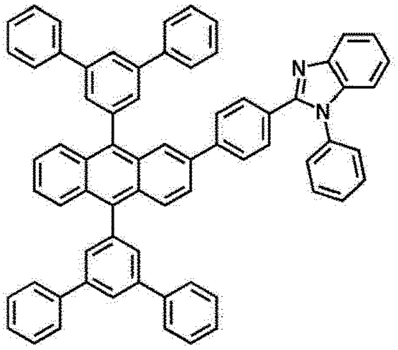

ET7

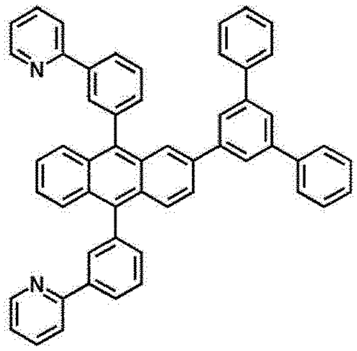

ET8

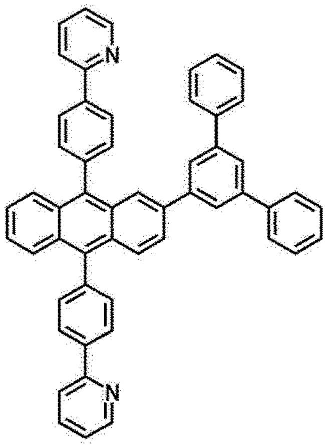

ET9

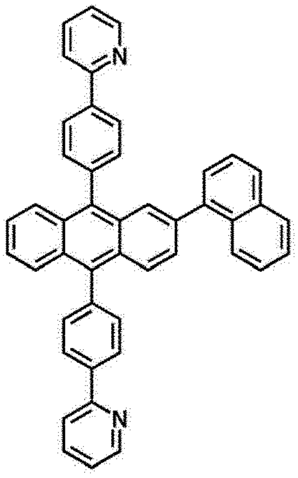

ET10

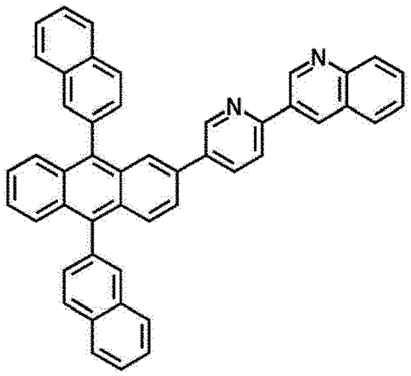

ET11

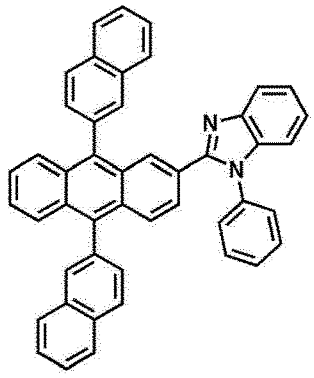

ET12

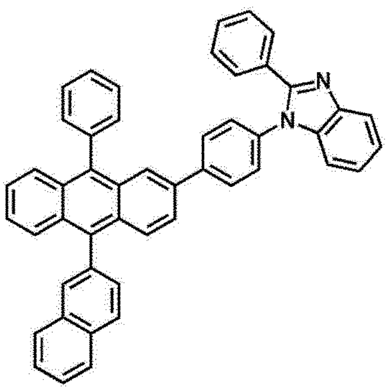

ET13

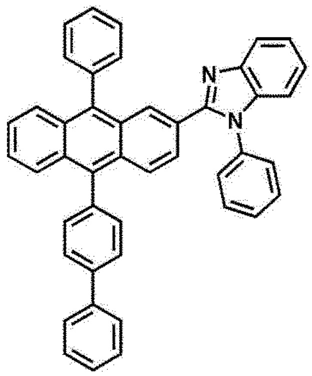

ET14

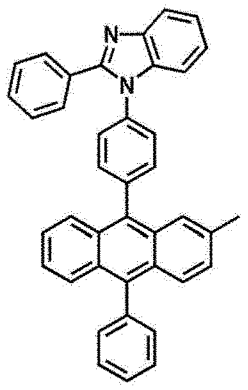

ET15

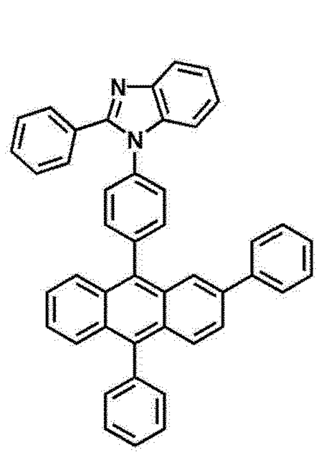

**ET16**

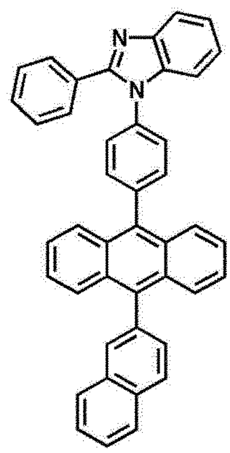

**ET17**

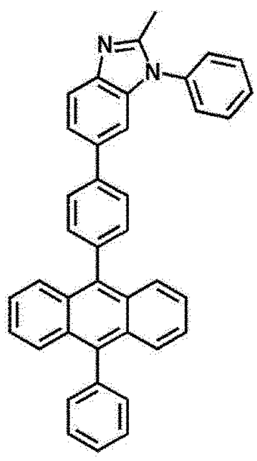

**ET18**

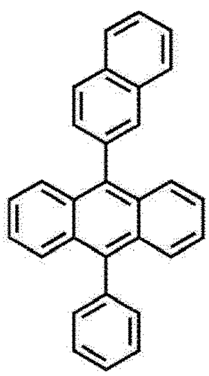

**ET19**

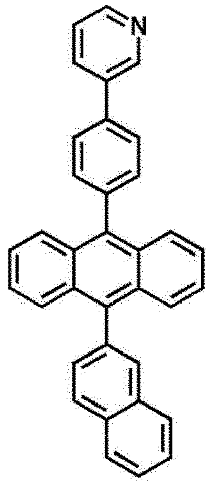

**ET20**

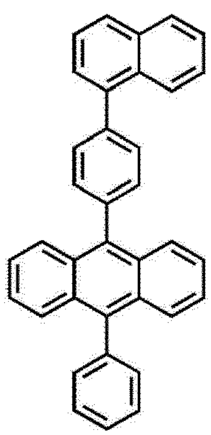

**ET21**

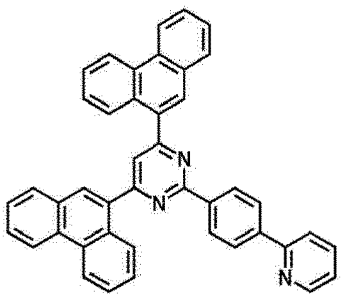

**ET22**

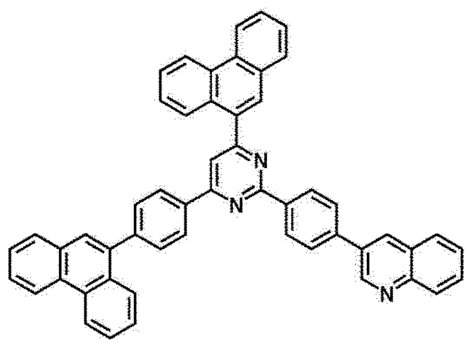

**ET23**

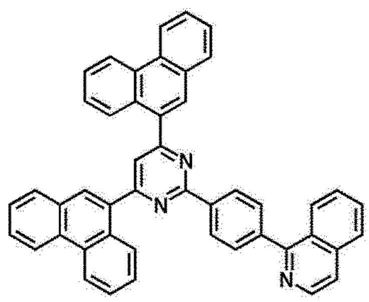

**ET24**

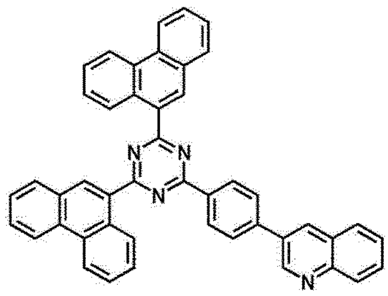

ET25

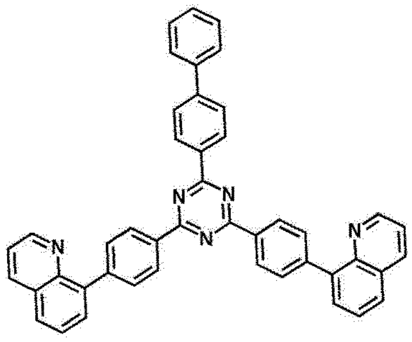

ET26

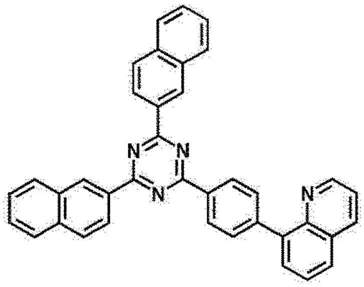

ET27

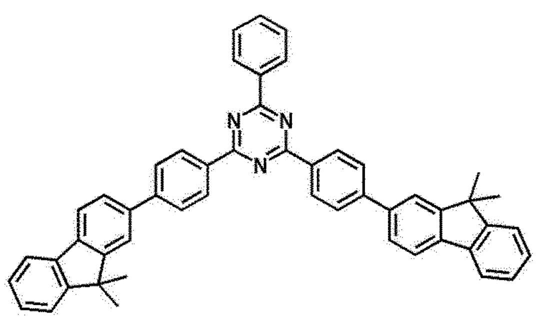

ET28

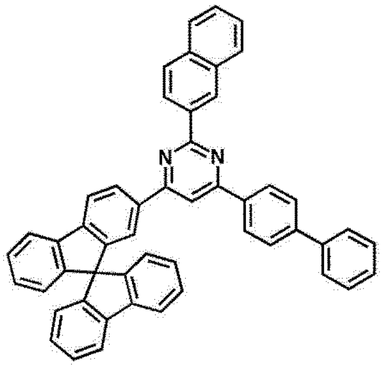

ET29

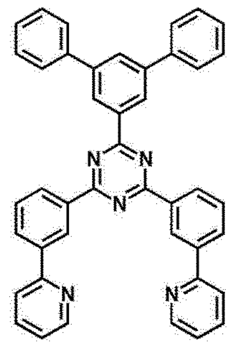

ET30

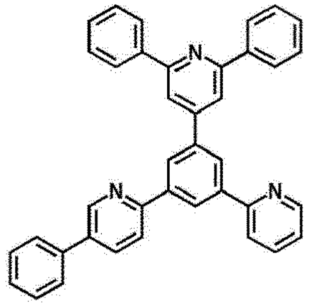

ET31

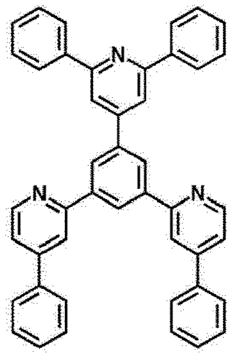

ET32

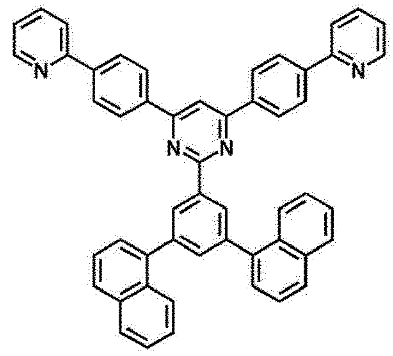

ET33

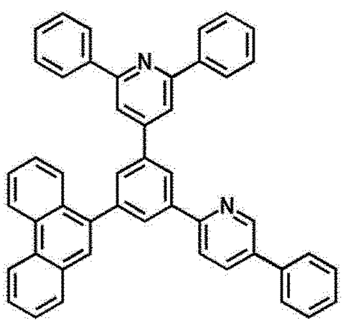

ET34

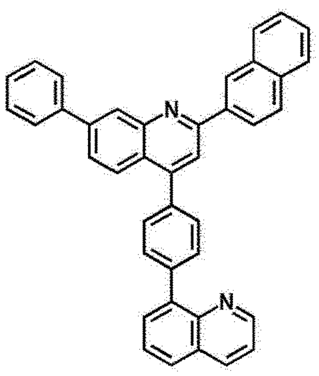

ET35

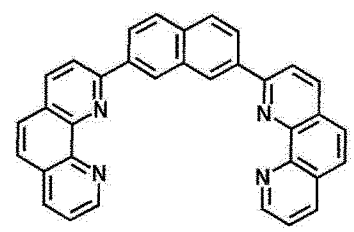

ET36

In some embodiments, the electron transport region may include at least one compound selected from 2,9-dimethyl-4,7-diphenyl-1,10-phenanthroline (BCP), 4,7-diphenyl-1,10-phenanthroline (Bphen), Alq3, BAlq, 3-(biphenyl-4-yl)-5-(4-tert-butylphenyl)-4-phenyl-4H-1,2,4-triazole (TAZ), and NTAZ:

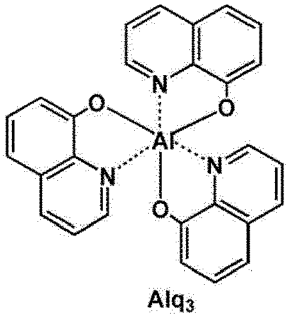

Alq$_3$

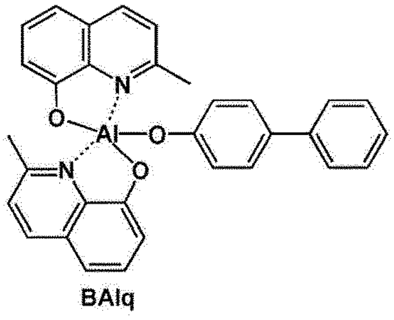

BAlq

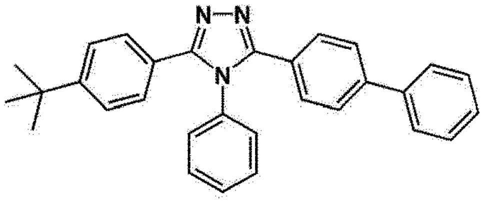

TAZ

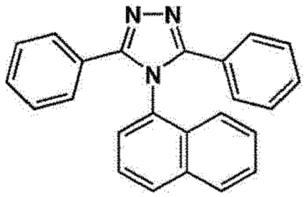

NTAZ

The thicknesses of the buffer layer, the hole blocking layer, or the electron control layer may each independently be in a range of about 20 Å to about 1,000 Å, and in some embodiments, about 20 Å to about 700 Å, about 20 Å to about 500 Å, about 20 Å to about 400 Å, about 30 Å to about 400 Å or about 30 Å to about 300 Å. When the thicknesses of the buffer layer, the hole blocking layer or the electron control layer are within any of these ranges, excellent hole blocking characteristics or excellent electron controlling characteristics may be obtained without a substantial increase in driving voltage.

The thickness of the electron transport layer may be in a range of about 100 Å to about 1,000 Å, and in some embodiments, about 100 Å to about 700 Å, about 100 Å to about 600 Å, about 150 Å to about 600 Å, about 150 Å to about 500 Å or about 350 Å to about 450 Å. When the thickness of the electron transport layer is within any of these ranges, excellent electron transport characteristics may be obtained without a substantial increase in driving voltage.

The electron transport region (for example, the electron transport layer in the electron transport region) may further include, in addition to the materials described above, a material including metal.

The material including metal may include at least one selected from an alkali metal complex and an alkaline earth metal complex. The alkali metal complex may include a metal ion selected from a lithium (Li) ion, a sodium (Na) ion, a potassium (K) ion, a rubidium (Rb) ion, and a cesium (Cs) ion. The alkaline earth metal complex may include a metal ion selected from a beryllium (Be) ion, a magnesium (Mg) ion, a calcium (Ca) ion, a strontium (Sr) ion, and a barium (Ba) ion. Each ligand coordinated with the metal ion of the alkali metal complex and the alkaline earth metal complex may independently be selected from a hydroxy quinoline, a hydroxy isoquinoline, a hydroxy benzoquinoline, a hydroxy acridine, a hydroxy phenanthridine, a hydroxy phenyloxazole, a hydroxy phenylthiazole, a hydroxy phenyloxadiazole, a hydroxy phenylthiadiazole, a hydroxy phenylpyridine, a hydroxy phenylbenzimidazole, a hydroxy phenylbenzothiazole, a bipyridine, a phenanthroline, and a cyclopentadiene, but embodiments are not limited thereto.

For example, the material including metal may include a Li complex. The Li complex may include, e.g., Compound ET-D1 (LiQ) or Compound ET-D2:

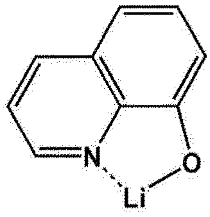

ET-D1

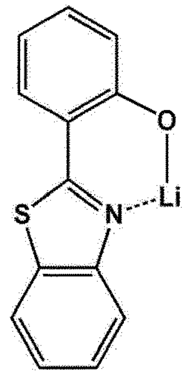

ET-D2

The electron transport region may include an electron injection layer that facilitates injection of electrons from the second electrode 190. The electron injection layer may be in direct contact with the second electrode 190.

The electron injection layer may have i) a single-layered structure consisting of a single layer consisting of a single material, ii) a single-layered structure consisting of a single layer including a plurality of different materials, or iii) a multi-layered structure having a plurality of layers, each including a plurality of different materials.

The electron injection layer may include an alkali metal, an alkaline earth metal, a rare earth metal, an alkali metal compound, an alkaline earth metal compound, a rare earth metal compound, an alkali metal complex, an alkaline earth metal complex, a rare earth metal complex, or a combination thereof.

The alkali metal may be selected from Li, Na, K, Rb, and Cs. In an embodiment, the alkali metal may be Li, Na, or Cs. In one or more embodiments, the alkali metal may be Li or Cs, but embodiments are not limited thereto.

The alkaline earth metal may be selected from Mg, Ca, Sr, and Ba.

The rare earth metal may be selected from Sc, Y, Ce, Tb, Yb, and Gd.

The alkali metal compound, the alkaline earth metal compound, and the rare earth metal compound may each independently be selected from oxides and halides (e.g., fluorides, chlorides, bromides, or iodides) of the alkali metal, the alkaline earth metal, and the rare earth metal, respectively.

The alkali metal compound may be selected from alkali metal oxides, such as $Li_2O$, $Cs_2O$, or $K_2O$, and alkali metal halides, such as LiF, NaF, CsF, KF, LiI, NaI, CsI, or KI. In an embodiment, the alkali metal compound may be selected from LiF, $Li_2O$, NaF, LiI, NaI, CsI, and KI, but embodiments are not limited thereto.

The alkaline earth-metal compound may be selected from alkaline earth-metal compounds, such as BaO, SrO, CaO, $Ba_xSr_{1-x}O$ (wherein $0<x<1$), and $Ba_xCa_{1-x}O$ (wherein $0<x<1$). In an embodiment, the alkaline earth metal compound may be selected from BaO, SrO, and CaO, but embodiments are not limited thereto.

The rare earth metal compound may be selected from YbF3, ScF3, $Sc_2O_3$, $Y_2O_3$, $Ce_2O_3$, GdF3, and TbF3. In an embodiment, the rare earth metal compound may be selected from $YbF_3$, $ScF_3$, $TbF_3$, $YbI_3$, $ScI_3$, and $TbI_3$, but embodiments are not limited thereto.

The alkali metal complex, the alkaline earth metal complex, and the rare earth metal complex may each include ions of the above-described alkali metal, alkaline earth metal, and rare earth metal. Each ligand coordinated with the metal ion of the alkali metal complex, the alkaline earth metal complex, and the rare earth metal complex may independently be selected from a hydroxy quinoline, a hydroxy isoquinoline, a hydroxy benzoquinoline, a hydroxy acridine, a hydroxy phenanthridine, a hydroxy phenyloxazole, a hydroxy phenylthiazole, a hydroxy phenyloxadiazole, a hydroxy phenylthiadiazole, a hydroxy phenylpyridine, a hydroxy phenylbenzimidazole, a hydroxy phenylbenzothiazole, a bipyridine, a phenanthroline, and a cyclopentadiene, but embodiments are not limited thereto.

The electron injection layer may consist of an alkali metal, an alkaline earth metal, a rare earth metal, an alkali metal compound, an alkaline earth metal compound, a rare earth metal compound, an alkali metal complex, an alkaline earth metal complex, a rare earth metal complex, or a combination thereof, as described above. In some embodiments, the electron injection layer may further include an organic material. When the electron injection layer further includes an organic material, the alkali metal, the alkaline earth metal, the rare earth metal, the alkali metal compound, the alkaline earth metal compound, the rare earth metal compound, the alkali metal complex, the alkaline earth metal complex, the rare earth metal complex, or a combination thereof may be homogeneously or non-homogeneously dispersed in a matrix including the organic material.

The thickness of the electron injection layer may be in a range of about 1 Å to about 100 Å, and in some embodiments, about 3 Å to about 90 Å or about 3 Å to about 20 Å. When the thickness of the electron injection layer is within any of these ranges, excellent electron injection characteristics may be obtained without a substantial increase in driving voltage.

Second electrode 190

The second electrode 190 may be on the organic layer 150. In an embodiment, the second electrode 190 may be a cathode that is an electron injection electrode. In this embodiment, a material for forming the second electrode 190 may be a material having a low work function, for example, a metal, an alloy, an electrically conductive compound, or a combination thereof.

The second electrode 190 may include at least one selected from lithium (Li), silver (Ag), magnesium (Mg), aluminium (Al), aluminium-lithium (Al-Li), calcium (Ca), magnesium-indium (Mg-In), magnesium-silver (Mg-Ag), ITO, and IZO, but embodiments are not limited thereto. The second electrode 190 may be a transmissive electrode, a semi-transmissive electrode, or a reflective electrode.

The second electrode 190 may have a single-layered structure, or a multi-layered structure including two or more layers.

Description of FIGS. 2 to 4

Referring to FIG. 2, an organic light-emitting device 20 has a first capping layer 210, the first electrode 110, the organic layer 150, and the second electrode 190 structure, wherein the layers are sequentially stacked in this stated order. Referring to FIG. 3, an organic light-emitting device 30 has the first electrode 110, the organic layer 150, the second electrode 190, and a second capping layer 220 structure, wherein the layers are sequentially stacked in this stated order. Referring to FIG. 4, an organic light-emitting device 40 has the first capping layer 210, the first electrode 110, the organic layer 150, the second electrode 190, and the second capping layer 220 structure, wherein the layers are stacked in this stated order.

The first electrode 110, the organic layer 150, and the second electrode 190 illustrated in FIGS. 2 to 4 may be substantially the same as those illustrated in FIG. 1.

In the organic light-emitting devices 20 and 40, light emitted from the emission layer in the organic layer 150 may pass through the first electrode 110 (which may be a semi-transmissive electrode or a transmissive electrode) and through the first capping layer 210 to the outside. In the organic light-emitting devices 30 and 40, light emitted from the emission layer in the organic layer 150 may pass through the second electrode 190 (which may be a semi-transmissive electrode or a transmissive electrode) and through the second capping layer 220 to the outside.

The first capping layer 210 and the second capping layer 220 may improve the external luminescence efficiency based on the principle of constructive interference.

The first capping layer 210 and the second capping layer 220 may each independently be a capping layer including an organic material, an inorganic capping layer including an inorganic material, or a composite capping layer including an organic material and an inorganic material.

At least one of the first capping layer 210 and the second capping layer 220 may each independently include at least one material selected from carbocyclic compounds, heterocyclic compounds, amine-based compounds, porphyrine derivatives, phthalocyanine derivatives, naphthalocyanine derivatives, alkali metal complexes, and alkaline earth metal complexes. The carbocyclic compound, the heterocyclic compound, and the amine-based compound may optionally be substituted with a substituent containing at least one element selected from O, N, S, Se, Si, F, Cl, Br, and I. In an embodiment, at least one of the first capping layer 210 and the second capping layer 220 may each independently include an amine-based compound.

In one or more embodiments, at least one of the first capping layer 210 and the second capping layer 220 may each independently include a compound represented by Formula 201 or a compound represented by Formula 202.

In one or more embodiments, at least one of the first capping layer 210 and the second capping layer 220 may each independently include a compound selected from Compounds HT28 to HT33 and Compound CP1 to CP5, but embodiments are not limited thereto:

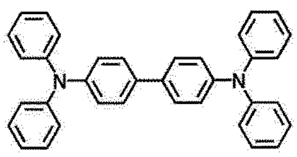

CP1

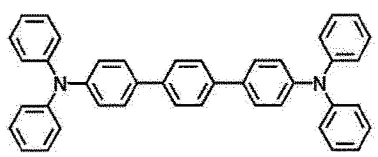

CP2

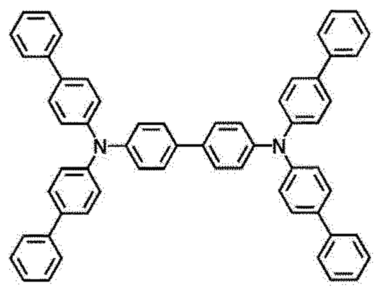

CP3

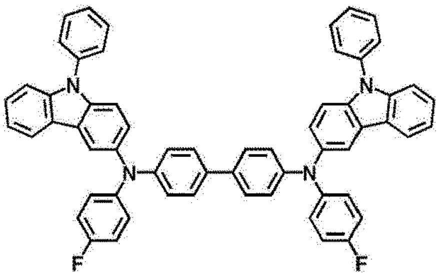

CP4

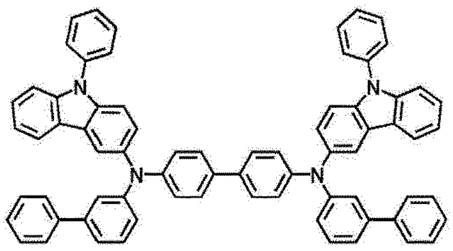

CP5

Hereinbefore, the organic light-emitting device has been described with reference to FIGS. 1 to 4, but embodiments are not limited thereto.

The layers constituting the hole transport region, the emission layer, and the layers constituting the electron transport region may be formed in a specific region by using one or more suitable methods such as vacuum deposition, spin coating, casting, Langmuir-Blodgett (LB) deposition, ink-jet printing, laser printing, and laser-induced thermal imaging.

When layers constituting the hole transport region, an emission layer, and layers constituting the electron transport region are each independently formed by vacuum-deposition, the vacuum-deposition may be performed at a deposition temperature in a range of about 100 °C to about 500 °C, at a vacuum degree in a range of about $10^{-8}$ torr to about $10^{-3}$ torr, and at a deposition rate in a range of about 0.01 Angstroms per second (Å/sec) to about 100 A/sec, depending on the material to be included in each layer and the structure of each layer to be formed.

When layers constituting the hole transport region, the emission layer, and layers constituting the electron transport region are each independently formed by spin coating, the spin coating may be performed at a coating rate of about 2,000 revolutions per minute (rpm) to about 5,000 rpm and at a heat treatment temperature of about 80 °C to 200 °C, depending on the material to be included in each layer and the structure of each layer to be formed.

General definitions of substituents

The term "$C_1$-$C_{60}$ alkyl group" as used herein refers to a linear or branched aliphatic hydrocarbon monovalent group having 1 to 60 carbon atoms. Examples thereof include a methyl group, an ethyl group, a propyl group, an isobutyl group, a sec-butyl group, a tert-butyl group, a pentyl group, an iso-amyl group, and a hexyl group. The term "$C_1$-$C_{60}$ alkylene group" as used herein refers to a divalent group having the same structure as the $C_1$-$C_{60}$ alkyl group. Corresponding definitions apply to other ranges given for the number of carbon atoms in an alkyl/alkylene group.

The term "$C_2$-$C_{60}$ alkenyl group" as used herein refers to a hydrocarbon group having at least one carbon-carbon double bond in the middle or at the terminus of the $C_2$-$C_{60}$ alkyl group. Examples thereof include an ethenyl group, a propenyl group, and a butenyl group. The term "$C_2$-$C_{60}$ alkenylene group" as used herein refers to a divalent group having the same structure as the $C_2$-$C_{60}$ alkenyl group. Corresponding definitions apply to other ranges given for the number of carbon atoms in an alkenyl/alkenylene group.

The term "$C_2$-$C_{60}$ alkynyl group" as used herein refers to a hydrocarbon group having at least one carbon-carbon triple bond in the middle or at the terminus of the $C_2$-$C_{60}$ alkyl group. Examples thereof include an ethynyl group and a propynyl group. The term "$C_2$-$C_{60}$ alkynylene group" as used herein refers to a divalent group having the same structure as the C2-C60 alkynyl group. Corresponding definitions apply to other ranges given for the number of carbon atoms in an alkynyl/alkynylene group.

The term "$C_1$-$C_{60}$ alkoxy group" as used herein refers to a monovalent group represented by $-OA_{101}$ (wherein $A_{101}$ is a $C_1$-$C_{60}$ alkyl group). Examples thereof include a methoxy group, an ethoxy group, and an isopropyloxy group. Corresponding definitions apply to other ranges given for the number of carbon atoms in an alkoxy group.

The term "$C_3$-$C_{10}$ cycloalkyl group" as used herein refers to a monovalent monocyclic saturated hydrocarbon group including 3 to 10 carbon atoms. Examples thereof include a cyclopropyl group, a cyclobutyl group, a cyclopentyl group, a cyclohexyl group, and a cycloheptyl group. The term "$C_3$-$C_{10}$ cycloalkylene group" as used herein refers to a divalent group having the same structure as the $C_3$-$C_{10}$ cycloalkyl group.

The term "$C_1$-$C_{10}$ heterocycloalkyl group" as used herein refers to a monovalent monocyclic group including at least one heteroatom selected from N, O, Si, P, and S as a ring-forming atom and 1 to 10 carbon atoms. Examples thereof include a 1,2,3,4-oxatriazolidinyl group, a tetrahydrofuranyl group, and a tetrahydrothiophenyl group. The term "$C_1$-$C_{10}$ heterocycloalkylene group" as used herein refers to a divalent group having the same structure as the $C_1$-$C_{10}$ heterocycloalkyl group.

The term "$C_3$-$C_{10}$ cycloalkenyl group" as used herein refers to a monovalent monocyclic group that has 3 to

10 carbon atoms and at least one double bond in its ring, and is not aromatic. Examples thereof include a cyclopentenyl group, a cyclohexenyl group, and a cycloheptenyl group. The term "$C_3$-$C_{10}$ cycloalkenylene group" as used herein refers to a divalent group having the same structure as the $C_3$-$C_{10}$ cycloalkenyl group.

The term "$C_1$-$C_{10}$ heterocycloalkenyl group" as used herein refers to a monovalent monocyclic group including at least one heteroatom selected from N, O, Si, P, and S as a ring-forming atom, 1 to 10 carbon atoms, and at least one double bond in its ring. Examples of the $C_1$-$C_{10}$ heterocycloalkenyl group include a 4,5-dihydro-1,2,3,4-oxatriazolyl group, a 2,3-dihydrofuranyl group, and a 2,3-dihydrothiophenyl group. The term "$C_1$-$C_{10}$ heterocycloalkylene group" as used herein refers to a divalent group having the same structure as the $C_1$-$C_{10}$ heterocycloalkyl group.

The term "$C_6$-$C_{60}$ aryl group" as used herein refers to a monovalent group having a carbocyclic aromatic system having 6 to 6 carbon atoms. The term "$C_6$-$C_{60}$ arylene group" as used herein refers to a divalent group having a carbocyclic aromatic system having 6 to 60 carbon atoms. Examples of the $C_6$-$C_{60}$ aryl group include a phenyl group, a naphthyl group, an anthracenyl group, a phenanthrenyl group, a pyrenyl group, and a chrysenyl group. When the $C_6$-$C_{60}$ aryl group and the $C_6$-$C_{60}$ arylene group each independently include two or more rings, the respective rings may be fused. Corresponding definitions apply to other ranges given for the number of carbon atoms in an aryl/arylene group.

The term "$C_1$-$C_{60}$ heteroaryl group" as used herein refers to a monovalent group having a heterocyclic aromatic system having at least one heteroatom selected from N, O, Si, P, and S as a ring-forming atom and 1 to 60 carbon atoms. The term "$C_1$-$C_{60}$ heteroarylene group" as used herein refers to a divalent group having a heterocyclic aromatic system having at least one heteroatom selected from N, O, Si, P, and S as a ring-forming atom and 1 to 60 carbon atoms. Examples of the $C_1$-$C_{60}$ heteroaryl group include a pyridinyl group, a pyrimidinyl group, a pyrazinyl group, a pyridazinyl group, a triazinyl group, a quinolinyl group, and an isoquinolinyl group. When the $C_1$-$C_{60}$ heteroaryl group and the $C_1$-$C_{60}$ heteroarylene group each independently include two or more rings, the respective rings may be fused. Corresponding definitions apply to other ranges given for the number of carbon atoms in an heteroaryl/heteroarylene group.

The term "$C_6$-$C_{60}$ aryloxy group" as used herein indicates -OA102 (wherein A102 is a $C_6$-$C_{60}$ aryl group), and a $C_6$-$C_{60}$ arylthio group as used herein indicates -SA103 (wherein A103 is a $C_6$-$C_{60}$ aryl group). Corresponding definitions apply to other ranges given for the number of carbon atoms in an aryloxy group and an arylthio group.

The term "$C_1$-$C_{60}$ heteroaryloxy group" as used herein indicates -OA104 (wherein A104 is a $C_1$-$C_{60}$ heteroaryl group). The term "$C_1$-$C_{60}$ heteroarylthio group" as used herein indicates -$SA_{105}$ (wherein A105 is a $C_1$-$C_{60}$ heteroaryl group). Corresponding definitions apply to other ranges given for the number of carbon atoms in a heteroaryloxy group and a heteroarylthio group.

The term "monovalent non-aromatic condensed polycyclic group" as used herein refers to a monovalent group that has two or more rings condensed and only carbon atoms as ring forming atoms (e.g., 8 to 60 carbon atoms), wherein the entire molecular structure is non-aromatic. Examples of the monovalent non-aromatic condensed polycyclic group may include a fluorenyl group. The term "divalent non-aromatic condensed polycyclic group" as used herein refers to a divalent group having substantially the same structure as the monovalent non-aromatic condensed polycyclic group.

The term "monovalent non-aromatic condensed heteropolycyclic group" as used herein refers to a monovalent group that has two or more condensed rings and at least one heteroatom selected from N, O, Si, P, and S, in addition to carbon atoms (e.g., 1 to 60 carbon atoms), as a ring-forming atom, wherein the entire molecular structure is non-aromatic. Examples of the monovalent non-aromatic condensed heteropolycyclic group may include a carbazolyl group. The term "divalent non-aromatic condensed heteropolycyclic group" as used herein refers to a divalent group having substantially the same structure as the monovalent non-aromatic condensed heteropolycyclic group.

The term "$C_5$-$C_{60}$ carbocyclic group" as used herein refers to a monocyclic or polycyclic group having 5 to 60 carbon atoms only as ring-forming atoms. The $C_5$-$C_{60}$ carbocyclic group may be an aromatic carbocyclic group or a non-aromatic carbocyclic group. The term "$C_5$-$C_{60}$ carbocyclic group" as used herein refers to a ring (e.g., a benzene group), a monovalent group (e.g., a phenyl group), or a divalent group (e.g., a phenylene group). Also, depending on the number of substituents connected to the $C_5$-$C_{60}$ carbocyclic group, the $C_5$-$C_{60}$ carbocyclic group may be a trivalent group or a quadrivalent group. Corresponding definitions apply to other ranges given for the number of carbon atoms in a carbocyclic group.

The term "$C_1$-$C_{60}$ heterocyclic group" as used herein refers to a group having substantially the same structure as the $C_5$-$C_{60}$ carbocyclic group, except that at least one heteroatom selected from N, O, Si, P, and S is used as a ring-forming atom, in addition to carbon atoms (e.g., 1 to 60 carbon atoms). Corresponding definitions apply to other ranges given for the number of carbon atoms in a heterocyclic group.

In the present specification, at least one substituent of the substituted $C_5$-$C_{60}$ (*e.g.* $C_5$-$C_{30}$) carbocyclic group, the substituted $C_1$-$C_{60}$ (*e.g.* $C_1$-$C_{20}$) heterocyclic group, the substituted $C_3$-$C_{10}$ cycloalkylene group, the substituted $C_1$-$C_{10}$ heterocycloalkylene group, the substituted $C_3$-$C_{10}$ cycloalkenylene group, the substituted $C_1$-$C_{10}$ heterocycloalkenylene group, the substituted $C_6$-$C_{60}$ (*e.g.* $C_6$-$C_{30}$) arylene group, the substituted $C_1$-$C_{60}$ (*e.g.* $C_1$-$C_{20}$) heteroarylene group, the substituted divalent non-aromatic condensed polycyclic group, the substituted divalent non-aromatic condensed heteropolycyclic group, the substituted $C_1$-$C_{60}$ (*e.g.* $C_1$-$C_{20}$) alkyl group, the substituted $C_2$-$C_{60}$ (*e.g.* $C_2$-$C_{20}$)

alkenyl group, the substituted $C_2$-$C_{60}$ (*e.g.* $C_2$-$C_{20}$) alkynyl group, the substituted $C_1$-$C_{60}$ (*e.g.* $C_1$-$C_{20}$) alkoxy group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_1$-$C_{10}$ heterocycloalkenyl group, the substituted $C_6$-$C_{60}$ (*e.g.* $C_6$-$C_{30}$) aryl group, the substituted $C_6$-$C_{60}$ (*e.g.* $C_6$-$C_{30}$) aryloxy group, the substituted $C_6$-$C_{60}$ (*e.g.* $C_6$-$C_{30}$) arylthio group, the substituted $C_1$-$C_{60}$ (*e.g.* $C_1$-$C_{20}$) heteroaryl group, the substituted $C_1$-$C_{60}$ (*e.g.* $C_1$-$C_{20}$) heteroaryloxy group, the substituted $C_1$-$C_{60}$ (*e.g.* $C_1$-$C_{20}$) heteroarylthio group, the substituted monovalent non-aromatic condensed polycyclic group, and the substituted monovalent non-aromatic condensed heteropolycyclic group may be selected from:

deuterium (-D), -F, -Cl, -Br, -I, a hydroxyl group, a cyano group, a nitro group, an amidino group, a hydrazino group, a hydrazono group, a $C_1$-$C_{60}$ (*e.g.* $C_1$-$C_{20}$) alkyl group, a C2-$C_{60}$ (*e.g.* $C_2$-$C_{20}$) alkenyl group, a $C_2$-$C_{60}$ (*e.g.* $C_2$-$C_{20}$) alkynyl group, and a $C_1$-$C_{60}$ (*e.g.* $C_1$-$C_{20}$) alkoxy group;

a $C_1$-$C_{60}$ (*e.g.* $C_1$-$C_{20}$) alkyl group, a $C_2$-$C_{60}$ (*e.g.* $C_2$-$C_{20}$) alkenyl group, a $C_2$-$C_{60}$ (*e.g.* $C_2$-$C_{20}$) alkynyl group, and a $C_1$-$C_{60}$ (*e.g.* $C_1$-$C_{20}$) alkoxy group, each substituted with at least one selected from deuterium, -F, -Cl, -Br, -I, a hydroxyl group, a cyano group, a nitro group, an amidino group, a hydrazino group, a hydrazono group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ (*e.g.* $C_6$-$C_{30}$) aryl group, a $C_6$-$C_{60}$ (*e.g.* $C_6$-$C_{30}$) aryloxy group, a $C_6$-$C_{60}$ (*e.g.* $C_6$-$C_{30}$) arylthio group, a $C_1$-$C_{60}$ (*e.g.* $C_1$-$C_{20}$) heteroaryl group, a monovalent non-aromatic condensed polycyclic group, a monovalent non-aromatic condensed heteropolycyclic group,-$Si(Q_{11})(Q_{12})(Q_{13})$, -$N(Q_{11})(Q_{12})$, -$B(Q_{11})(Q_{12})$, -C(=O)(Q11), -$S(=O)_2(Q_{11})$, and -$P(=O)(Q_{11})(Q_{12})$;

a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ (*e.g.* $C_6$-$C_{30}$) aryl group, a $C_6$-$C_{60}$ (*e.g.* C6-$C_{30}$) aryloxy group, a $C_6$-$C_{60}$ (*e.g.* $C_6$-$C_{30}$) arylthio group, a $C_1$-$C_{60}$ (*e.g.* $C_1$-$C_{20}$) heteroaryl group, a monovalent non-aromatic condensed polycyclic group, and a monovalent non-aromatic condensed heteropolycyclic group;

a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ (*e.g.* $C_6$-$C_{30}$) aryl group, a $C_6$-$C_{60}$ (*e.g.* C6-C30) aryloxy group, a $C_6$-$C_{60}$ (*e.g.* $C_6$-$C_{30}$) arylthio group, a $C_1$-$C_{60}$ (*e.g.* $C_1$-$C_{20}$) heteroaryl group, a monovalent non-aromatic condensed polycyclic group, and a monovalent non-aromatic condensed heteropolycyclic group, each substituted with at least one selected from deuterium, - F, -Cl, -Br, -I, a hydroxyl group, a cyano group, a nitro group, an amidino group, a hydrazino group, a hydrazono group, a $C_1$-$C_{60}$ (*e.g.* $C_1$-$C_{20}$) alkyl group, a $C_2$-$C_{60}$ (*e.g.* $C_2$-$C_{20}$) alkenyl group, a $C_2$-$C_{60}$ (*e.g.* $C_2$-$C_{20}$) alkynyl group, a $C_1$-$C_{60}$ (*e.g.* $C_1$-$C_{20}$) alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ (*e.g.* $C_6$-$C_{30}$) aryl group, a $C_6$-$C_{60}$ (*e.g.* $C_6$-$C_{30}$) aryloxy group, a $C_6$-$C_{60}$ (*e.g.* $C_6$-$C_{30}$) arylthio group, a $C_1$-$C_{60}$ (*e.g.* $C_1$-$C_{20}$) heteroaryl group, a monovalent non-aromatic condensed polycyclic group, a monovalent non-aromatic condensed heteropolycyclic group, -$Si(Q_{21})(Q_{22})(Q_{23})$, -$N(Q_{21})(Q_{22})$, -$B(Q_{21})(Q_{22})$, -$C(=O)(Q_{21})$, -$S(=O)_2(Q_{21})$, and-$P(=O)(Q_{21})(Q_{22})$; and

-$Si(Q_{31})(Q_{32})(Q_{33})$, -$N(Q_{31})(Q_{32})$, -$B(Q_{31})(Q_{32})$, -$C(=O)(Q_{31})$, -$S(=O)_2(Q_{31})$, and-$P(=O)(Q_{31})(Q_{32})$,

wherein $Q_{11}$ to $Q_{13}$, $Q_{21}$ to $Q_{23}$, and $Q_{31}$ to $Q_{33}$ may each independently be selected from hydrogen, deuterium, -F, -Cl, -Br, -I, a hydroxyl group, a cyano group, a nitro group, an amidino group, a hydrazino group, a hydrazono group, a $C_1$-$C_{60}$ (*e.g.* $C_1$-$C_{20}$) alkyl group, a $C_2$-$C_{60}$ (*e.g.* $C_2$-$C_{20}$) alkenyl group, a $C_2$-$C_{60}$ (*e.g.* $C_2$-$C_{20}$) alkynyl group, a $C_1$-$C_{60}$ (*e.g.* $C_1$-$C_{20}$) alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ (*e.g.* $C_6$-$C_{30}$) aryl group, a $C_1$-$C_{60}$ (*e.g.* $C_1$-$C_{20}$) heteroaryl group, a monovalent non-aromatic condensed polycyclic group, a monovalent non-aromatic condensed heteropolycyclic group, a biphenyl group, and a terphenyl group.

The term "Ph" as used herein refers to a phenyl group. The term "Me" as used herein refers to a methyl group. The term "Et" as used herein refers to an ethyl group. The term "ter-Bu" or "$Bu^t$" as used herein refers to a tert-butyl group. The term "OMe" as used herein refers to a methoxy group.

The term "biphenyl group" as used herein refers to a phenyl group substituted with a phenyl group. The "biphenyl group" may be a substituted phenyl group having a $C_6$-$C_{60}$ aryl group as a substituent.

The term "terphenyl group" as used herein refers to a phenyl group substituted with a biphenyl group. The "terphenyl group" may be a substituted phenyl group having a $C_6$-$C_{60}$ aryl group substituted with a $C_6$-$C_{60}$ aryl group as a substituent.

The symbols * and *' as used herein, unless defined otherwise, refer to a binding site to an adjacent atom in a corresponding formula.

Hereinafter, compounds and an organic light-emitting device according to one or more embodiments will be described in more detail with reference to Synthesis Examples and Examples. The wording "B was used instead of A" used in describing Synthesis Examples refers to that an identical number of molar equivalents of B was used in place of A.

Examples

Evaluation Example 1: Measurement of energy level

Regarding Compounds 1-1 and 1-6 (i.e., the hosts) and Comparative Compounds A, B, C, CBP, mCP, and ADN, a mixture of toluene and each Compound (the mixture containing 3 mL of toluene and 1 mg of each Compound) was loaded into a quartz cell. Subsequently, the resultant quartz cell was loaded into liquid nitrogen (77 Kelvins (K)), and a photoluminescence spectrum thereof was measured by using a device for measuring photoluminescence. The obtained spectrum was compared with a photoluminescence spectrum measured at room temperature, and peaks observed only at a low temperature were analyzed to calculate the singlet (S1) energy level, the triplet (T1) energy level, and $\Delta E_{st}$, which are shown in Table 1.

In addition, the triplet (T1) energy level of each of Compounds 2-5, 2-7, 2-10, 2-11, and 2-12 (i.e., the phosphorescent dopants) was measured. The results thereof are shown in Table 1.

Table 1

| Compound | S1 (eV) | T1 (eV) | ΔEst (eV) |
|---|---|---|---|
| Compound 1-1 | 3.02 | 2.85 | 0.17 |
| Compound 1-6 | 3.00 | 2.75 | 0.25 |
| Compound A | 2.96 | 2.65 | 0.31 |
| Compound B | 2.91 | 2.54 | 0.37 |
| Compound C | 2.93 | 2.58 | 0.35 |
| CBP | 3.10 | 2.58 | 0.52 |
| mCP | 3.50 | 2.90 | 0.60 |
| ADN | 3.00 | 1.80 | 1.20 |
| Compound 2-5 | - | 2.80 | - |
| Compound 2-7 | - | 2.85 | - |
| Compound 2-10 | - | 2.78 | - |
| Compound 2-11 | - | 2.78 | - |
| Compound 2-12 | - | 2.70 | - |

Compound 1-1

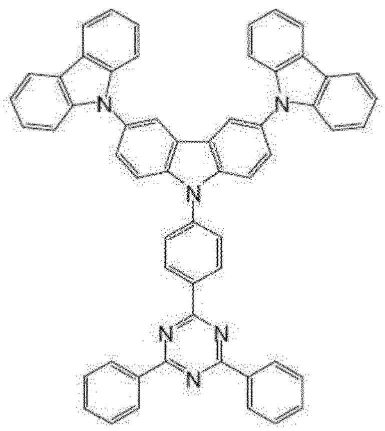

Compound 1-6

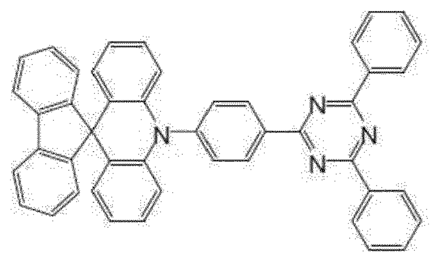

Compound 2-5

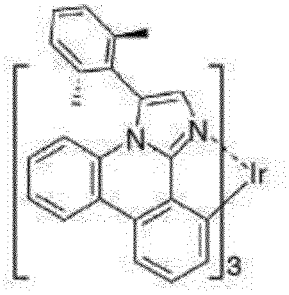

Compound 2-7

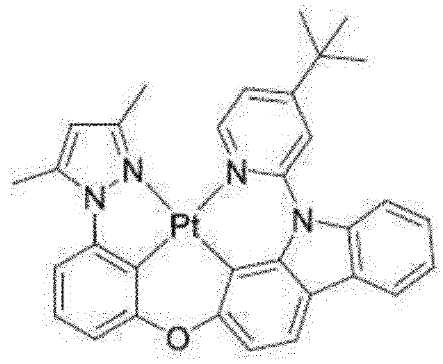

Compound 2-10

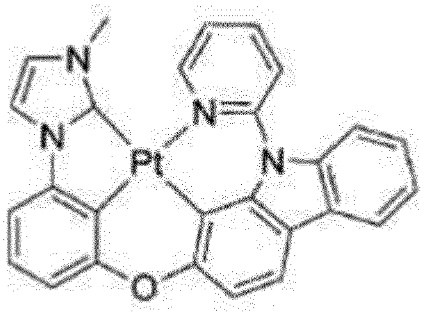

Compound 2-11

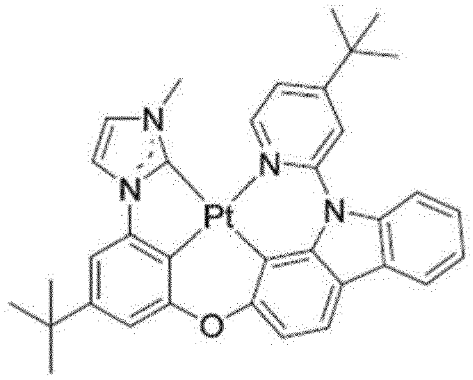

Compound 2-12

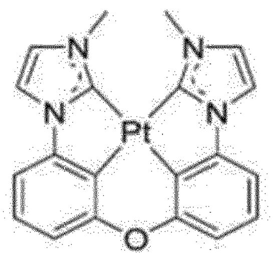

Compound A

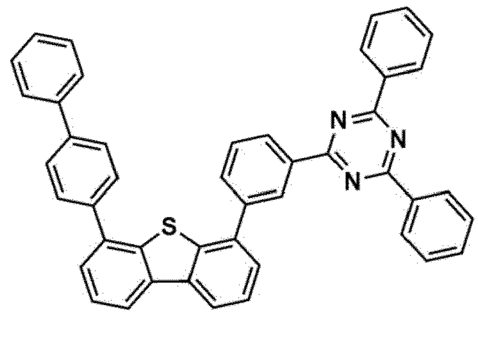

Compound B

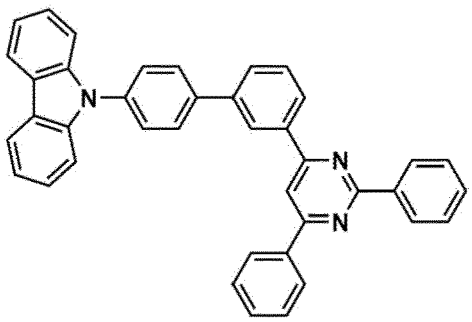

Compound C

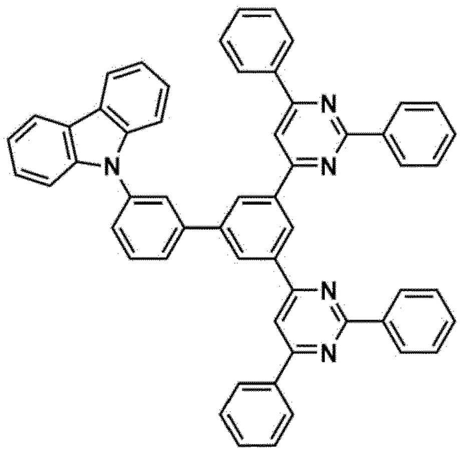

Example 1

An ITO glass substrate (25 millimeters (mm) × 25 mm and 15 Ohms per square centimeter ($\Omega/cm^2$)), an OLED glass (available from Samsung-Corning) substrate, was sequentially sonicated using distilled water and isopropyl alcohol, and cleaned by exposure to ultraviolet rays with ozone for about 30 minutes. Once the sonication was complete, a glass substrate with a transparent electrode line was mounted into a vacuum deposition device. 1,4,5,8,9,11-hexaazatriphenylene-hexacarbonitrile (HATCN) was deposited on the ITO electrode (anode) to form a hole injection layer having a thickness of 10 nm. Subsequently, N,N'-bis(naphthalen-1-yl)-N,N'-bis(phenyl)benzidine (NPB) was deposited on the hole injection layer to form a hole transport layer having a thickness of 40 nm.

9,9',9"-triphenyl-9H,9'H,9"H-3,3':6'3" -tercarbazole (TrisPCz) was deposited on the hole transport layer to form an electron blocking layer having a thickness of 10 nm, Compounds 1-1 and 2-5 were co-deposited on the electron blocking layer at a weight ratio of 9:1 to form an emission layer having a thickness of 25 nm.

mCBT (9,9'-(2,8-dibenzothiophenediyl)bis-9H-carbazole) was deposited on the emission layer to form a hole blocking layer having a thickness of 10 nm, and 2,7-di(2,2'-bipyridin-5-yl)triphenylene (BByTP) was deposited on the hole blocking layer to form an electron transport layer having a thickness of 40 nm.

Next, LiF was deposited on the electron transport layer to form an electron injection layer having a thickness of 1 nm, and aluminium (Al) was then deposited on the electron injection layer to form a cathode having a thickness of 100 nm, thereby completing the manufacture of an organic light-emitting device. Deposition equipment (Sunicel plus 200) manufactured by Sunic System Co., Ltd. was used for the deposition.

HAT-CN

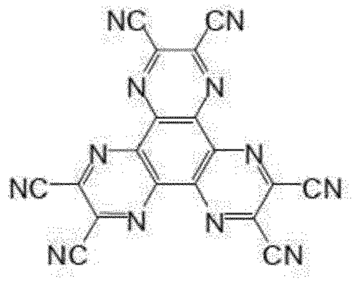

NPB

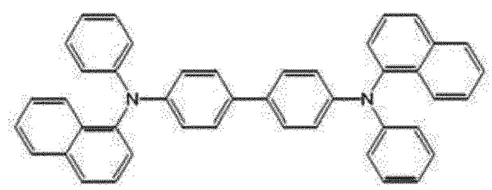

TrisPCz

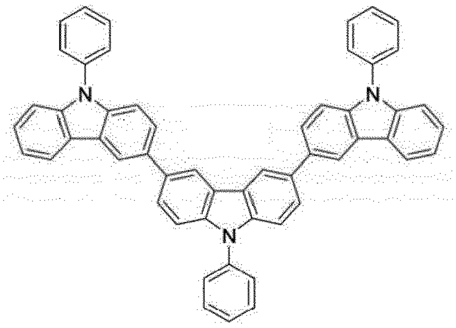

mCBT

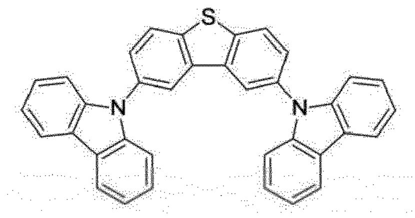

BByTP

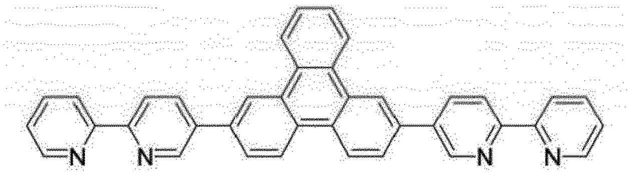

Examples 2 to 6 and Comparative Examples 1 to 6

Organic light-emitting devices were manufactured in substantially the same manner as in Example 1, except that the compounds shown in Table 2 were used instead of Compound 1-1 and Compound 2-5 in the formation of the emission layer.

Evaluation Example 2

The driving voltage, efficiency, and colorimetric purity of the organic light-emitting devices manufactured in Examples 1 to 6 and Comparative Examples 1 to 6 at a current density of 10 mA/cm$^2$ were evaluated as follows. The results thereof are shown in Table 2.

**[0244]** - The color-coordinate was measured using a luminance meter PR650 powered by a current voltmeter (Keithley SMU 236).

**[0245]** - The luminance was measured using a luminance meter PR650 powered by a current voltmeter (Keithley SMU 236).

**[0246]** - The efficiency was measured using a luminance meter PR650 powered by a current voltmeter (Keithley SMU 236).

Table 2

| | Emission layer | | Driving voltage (V) | External quantum efficiency (%) | Color coordinate | |
|---|---|---|---|---|---|---|
| | Host | Dopant | | | CIEx | CIEy |
| Example 1 | Compound 1-1 | Compound 2-5 | 4.2 | 20.1 | 0.15 | 0.18 |
| Example 2 | Compound 1-1 | Compound 2-7 | 4.3 | 17.5 | 0.16 | 0.12 |
| Example 3 | Compound 1-1 | Compound 2-10 | 4.1 | 17.8 | 0.14 | 0.23 |
| Example 4 | Compound 1-1 | Compound 2-11 | 4.2 | 20.5 | 0.15 | 0.12 |
| Example 5 | Compound 1-1 | Compound 2-12 | 4.4 | 18.8 | 0.18 | 0.31 |
| Example 6 | Compound 1-6 | Compound 2-12 | 4.2 | 19.4 | 0.18 | 0.28 |
| Comparative Example 1 | Compound Å | Compound 2-5 | 4.2 | 12.8 | 0.15 | 0.24 |

(continued)

| | Emission layer | | Driving voltage (V) | External quantum efficiency (%) | Color coordinate | |
|---|---|---|---|---|---|---|
| | Host | Dopant | | | CIEx | CIEy |
| Comparative Example 2 | Compound B | Compound 2-5 | 4.5 | 11.2 | 0.15 | 0.22 |
| Comparative Example 3 | Compound C | Compound 2-5 | 4.4 | 11.5 | 0.16 | 0.24 |
| Comparative Example 4 | CBP | Compound 2-5 | 5.2 | 15.4 | 0.17 | 0.20 |
| Comparative Example 5 | mCP | Compound 2-5 | 5.4 | 18.7 | 0.16 | 0.18 |
| Comparative Example 6 | ADN | Compound 2-5 | 6.8 | 2.5 | 0.22 | 0.25 |

Compound A

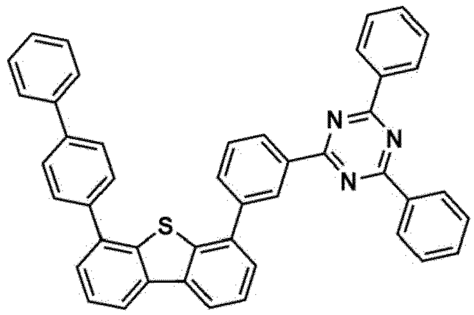

Compound B

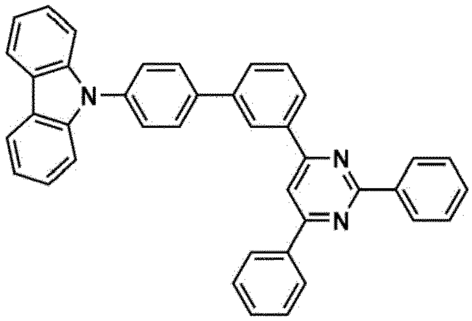

Compound C

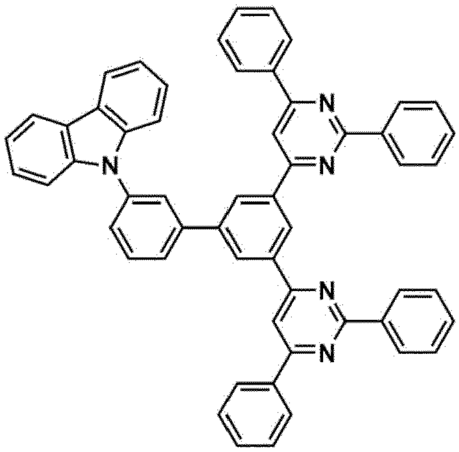

Referring to Tables 1 and 2, it was found that the organic light-emitting device according to one or more embodiments satisfied Equations 1 to 3 and had a low driving voltage and excellent external quantum efficiency, as compared with the organic light-emitting devices including Compounds A, B, and C that fail to satisfy Equations 1 to 3.

In addition, the organic light-emitting device according to one or more embodiments was found to emit blue light.

In other words, it was found that the organic light-emitting device according to one or more embodiments had a low driving voltage, excellent external quantum efficiency, and emit blue light.

As apparent from the foregoing description, the organic light-emitting device may have a low driving voltage and high external quantum efficiency.

Although certain embodiments and implementations have been described herein, other embodiments and modifications will be apparent from this description. Accordingly, the inventive concepts are not limited to such embodiments, but rather to the broader scope of the appended claims and various obvious modifications and equivalent arrangements as would be apparent to a person of ordinary skill in the art.

## Claims

1. An organic light-emitting device comprising:

a first electrode;
a second electrode facing the first electrode; and
an organic layer disposed between the first electrode and the second electrode, wherein the organic layer comprises an emission layer, the emission layer comprises a host and a phosphorescent dopant, the host satisfies Equation 1 and Equation 2, and the host and the phosphorescent dopant satisfy Equation 3:

Equation 1:

$$S1(H) - T1(H) \geq 0.3 \text{ eV}$$

Equation 2:

$$T1(H) \leq 2.7 \text{ eV}$$

Equation 3:

$$T1(D) \geq T1(H)$$

wherein, in Equations 1, 2, and 3, T1(H) indicates a triplet energy of the host, S1(H) indicates a singlet energy of the host, and T1(D) indicates a triplet energy of the phosphorescent dopant.

2. An organic light-emitting device according to claim 1, wherein the host comprises a heterocyclic compound represented by Formula 1:

Formula 1: $(Ar_1)_{n1}-(L_1)_{m1}-(Ar_2)_{n2}$

wherein, in Formula 1,

$L_1$ is a single bond, a $C_5$-$C_{60}$ carbocyclic group, or a $C_1$-$C_{60}$ heterocyclic group,
n1 and n2 are each independently an integer from 0 to 3, n1 + n2 ≤ 1,
m1 is an integer from 0 to 5, and
$Ar_1$ and $Ar_2$ are each independently a group represented by Formula 1A or Formula 1B:

Formula 1A:

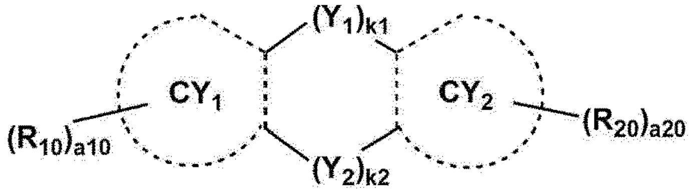

;

Formula 1B:

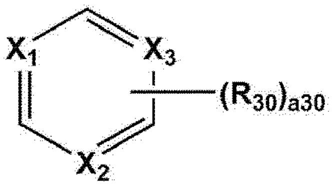

wherein, in Formulae 1A and 1B,

$Y_1$ and $Y_2$ are each independently selected from a single bond, *-O-*', *-S-*', *-C($R_1$)($R_2$)- *', *-N($R_1$)- *', *-Si($R_1$)($R_2$)-*', *-C(=O)-*', *-S(=O)$_2$-*' *-B($R_1$)-*', *-P($R_1$)-*', and *-P(=O)(R1)-*',
k1 and k2 are each independently 0 or 1, $k1 + k2 \leq 1$,
$CY_1$ and $CY_2$ are each independently a $C_5$-$C_{60}$ carbocyclic group or a $C_1$-$C_{60}$ heterocyclic group,
$X_1$ to $X_3$ are each independently C or N,
in a case where $X_1$ to $X_3$ are each C, at least one selected from $R_{30}$(s) is a cyano group,
$R_1$, $R_2$, $R_{10}$, $R_{20}$, and $R_{30}$ are each independently selected from hydrogen, deuterium, -F,-Cl, -Br, -I, a hydroxyl group, a cyano group, a nitro group, an amidino group, a hydrazino group, a hydrazono group, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, -Si($Q_1$)($Q_2$)($Q_3$),-N($Q_1$)($Q_2$), -B($Q_1$)($Q_2$), -C(=O)($Q_1$), -S(=O)$_2$($Q_1$), and -P(=O)($Q_1$)($Q_2$),

a10 and a20 are each independently an integer from 1 to 10,
a30 is an integer from 1 to 6,

$R_1$ and $R_2$ are optionally bound to form a substituted or unsubstituted $C_5$-$C_{60}$ carbocyclic group or a substituted or unsubstituted $C_1$-$C_{60}$ heterocyclic group,
$R_{10}$(s) and $R_{20}$(s) are optionally bound to at least one selected from $R_{10}$(s) and $R_{20}$(s) to form a substituted or unsubstituted $C_5$-$C_{60}$ carbocyclic group or a substituted or unsubstituted $C_1$-$C_{60}$ heterocyclic group,
when a30 is 2 or greater, at least two $R_{30}$(s) are optionally bound to form a substituted or unsubstituted $C_5$-$C_{60}$ carbocyclic group or a substituted or unsubstituted $C_1$-$C_{60}$ heterocyclic group,

at least one selected from $R_{10}$ and $R_{20}$ in Formula 1A is a binding site to $L_1$, $Ar_1$, or $Ar_2$,
at least one selected from $R_{30}$(s) in Formula 1B is a binding site to $L_1$, An, or $Ar_2$, and
at least one substituent of the substituted $C_5$-$C_{60}$ carbocyclic group, the substituted $C_1$-$C_{60}$ heterocyclic group, the substituted $C_1$-$C_{60}$ alkyl group, the substituted $C_2$-$C_{60}$ alkenyl group, the substituted $C_2$-$C_{60}$ alkynyl group, the substituted $C_1$-$C_{60}$ alkoxy group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_1$-$C_{10}$ heterocycloalkenyl group, the substituted $C_6$-$C_{60}$ aryl group, the substituted $C_6$-$C_{60}$ aryloxy group, the substituted $C_6$-$C_{60}$ arylthio group, the substituted $C_1$-$C_{60}$ heteroaryl group, the substituted $C_1$-$C_{60}$ heteroaryloxy group, the substituted $C_1$-$C_{60}$ heteroarylthio group, the substituted monovalent non-aromatic condensed polycyclic group, and the substituted monovalent non-aromatic condensed heteropolycyclic group is selected from:

deuterium, -F, -Cl, -Br, -I, a hydroxyl group, a cyano group, a nitro group, an amidino group, a hydrazino group, a hydrazono group, a $C_1$-$C_{60}$ alkyl group, a $C_2$-$C_{60}$ alkenyl group, a $C_2$-$C_{60}$ alkynyl group, and a $C_1$-$C_{60}$ alkoxy group;
a $C_1$-$C_{60}$ alkyl group, a $C_2$-$C_{60}$ alkenyl group, a $C_2$-$C_{60}$ alkynyl group, and a $C_1$-$C_{60}$ alkoxy group, each substituted with at least one selected from deuterium, -F, -Cl, -Br, -I, a hydroxyl group, a cyano group, a nitro group, an amidino group, a hydrazino group, a hydrazono group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a

$C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, -Si($Q_{11}$)($Q_{12}$)($Q_{13}$),-N($Q_{11}$)($Q_{12}$), -B($Q_{11}$)($Q_{12}$), -C(=O)($Q_{11}$), -S(=O)$_2$($Q_{11}$), and -P(=O)($Q_{11}$)($Q_{12}$);

a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, and a monovalent non-aromatic condensed heteropolycyclic group;

a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, and a monovalent non-aromatic condensed heteropolycyclic group, each substituted with at least one selected from deuterium, -F, -Cl, -Br, -I, a hydroxyl group, a cyano group, a nitro group, an amidino group, a hydrazino group, a hydrazono group, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, -Si($Q_{21}$)($Q_{22}$)($Q_{23}$), -N($Q_{21}$)($Q_{22}$), -B($Q_{21}$)($Q_{22}$), -C(=O)($Q_{21}$),-S(=O)$_2$($Q_{21}$), and -P(=O)($Q_{21}$)($Q_{22}$); and -Si($Q_{31}$)($Q_{32}$)($Q_{33}$), -N($Q_{31}$)($Q_{32}$), -B($Q_{31}$)($Q_{32}$), -C(=O)($Q_{31}$), -S(=O)$_2$($Q_{31}$), and-P(=O)($Q_{31}$)($Q_{32}$),

wherein $Q_1$ to $Q_3$, $Q_{11}$ to $Q_{13}$, $Q_{21}$ to $Q_{23}$, and $Q_{31}$ to $Q_{33}$ are each independently selected from hydrogen, deuterium, -F, -Cl, -Br, -I, a hydroxyl group, a cyano group, a nitro group, an amidino group, a hydrazino group, a hydrazono group, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_1$-$C_{60}$ heteroaryl group, a monovalent non-aromatic condensed polycyclic group, a monovalent non-aromatic condensed heteropolycyclic group, a biphenyl group, and a terphenyl group.

**3.** An organic light-emitting device according to claim 2, wherein An and $Ar_2$ are each independently a group represented by at least one of Formulae 1(1), 1(2), 1(3), and 1(4):

Formula 1(1)

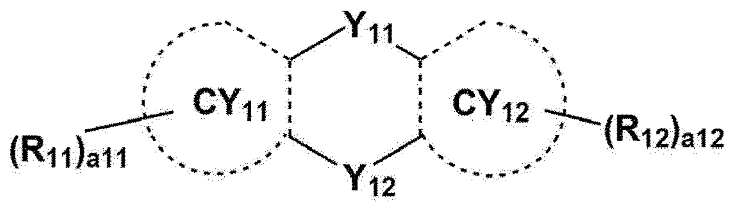

Formula 1(2)

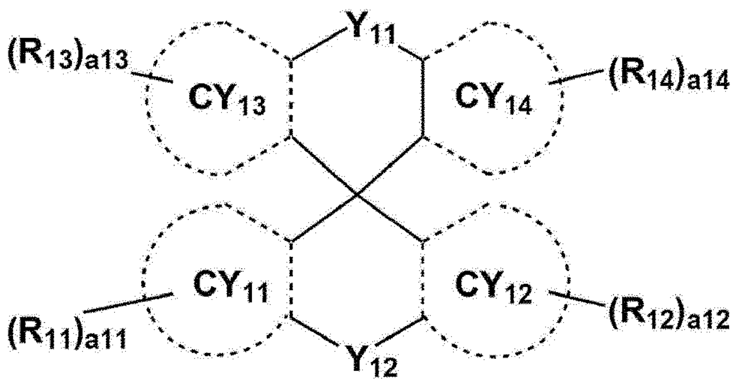

Formula 1(3)

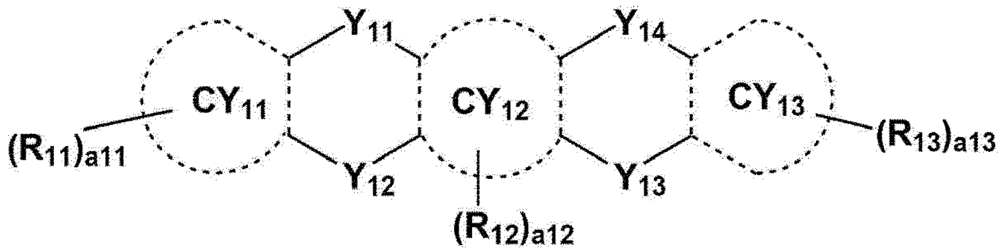

Formula 1(4)

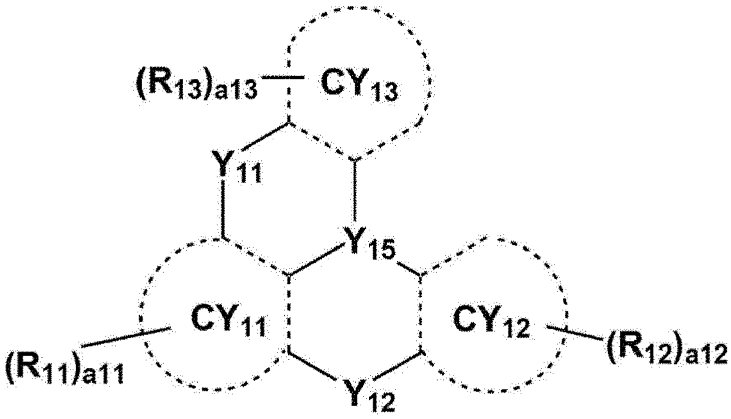

wherein, in Formulae 1(1) to 1(4),

$Y_{11}$ to $Y_{14}$ are each independently selected from a single bond, -O-, -S-, -C($R_{15}$)($R_{16}$)-,-N($R_{15}$)-, -Si($R_{15}$)($R_{16}$)-, -C(=O)-, -S(=O)$_2$-, -B($R_{15}$)-, -P($R_{15}$)-, and -P(=O)($R_{15}$)($R_{16}$)-,
$Y_{15}$ is N, B, or P,
$CY_{11}$ to $CY_{14}$ are each independently selected from a benzene group, a naphthalene group, a carbazole group, a dibenzofuran group, a dibenzothiophene group, and a dibenzosilole group,
$R_{11}$ to $R_{16}$ are each independently a binding site to $L_1$, $Ar_1$, or $Ar_2$, and be selected from hydrogen, deuterium, -F, -Cl, -Br, -I, a hydroxyl group, a cyano group, a nitro group, an amidino group, a hydrazino group, a hydrazono group, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, -Si($Q_1$)($Q_2$)($Q_3$),-N($Q_1$)($Q_2$), -B($Q_1$)($Q_2$), -C(=O)($Q_1$), -S(=O)$_2$($Q_1$), and -P(=O)($Q_1$)($Q_2$),

at least one selected from $R_{11}$ to $R_{16}$ is a binding site to $L_1$, An, or $Ar_2$,

wherein $Q_1$ to $Q_3$ are each independently selected from hydrogen, deuterium, -F, -Cl, -Br, -I, a hydroxyl group, a cyano group, a nitro group, an amidino group, a hydrazino group, a hydrazono group, a $C_1$-$C_{60}$ alkyl group, a $C_2$-$C_{60}$ alkenyl group, a $C_2$-$C_{60}$ alkynyl group, a $C_1$-$C_{60}$ alkoxy group, a phenyl group, a biphenyl group, a terphenyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a pyridinyl group, a pyrazinyl group, a pyrimidinyl group, a quinolinyl group, an isoquinolinyl group, a benzoquinolinyl group, a naphthyridinyl group, a quinoxalinyl group, a quinazolinyl group, a carbazolyl group, a phenanthridinyl group, an acridinyl group, a phenanthrolinyl group, a phenazinyl group, a triazinyl

group, a dibenzofuranyl group, a dibenzothiophenyl group, a biphenyl group, and a terphenyl group, and a11 to a14 are each independently an integer from 1 to 6.

**4.** An organic light-emitting device according to claim 2 or claim 3, wherein $L_1$ is a single bond or a group represented by one of Formulae 3-1 to 3-35:

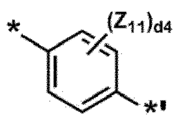
3-1

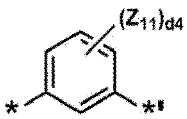
3-2

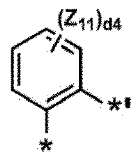
3-3

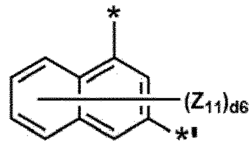
3-4

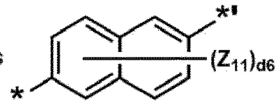
3-5

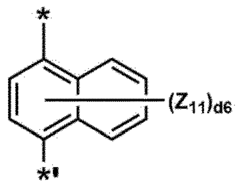
3-6

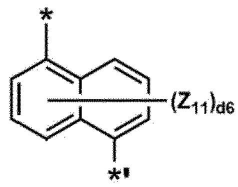
3-7

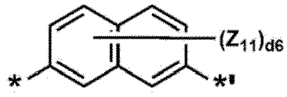
3-8

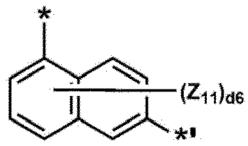
3-9

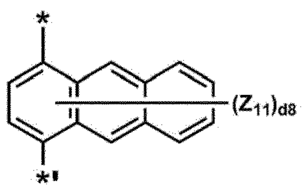
3-10

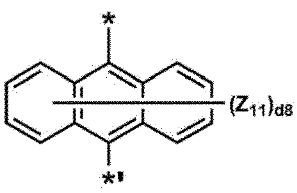
3-11

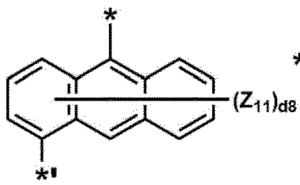
3-12

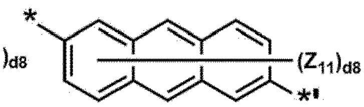
3-13

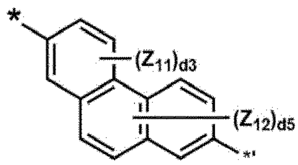
3-14

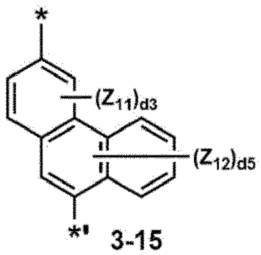
3-15

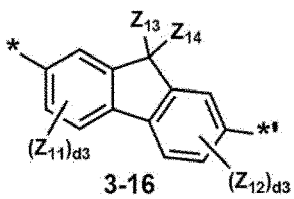
3-16

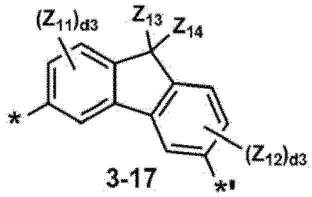
3-17

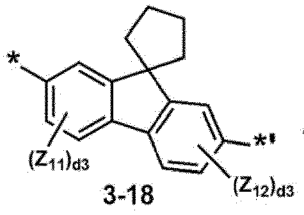
3-18

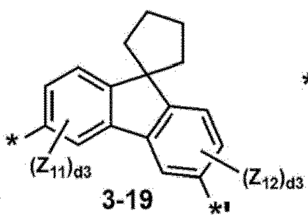
3-19

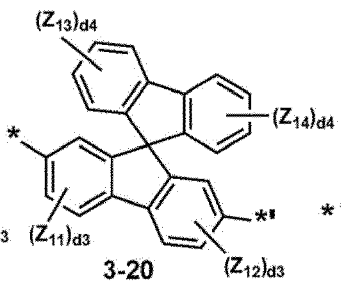
3-20

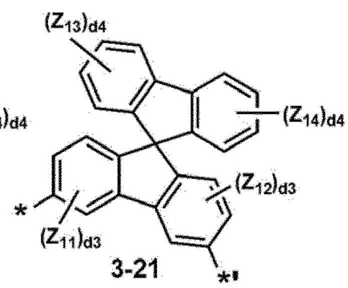
3-21

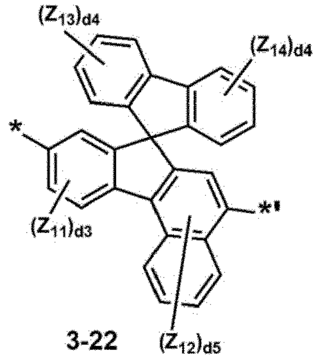

3-22

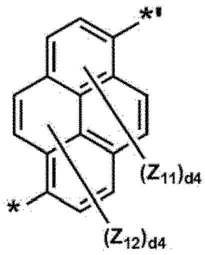

3-23

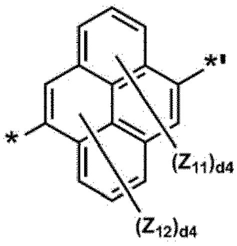

3-24

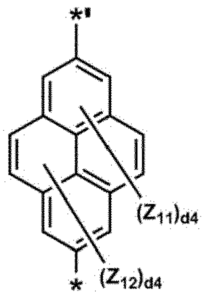

3-25

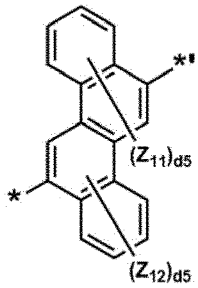

3-26

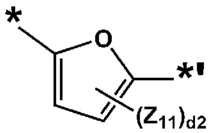

3-27

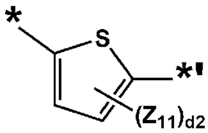

3-28

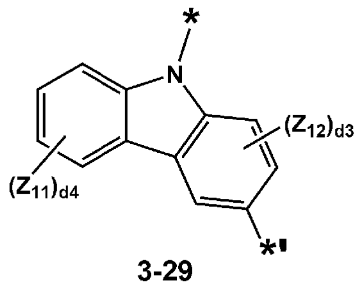

3-29

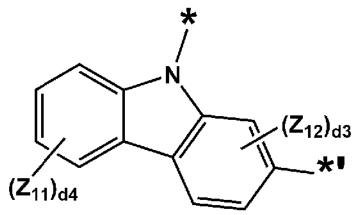

3-30

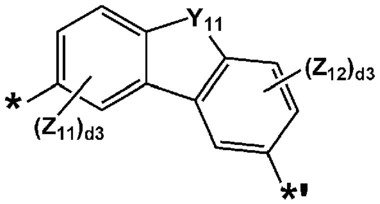

3-31

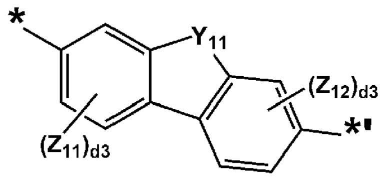

3-32

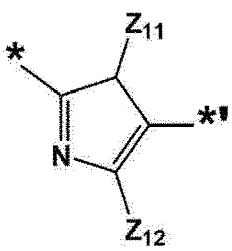

3-33

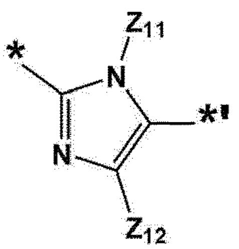

3-34

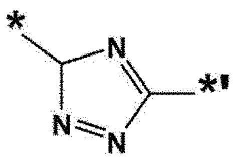

3-35

wherein, in Formulae 3-1 to 3-35,

$Y_{11}$ is *-O-*', *-S-*', or *-N($Z_{15}$)-*',
$Z_{11}$ to $Z_{15}$ are each independently selected from hydrogen, deuterium, -F, -Cl, -Br, -I, a hydroxyl group, a cyano group, a nitro group, an amino group, an amidino group, a hydrazino group, a hydrazono group, a $C_1$-$C_{20}$ alkyl group, a $C_1$-$C_{20}$ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, a dibenzofuranyl group, a dibenzothiophenyl group, a triazinyl group, a benzimidazolyl group, a phenanthrolinyl group, and -Si($Q_{31}$)($Q_{32}$)($Q_{33}$),

wherein $Q_{31}$ to $Q_{33}$ are each independently selected from a $C_1$-$C_{10}$ alkyl group, a $C_1$-$C_{10}$ alkoxy group, a phenyl

group, a biphenyl group, a terphenyl group, and a naphthyl group,

d2 is an integer from 0 to 2,
d3 is an integer from 0 to 3,
d4 is an integer from 0 to 4,
d5 is an integer from 0 to 5,
d6 is an integer from 0 to 6,
d8 is an integer from 0 to 8, and
* and *' each indicates a binding site to an adjacent atom.

**5.** An organic light-emitting device according to any one of claims 2 to 4, wherein at least one selected from $Ar_1$ and $Ar_2$ is represented by one of Formulae 4-1 to 4-34:

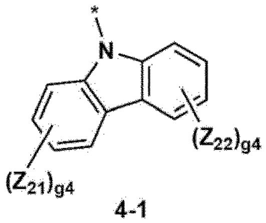
4-1

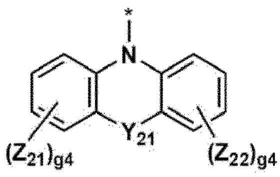
4-2

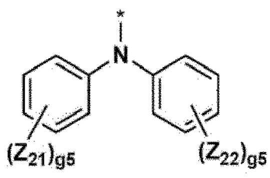
4-3

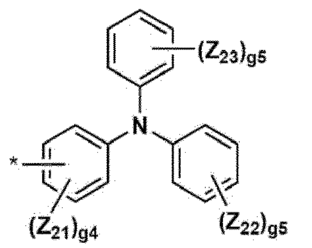
4-4

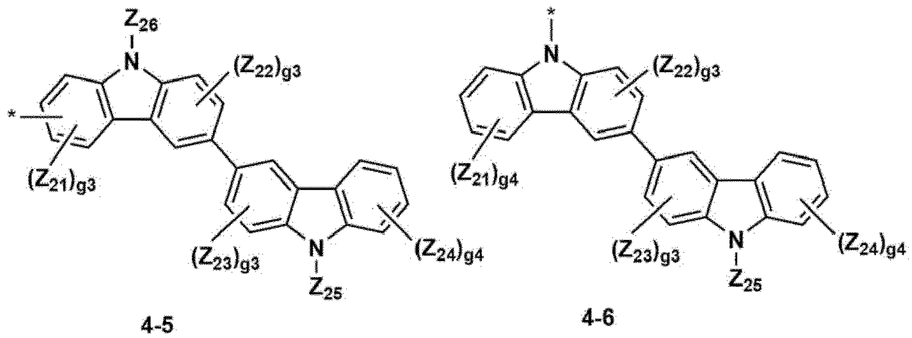
4-5

4-6

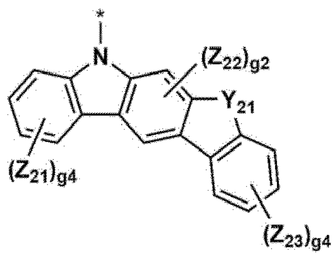
4-7

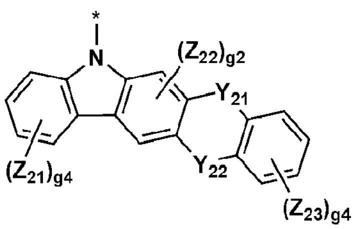
4-8

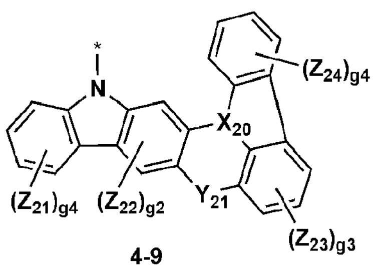
4-9

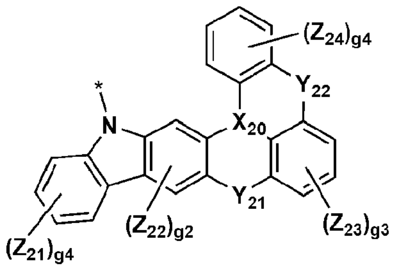
4-10

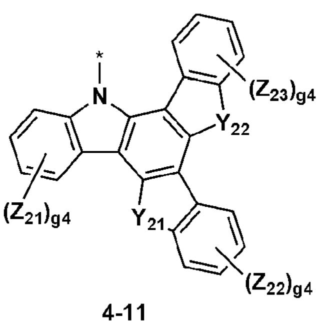
4-11

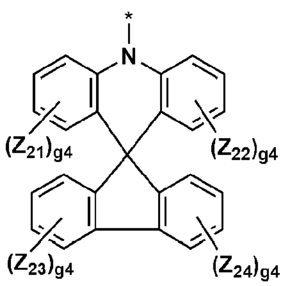
4-12

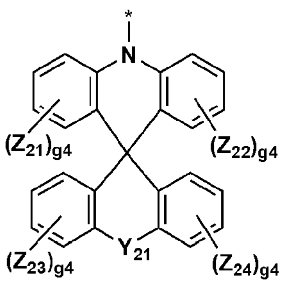
4-13

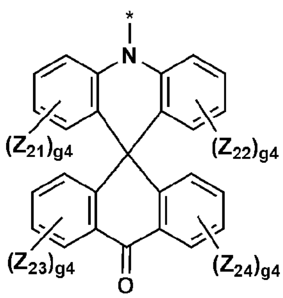
4-14

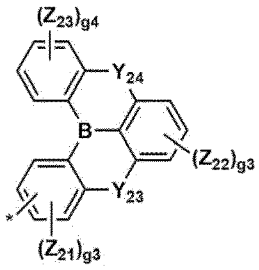

4-15

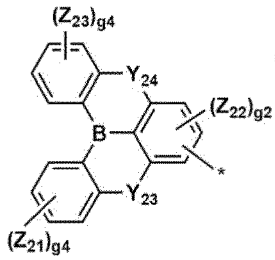

4-16

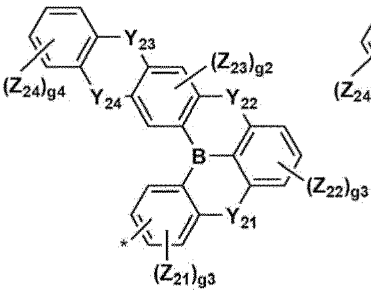

4-17

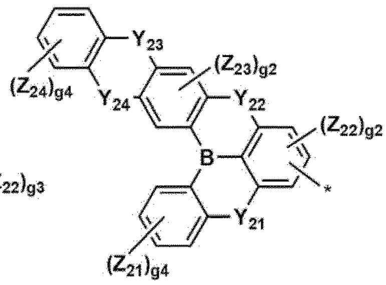

4-18

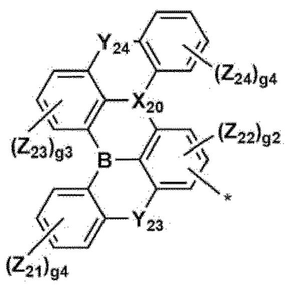

4-19

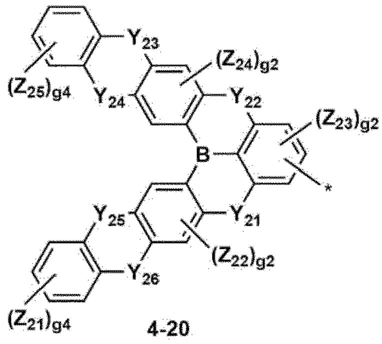

4-20

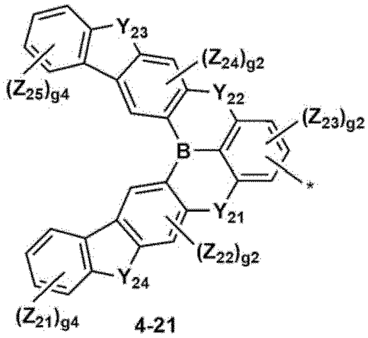

4-21

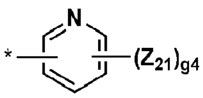

4-22

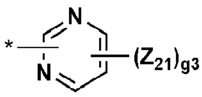

4-23

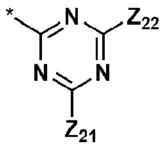

4-24

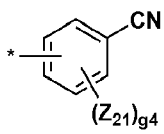

4-25

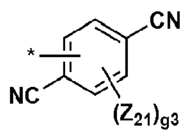

4-26

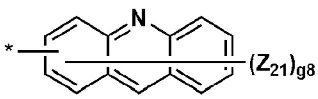

4-27

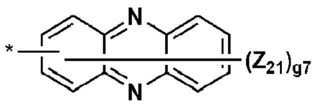

4-28

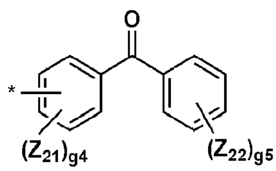

4-29

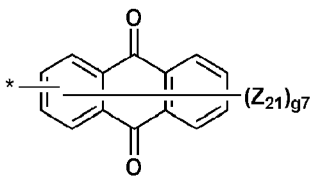

4-30

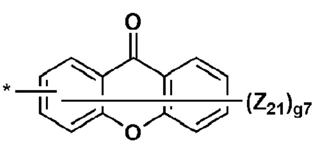

4-31

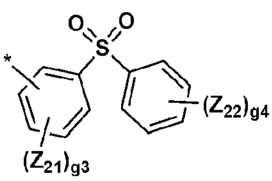

4-32

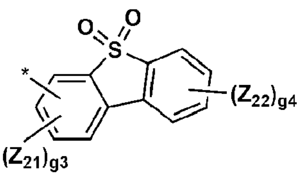

4-33

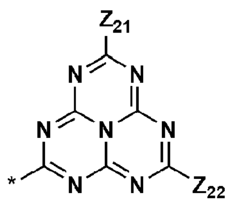

4-34

wherein, in Formulae 4-1 to 4-34,

$X_{20}$ is N, B, or P,
$Y_{21}$ and $Y_{22}$ are each independently O, S, $C(Z_{26})(Z_{27})$, $N(Z_{26})$, or $Si(Z_{26})(Z_{27})$,
$Y_{23}$ to $Y_{26}$ are each independently a single bond, O, S, $C(Z_{28})(Z_{29})$, $N(Z_{28})$, or $Si(Z_{28})(Z_{29})$,
$Z_{21}$ to $Z_{29}$ are each independently selected from hydrogen, deuterium, -F, -Cl, -Br, -I, a hydroxyl group, a cyano group, a nitro group, an amino group, an amidino group, a hydrazino group, a hydrazono group, a $C_1$-$C_{20}$ alkyl group, a $C_1$-$C_{20}$ alkoxy group, a phenyl group, a biphenyl group, a terphenyl group, a naphthyl group, a fluorenyl

group, a spiro-bifluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, a dibenzofuranyl group, a dibenzothiophenyl group, a triazinyl group, a benzimidazolyl group, a phenanthrolinyl group, and $-Si(Q_{31})(Q_{32})(Q_{33})$,

wherein $Q_{31}$ to $Q_{33}$ are each independently selected from a $C_1$-$C_{10}$ alkyl group, a $C_1$-$C_{10}$ alkoxy group, a phenyl group, a biphenyl group, a terphenyl group, and a naphthyl group,

g2 is 1 or 2,
g3 is an integer from 1 to 3,
g4 is an integer from 1 to 4,
g5 is an integer from 1 to 5,
g7 is an integer from 1 to 7,
g8 is an integer from 1 to 8, and
* indicates a binding site to an adjacent atom.

**6.** An organic light-emitting device according to any one of claims 2 to 5, wherein $R_1$, $R_2$, $R_{10}$, $R_{20}$, and $R_{30}$ are each independently selected from

hydrogen, deuterium, -F, -Cl, -Br, -I, a methyl group, an ethyl group, an n-propyl group, an iso-propyl group, an n-butyl group, a sec-butyl group, an iso-butyl group, a tert-butyl group, an ethenyl group, a propenyl group, a butenyl group, a methoxy group, an ethoxy group, an n-propoxy group, an iso-propoxy group, an n-butoxy group, a sec-butoxy group, an iso-butoxy group, and a tert-butoxy group; and
a group represented by one of Formulae 5-1 to 5-26 and Formulae 6-1 to 6-55:

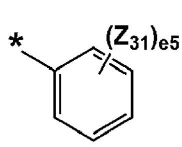

5-1

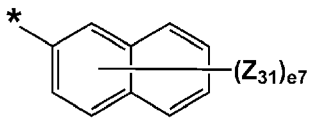

5-2

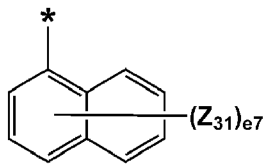

5-3

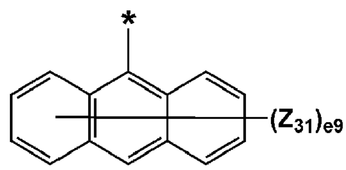

5-4

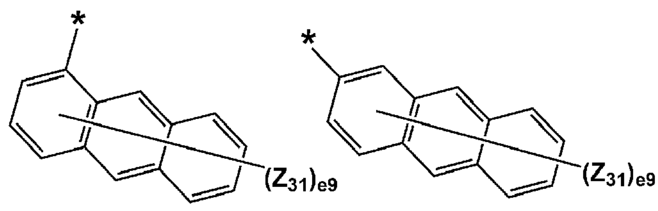

5-5 5-6

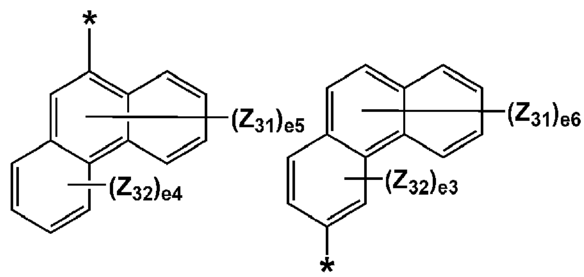

5-7 5-8

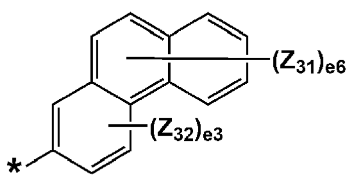

5-9

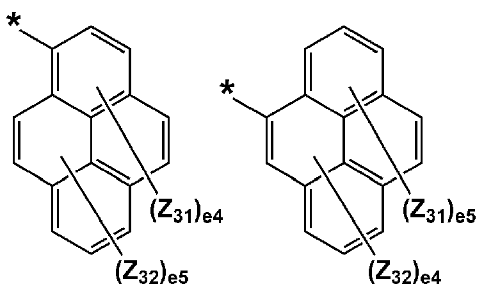

5-10 5-11

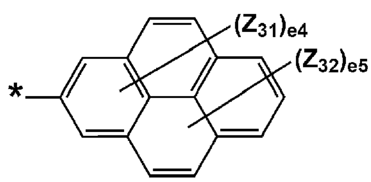

5-12

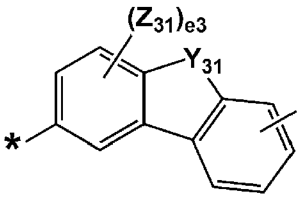

5-13

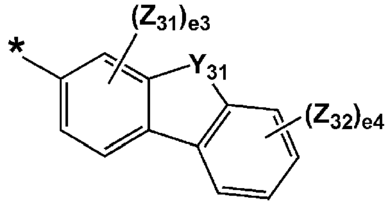

5-14

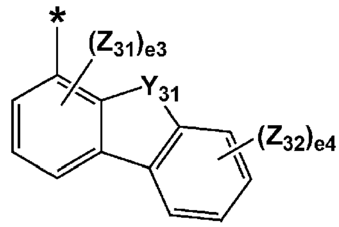

5-15

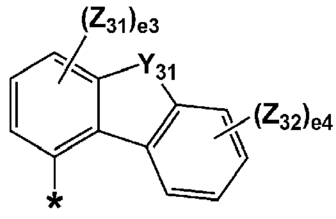

5-16

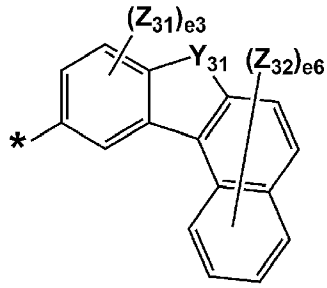

5-17

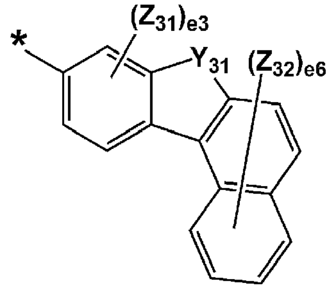

5-18

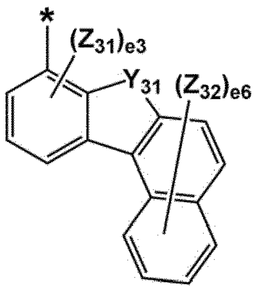

5-19

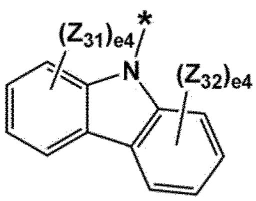

5-20

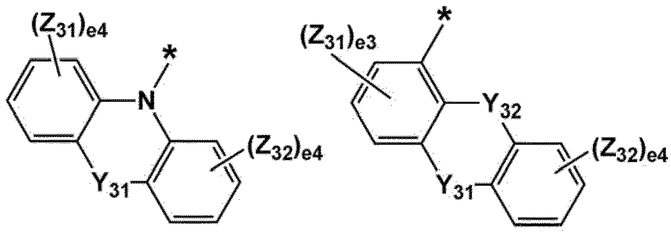

5-21

5-22

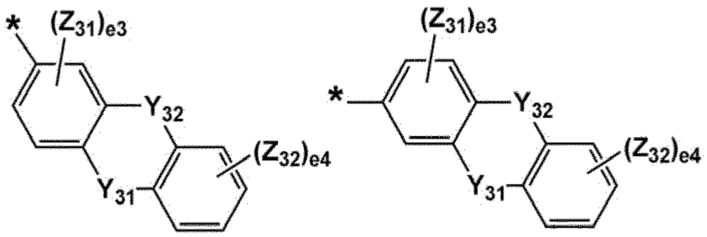

5-23

5-24

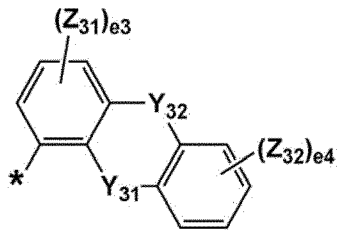

5-25

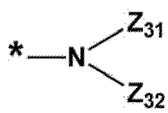

5-26

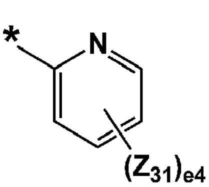

6-1

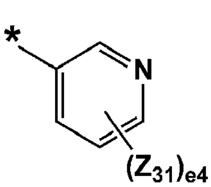

6-2

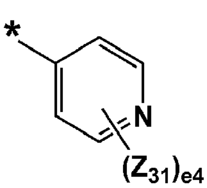

6-3

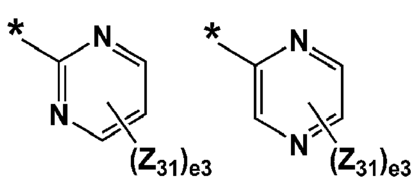

6-4

6-5

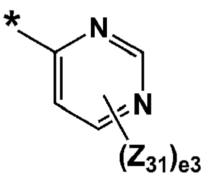

6-6

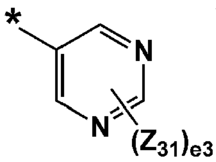

6-7

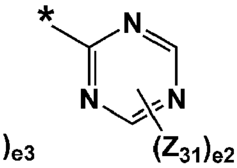

6-8

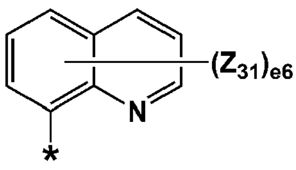

6-9

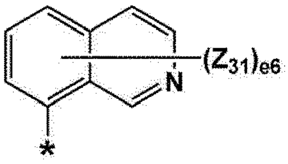

6-10

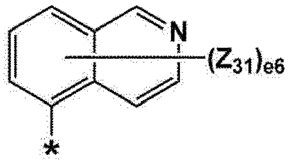

6-11

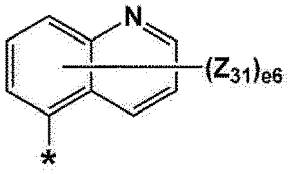

6-12

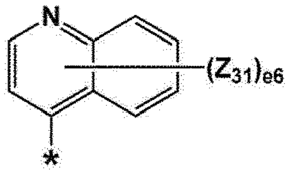

6-13

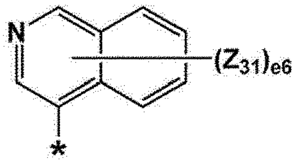

6-14

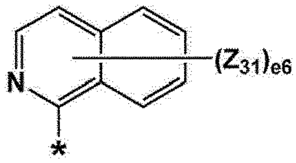

6-15

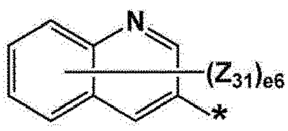

6-16

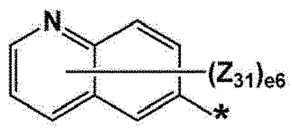

6-17

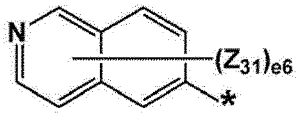

6-18

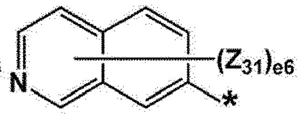

6-19

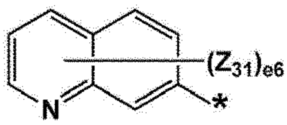

6-20

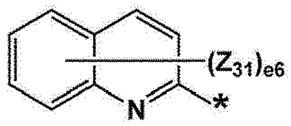

6-21

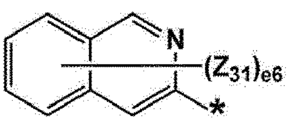

6-22

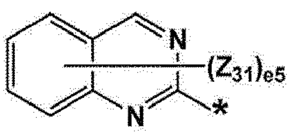

6-23

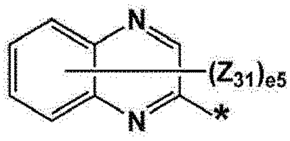

6-24

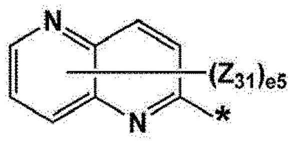

6-25

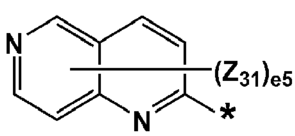

6-26

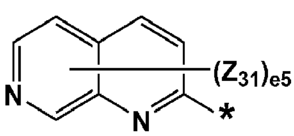

6-27

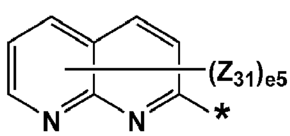

6-28

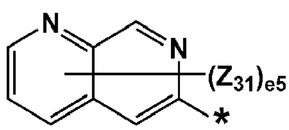

6-29

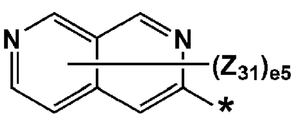

6-30

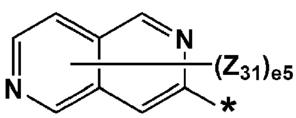

6-31

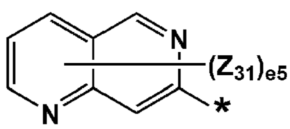

6-32

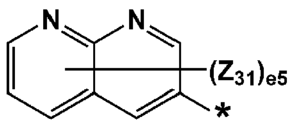

6-33

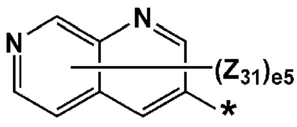
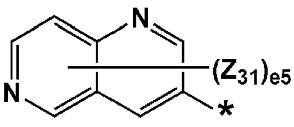
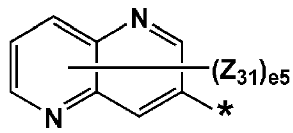
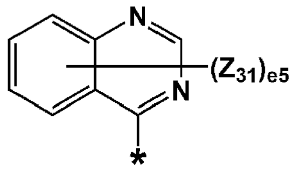

6-34 6-35 6-36 6-37

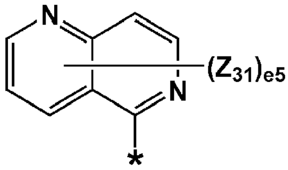
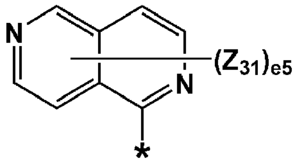
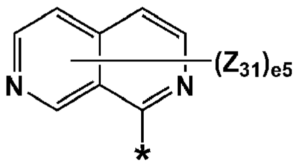
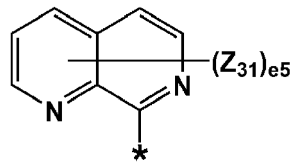

6-38 6-39 6-40 6-41

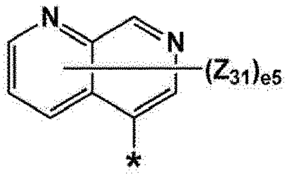
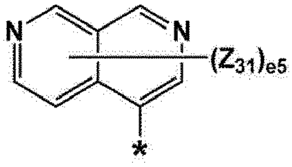
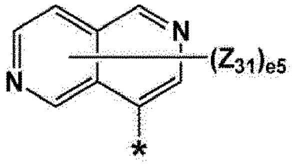
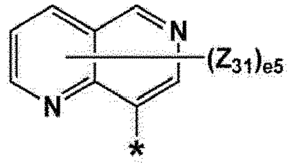

6-42 6-43 6-44 6-45

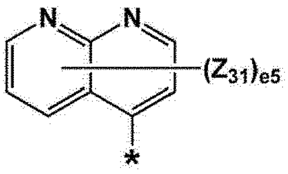
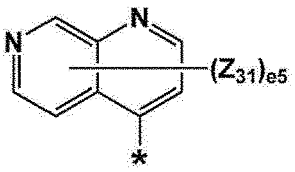
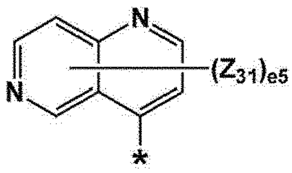
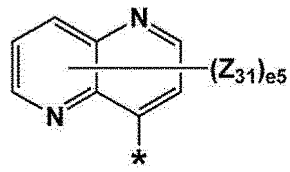

6-46 6-47 6-48 6-49

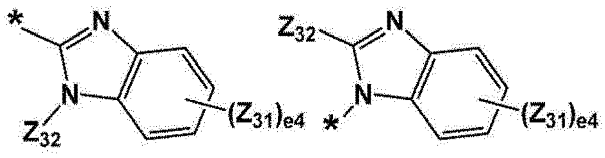
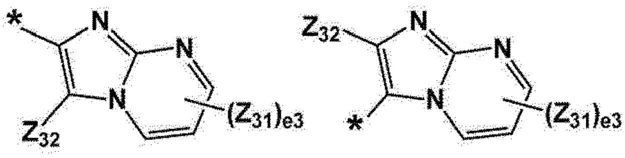

6-50 6-51 6-52 6-53

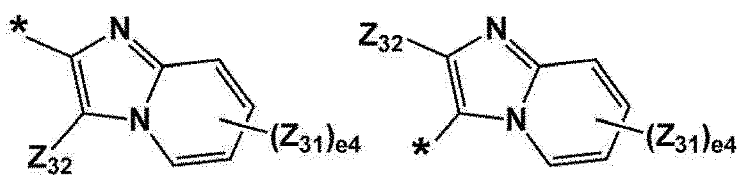

6-54 6-55

wherein, in Formulae 5-1 to 5-26 and Formulae 6-1 to 6-55,

$Y_{31}$ and $Y_{32}$ are each independently O, S, $C(Z_{34})(Z_{35})$, $N(Z_{34})$, or $Si(Z_{34})(Z_{35})$,

$Z_{31}$, $Z_{32}$, $Z_{34}$, and $Z_{35}$ are each independently selected from hydrogen, deuterium, -F, -Cl, -Br, -I, a hydroxyl group, a cyano group, a nitro group, an amidino group, a hydrazino group, a hydrazono group, a $C_1$-$C_{20}$ alkyl group, a $C_1$-$C_{20}$ alkenyl group, a $C_1$-$C_{20}$ alkynyl group, a $C_1$-$C_{20}$ alkoxy group, a phenyl group, a biphenyl group, a terphenyl group, a naphthyl group, a fluorenyl group, a spiro-bifluorenyl group, a phenanthrenyl group, an anthracenyl group, a triphenylenyl group, a pyridinyl group, a pyrimidinyl group, a carbazolyl group, and a triazinyl group,

e2 is 1 or 2,
e3 is an integer from 1 to 3,
e4 is an integer from 1 to 4,
e5 is an integer from 1 to 5,
e6 is an integer from 1 to 6,
e7 is an integer from 1 to 7,
e9 is an integer from 1 to 9, and
* indicates a binding site to an adjacent atom.

**7.** The organic light-emitting device of claim 1, wherein the host comprises at least one selected from Compounds 1-1 to 1-17:

Compound 1-1

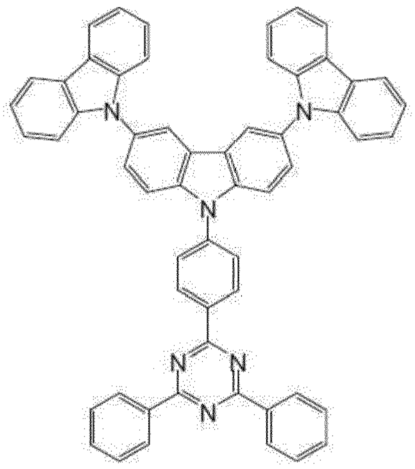

Compound 1-2

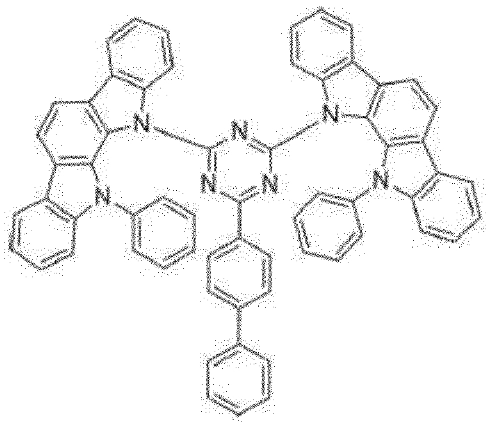

Compound 1-3

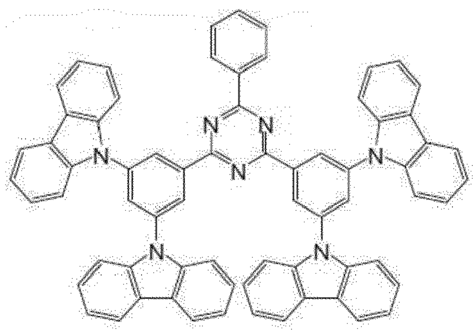

Compound 1-4

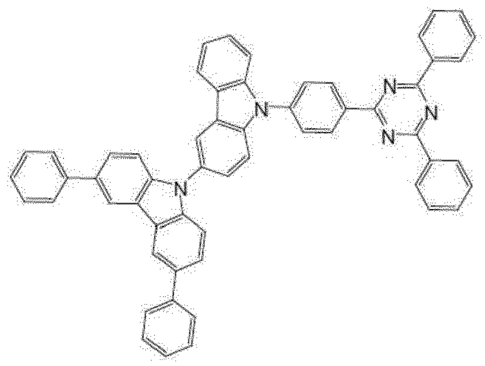

Compound 1-5

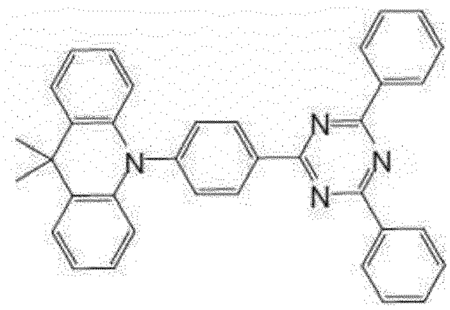

Compound 1-6

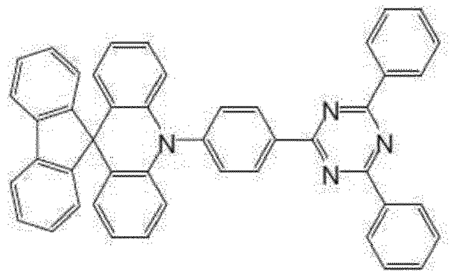

Compound 1-7

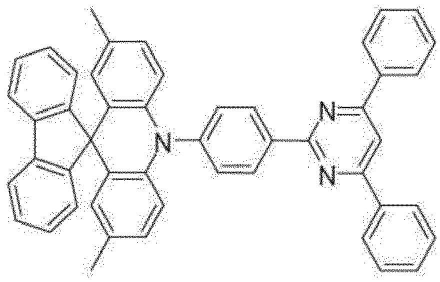

Compound 1-8

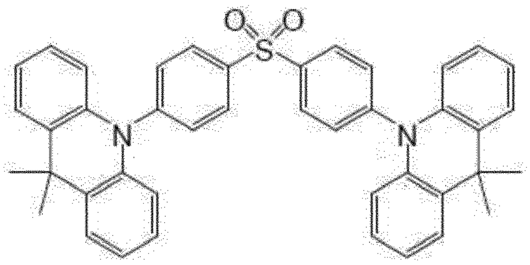

Compound 1-9

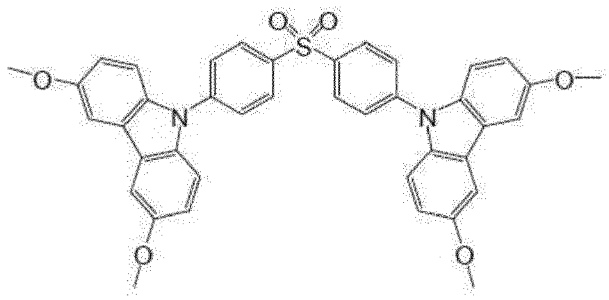

Compound 1-10

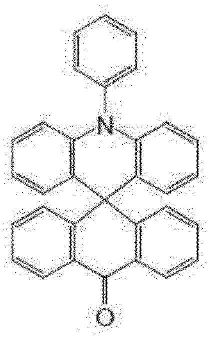

Compound 1-11

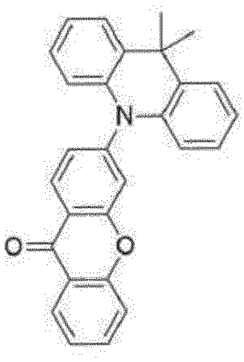

Compound 1-12

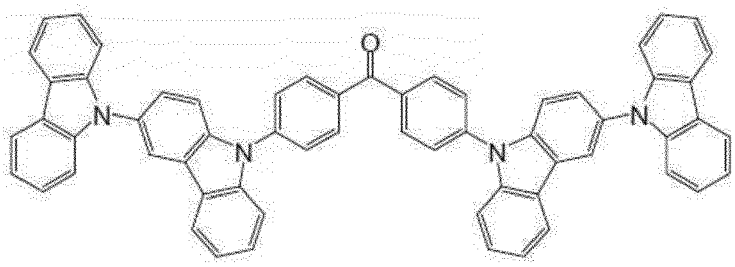

Compound 1-13

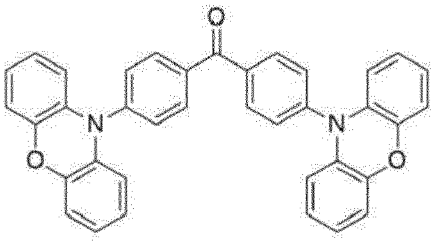

Compound 1-14

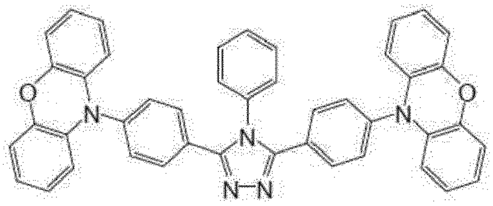

Compound 1-15

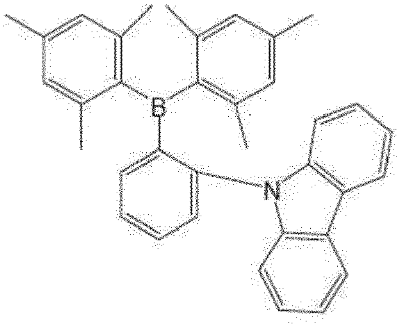

Compound 1-16

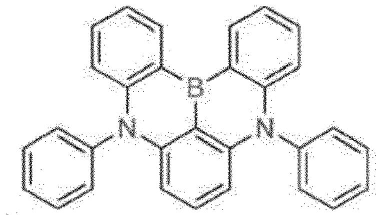

Compound 1-17

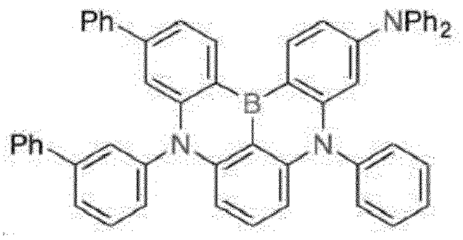

wherein "Ph" in Compounds 1-1 to 1-17 represents a phenyl group.

**8.** An organic light-emitting device according to any one of claims 2 to 7, wherein the host consists of one of the heterocyclic compound represented by Formula 1.

**9.** An organic light-emitting device according to any one of claims 1 to 8, wherein the phosphorescent dopant comprises an organometallic compound represented by one of Formulae 4 and 5:

Formula 4:

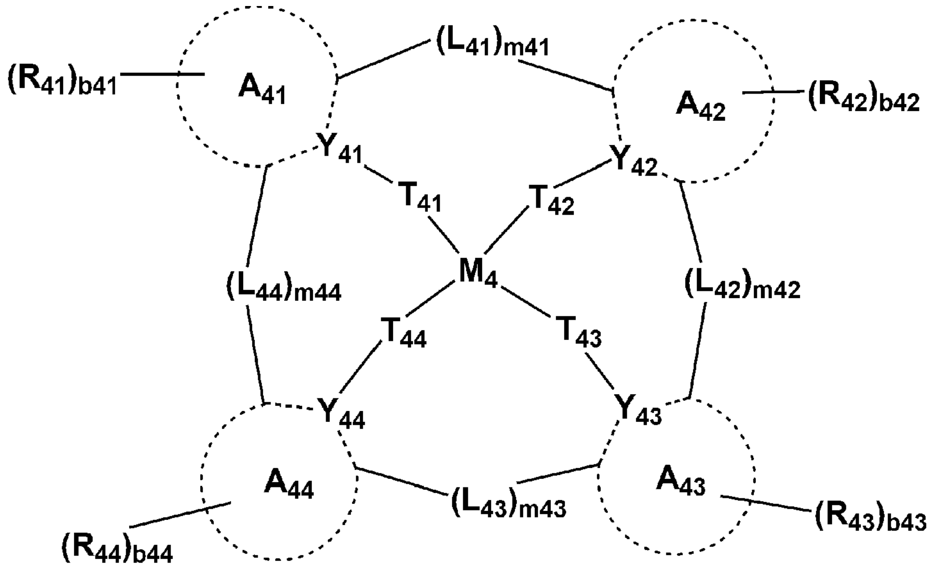

Formula 5:

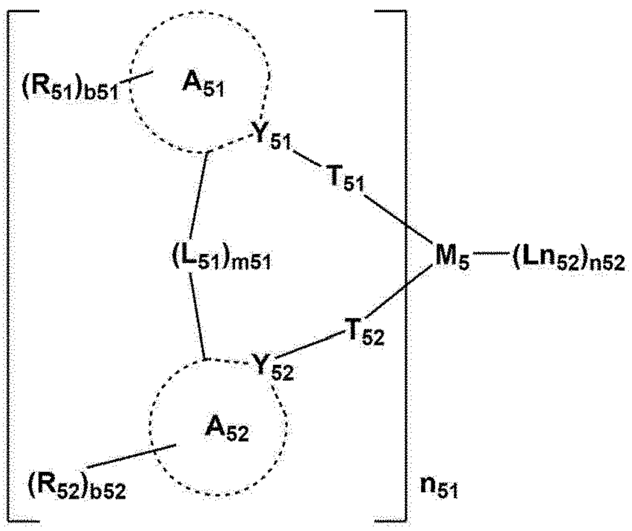

wherein, in Formulae 4 and 5,

M4 and $M_5$ are each independently selected from platinum (Pt), palladium (Pd), copper (Cu), silver (Ag), gold (Au), rhodium (Rh), iridium (Ir), ruthenium (Ru), osmium (Os), titanium (Ti), zirconium (Zr), hafnium (Hf), europium (Eu), terbium (Tb), and thulium (Tm),
n51 is an integer from 1 to 3,
$Ln_{52}$ is an organic ligand, n52 is an integer from 0 to 2,
$Y_{41}$ to $Y_{44}$, $Y_{51}$, and $Y_{52}$ are each independently N or C,
$A_{41}$ to $A_{44}$, $A_{51}$, and $A_{52}$ are each independently selected from a $C_5$-$C_{60}$ carbocyclic group and a $C_1$-$C_{60}$ heterocyclic group,
$T_{41}$ to $T_{44}$, $T_{51}$, and $T_{52}$ are each independently selected from a single bond, *-O-*', and *-S-*',
$L_{41}$ to $L_{44}$ and $L_{51}$ are each independently selected from a single bond, *-O-*', *-S-*', *-C($R_{45}$)($R_{46}$)-*', *-C($R_{45}$)=*', *=C($R_{45}$)-*', *-C($R_{45}$)=C($R_{45}$)-*', *-C(=O)-*', *-C(=S)-*', *-C=C-*', *-B($R_{45}$)-*', *-N($R_{45}$)-*', *-P($R_{45}$)-*', *-Si($R_{45}$)($R_{46}$)-*', *-P($R_{45}$)($R_{46}$)-*', and *-Ge($R_{45}$)($R_{46}$)-*', m41 to m44, and m51 are each an integer from 0 to 3,
$R_{41}$ to $R_{46}$, $R_{51}$, and $R_{52}$ are each independently selected from hydrogen, deuterium, -F,-Cl, -Br, -I, a hydroxyl group, a cyano group, a nitro group, an amidino group, a hydrazino group, a hydrazono group, a substituted or unsubstituted $C_1$-$C_{20}$ alkyl group, a substituted or unsubstituted $C_1$-$C_{20}$ alkoxy group, a substituted or unsubstituted $C_2$-$C_{60}$ alkynyl group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted

or unsubstituted $C_1$-$C_{10}$ heterocycloalkenyl group, a substituted or unsubstituted $C_6$-$C_{60}$ aryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted monovalent non-aromatic condensed polycyclic group and a substituted or unsubstituted monovalent non-aromatic condensed heteropolycyclic group, -Si($Q_{41}$)($Q_{42}$)($Q_{43}$), -N($Q_{41}$)($Q_{42}$),-B($Q_{41}$)($Q_{42}$), -C(=O)($Q_{41}$), -S(=O)$_2$($Q_{41}$), and -P(=O)($Q_{41}$)($Q_{42}$),
$R_{45}$ and $R_{41}$; $R_{45}$ and $R_{42}$; $R_{45}$ and $R_{43}$; or $R_{45}$ and $R_{44}$ are optionally bound to form a substituted or unsubstituted $C_5$-$C_{60}$ carbocyclic group or a substituted or unsubstituted $C_1$-$C_{60}$ heterocyclic group,
b41, b42, b43, b44, b51, and b52 are each independently an integer from 1 to 8,
* and *' each indicates a binding site to an adjacent atom, and

at least one substituent of the substituted $C_5$-$C_{60}$ carbocyclic group, the substituted $C_1$-$C_{60}$ heterocyclic group, the substituted $C_1$-$C_{20}$ alkyl group, the substituted $C_2$-$C_{60}$ alkynyl group, the substituted $C_1$-$C_{20}$ alkoxy group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_1$-$C_{10}$ heterocycloalkenyl group, the substituted $C_6$-$C_{60}$ aryl group, the substituted $C_6$-$C_{60}$ aryloxy group, the substituted $C_6$-$C_{60}$ arylthio group, the substituted $C_1$-$C_{60}$ heteroaryl group, the substituted monovalent non-aromatic condensed polycyclic group, and the substituted monovalent non-aromatic condensed heteropolycyclic group is selected from:
deuterium, -F, -Cl, -Br, -I, a hydroxyl group, a cyano group, a nitro group, an amidino group, a hydrazino group, a hydrazono group, a $C_1$-$C_{60}$ alkyl group, a $C_2$-$C_{60}$ alkenyl group, a $C_2$-$C_{60}$ alkynyl group, and a $C_1$-$C_{60}$ alkoxy group;
a $C_1$-$C_{60}$ alkyl group, a $C_2$-$C_{60}$ alkenyl group, a $C_2$-$C_{60}$ alkynyl group, and a $C_1$-$C_{60}$ alkoxy group, each substituted with at least one selected from deuterium, -F, -Cl, -Br, -I, a hydroxyl group, a cyano group, a nitro group, an amidino group, a hydrazino group, a hydrazono group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, -Si($Q_{51}$)($Q_{52}$)($Q_{53}$),-N($Q_{51}$)($Q_{52}$), -B($Q_{51}$)($Q_{52}$), -C(=O)($Q_{51}$), -S(=O)$_2$($Q_{51}$), and -P(=O)($Q_{51}$)($Q_{52}$);
a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, and a monovalent non-aromatic condensed heteropolycyclic group;
a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, and a monovalent non-aromatic condensed heteropolycyclic group, each substituted with at least one selected from deuterium, -F, -Cl, -Br, -I, a hydroxyl group, a cyano group, a nitro group, an amidino group, a hydrazino group, a hydrazono group, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, -Si($Q_{61}$)($Q_{62}$)($Q_{63}$), -N($Q_{61}$)($Q_{62}$), -B($Q_{61}$)($Q_{62}$), -C(=O)($Q_{61}$),-S(=O)$_2$($Q_{61}$), and -P(=O)($Q_{61}$)($Q_{62}$); and -Si($Q_{71}$)($Q_{72}$)($Q_{73}$), -N($Q_{71}$)($Q_{72}$), -B($Q_{71}$)($Q_{72}$), -C(=O)($Q_{71}$), -S(=O)$_2$($Q_{71}$), and-P(=O)($Q_{71}$)($Q_{72}$),

wherein $Q_{41}$ to $Q_{43}$, $Q_{51}$ to $Q_{53}$, $Q_{61}$ to $Q_{63}$, and $Q_{71}$ to $Q_{73}$ are each independently selected from hydrogen, deuterium, -F, -Cl, -Br, -I, a hydroxyl group, a cyano group, a nitro group, an amidino group, a hydrazino group, a hydrazono group, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a $C_1$-$C_{60}$ heteroaryloxy group, a $C_1$-$C_{60}$ heteroarylthio group, a monovalent non-aromatic condensed polycyclic group, a monovalent non-aromatic condensed heteropolycyclic group, a $C_1$-$C_{60}$ alkyl group substituted with at least one selected from deuterium, -F, and a cyano group, a $C_6$-$C_{60}$ aryl group substituted with at least one selected from deuterium, -F, and a cyano group, a biphenyl group, and a terphenyl group.

**10.** An organic light-emitting device according to claim 9, wherein $A_{41}$ to $A_{44}$, $A_{51}$, and $A_{52}$ are each independently selected from groups represented by one of Formulae 2-1 to 2-43:

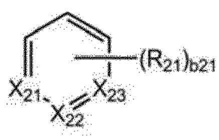
2-1

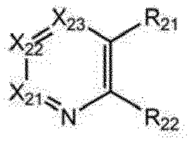
2-2

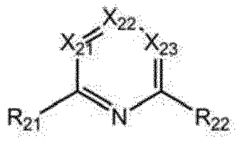
2-3

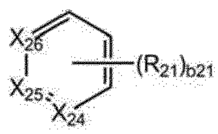
2-4

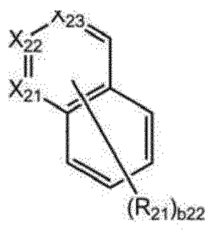
2-5

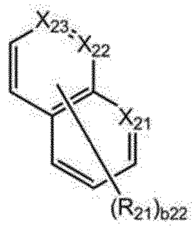
2-6

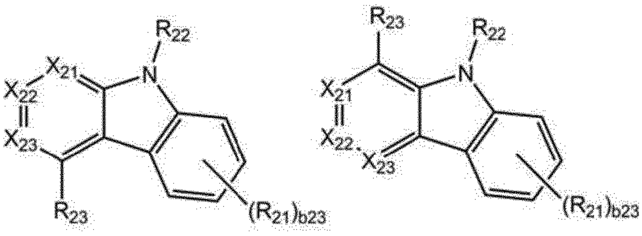
2-7

2-8

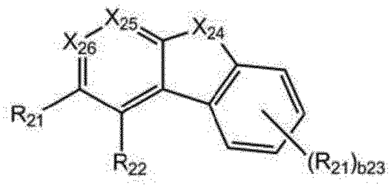
2-9

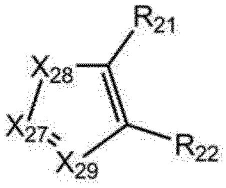
2-10

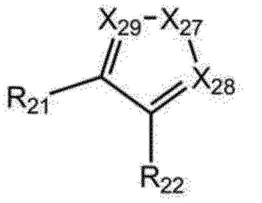
2-11

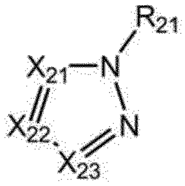
2-12

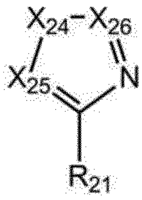
2-13

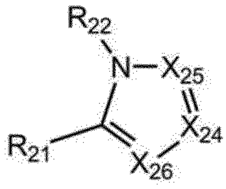
2-14

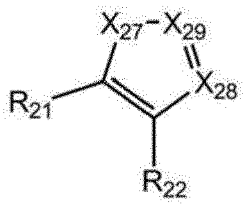
2-15

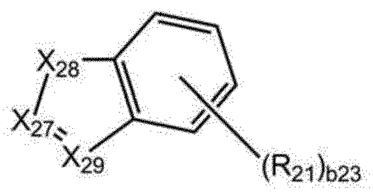
2-16

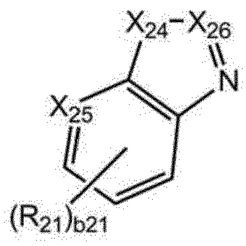
2-17

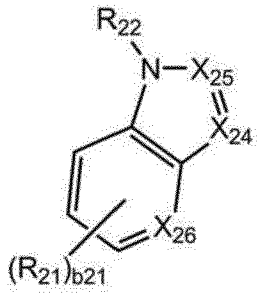
2-18

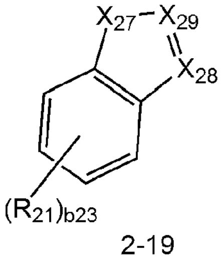
2-19

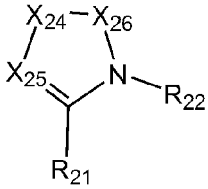
2-20

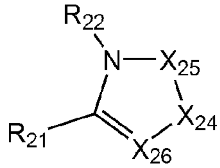
2-21

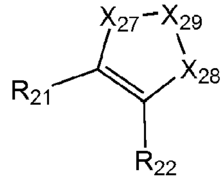
2-22

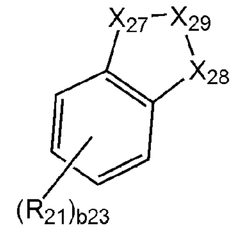
2-23

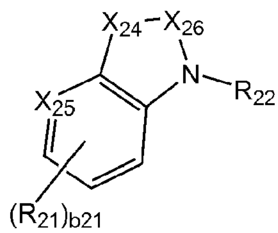
2-24

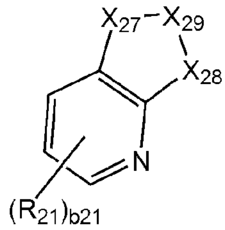
2-25

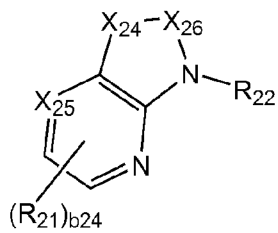
2-26

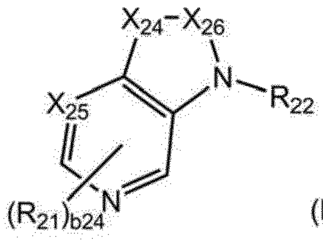
2-27

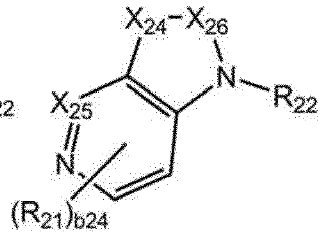
2-28

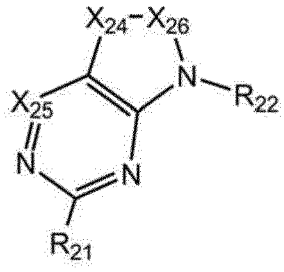
2-29

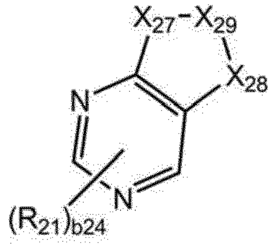
2-30

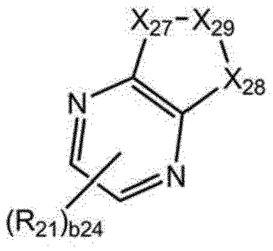
2-31

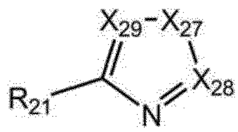
2-32

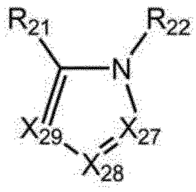
2-33

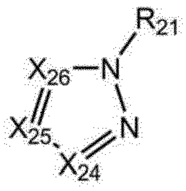
2-34

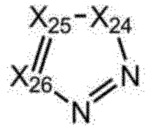
2-35

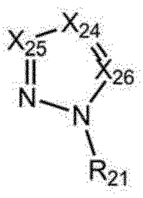
2-36

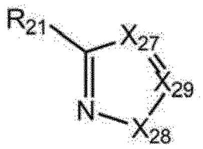
2-37

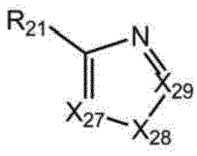
2-38

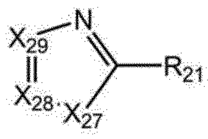
2-39

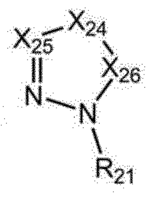
2-40

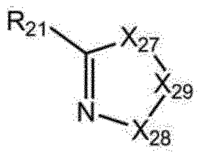
2-41

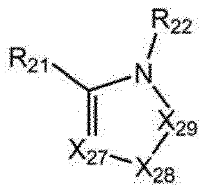
2-42

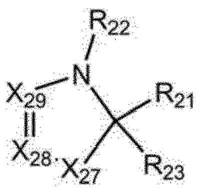
2-43

wherein, in Formulae 2-1 to 2-43,

$X_{21}$ to $X_{23}$ are each independently selected from $C(R_{24})$ and C-*, provided that at least two selected from $X_{21}$ to $X_{23}$ are each C-*,

$X_{24}$ is N-*, $X_{25}$ and $X_{26}$ are each independently selected from $C(R_{24})$ and C-*, provided that at least one selected

from $X_{25}$ and $X_{26}$ is C-*,

$X_{27}$ and $X_{28}$ are each independently selected from N, N($R_{25}$), and N-*, and $X_{29}$ is selected from C($R_{24}$) and C-*, provided that i) at least one selected from $X_{27}$ and $X_{28}$ is N-*, and $X_{29}$ is C-*, or ii) $X_{27}$ and $X_{28}$ are each N-*, and $X_{29}$ is C($R_{24}$),

$R_{21}$ to $R_{25}$ are each independently defined the same as described in connection with $R_{10}$ in claim 2,

b21 is selected from 1, 2, and 3,

b22 is selected from 1, 2, 3, 4, and 5,

b23 is selected from 1, 2, 3, and 4,

b24 is selected from 1 and 2, and

* indicates a binding site to an adjacent atom.

**11.** An organic light-emitting device according to claim 9 or claim 10, wherein

$M_4$ is Pt,

$M_5$ is Ir,

$T_{41}$ to $T_{44}$, $T_{51}$, and $T_{52}$ are each a single bond, and

$L_{41}$ to $L_{44}$ and $L_{51}$ are each independently selected from a single bond, *-O-*', *-S-*', *-C($R_{45}$)($R_{46}$)-*', *-C($R_{45}$)=*', *=C($R_{45}$)-*', *-C($R_{45}$)=C($R_{45}$)-*', *-C(=O)-*', and *-N($R_{45}$)-*'.

**12.** An organic light-emitting device according to any one of claims 9 to 11, wherein $R_{41}$ to $R_{46}$, $R_{51}$, and $R_{52}$ are each independently selected from

hydrogen, deuterium, -F, -Cl, -Br, -I, a cyano group, a $C_1$-$C_{20}$ alkyl group, and a $C_1$-$C_{20}$ alkoxy group;

a $C_1$-$C_{20}$ alkyl group and a $C_1$-$C_{20}$ alkoxy group, each substituted with at least one selected from deuterium, -F, -Cl, -Br, -I, a cyano group, a phenyl group, and a biphenyl group; and

a phenyl group, a biphenyl group, a terphenyl group, a naphthyl group, a fluorenyl group, a spiro-bifluorenyl group, a carbazolyl group, a dibenzofuranyl group, a dibenzothiophenyl group, and a dibenzosilole group; and

a phenyl group, a biphenyl group, a terphenyl group, a naphthyl group, a fluorenyl group, a spiro-bifluorenyl group, a carbazolyl group, a dibenzofuranyl group, a dibenzothiophenyl group, and a dibenzosilole group, each substituted with at least one selected from deuterium, -F, -Cl, -Br, -I, a cyano group, a $C_1$-$C_{20}$ alkyl group, a $C_1$-$C_{20}$ alkoxy group, a phenyl group, and a biphenyl group.

**13.** An organic light-emitting device according to any one of claims 9 to 12, wherein

in Formula 4, in a case where $Y_{41}$ and $Y_{42}$ are each N, $Y_{43}$ and $Y_{44}$ are each C, and m43 is 0, $A_{43}$ is a 6-membered heterocyclic group, and

in Formula 5, in a case where $A_{51}$ is a pyridine group, and $A_{52}$ is a benzene group, at least one selected from $R_{51}$ and $R_{52}$ is not hydrogen.

**14.** An organic light-emitting device according to any one of claims 1 to 13, wherein the phosphorescent dopant comprises at least one selected from Compounds 2-1 to 2-45:

Compound 2-1

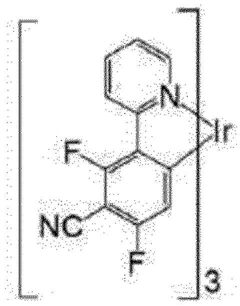

Compound 2-2

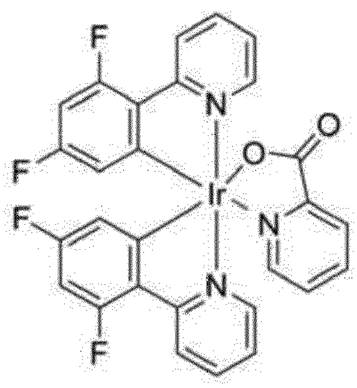

Compound 2-3

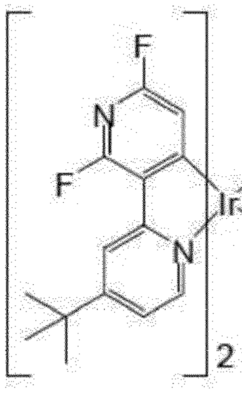

Compound 2-4

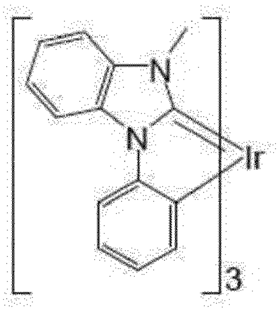

Compound 2-5

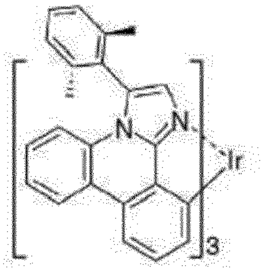

Compound 2-6

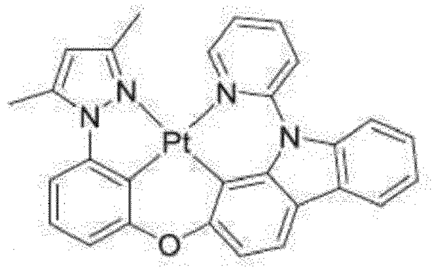

Compound 2-7

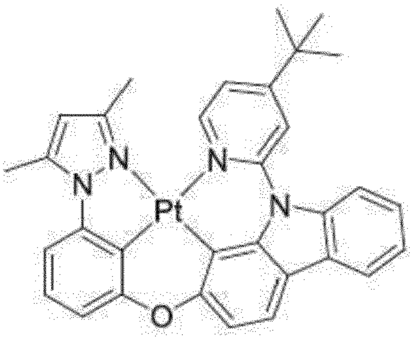

Compound 2-8

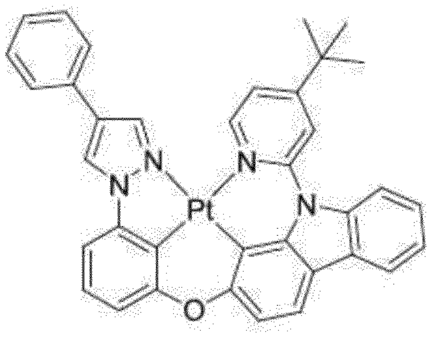

Compound 2-9

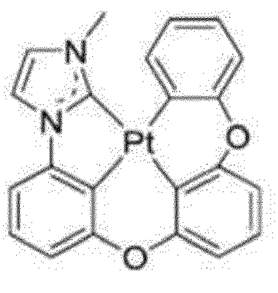

Compound 2-10

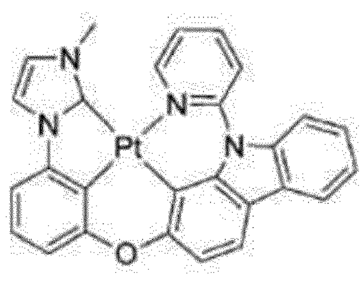

Compound 2-11

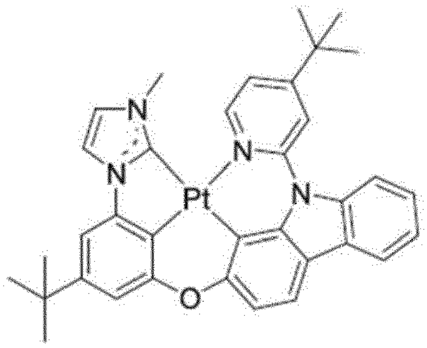

Compound 2-12

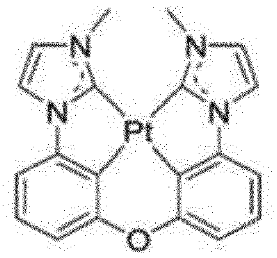

Compound 2-13

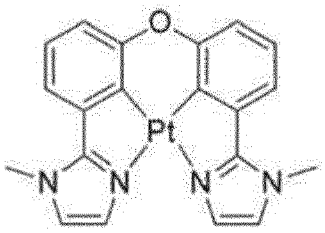

Compound 2-14

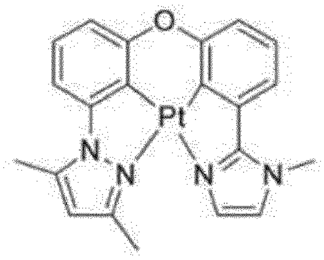

Compound 2-15

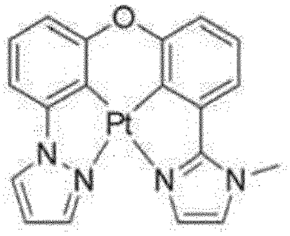

Compound 2-16

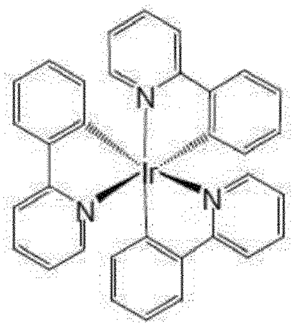

Compound 2-17

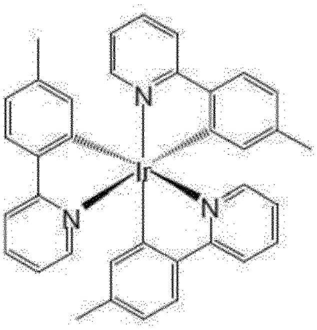

Compound 2-18

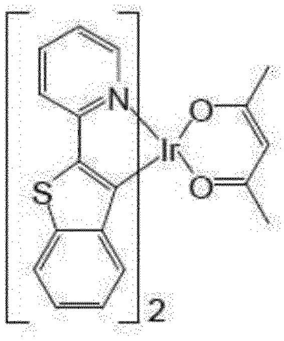

Compound 2-19

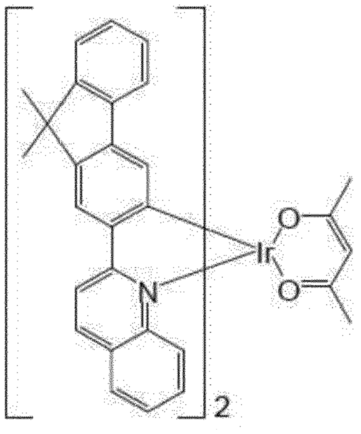

Compound 2-20

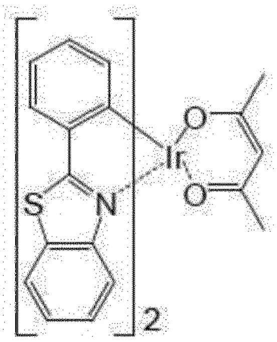

Compound 2-21

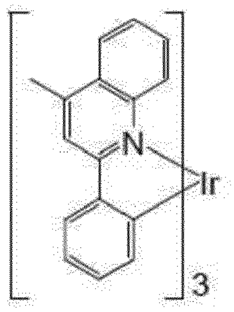

Compound 2-22

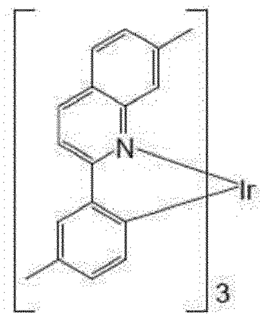

Compound 2-23

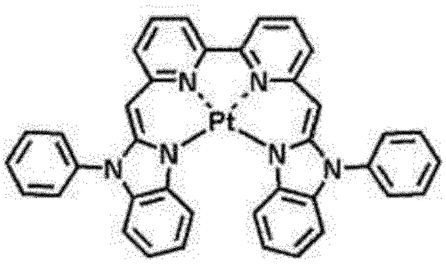

Compound 2-24

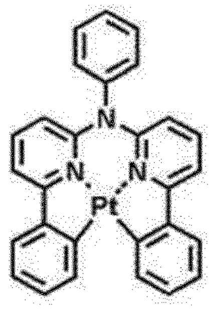

Compound 2-25

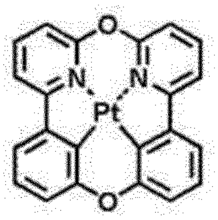

Compound 2-26

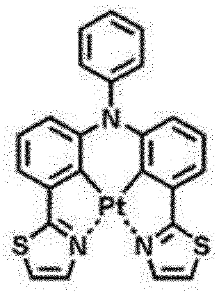

Compound 2-27

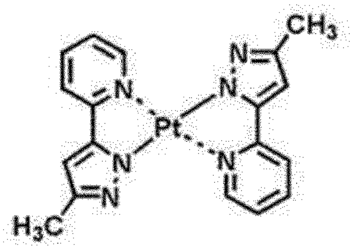

Compound 2-28

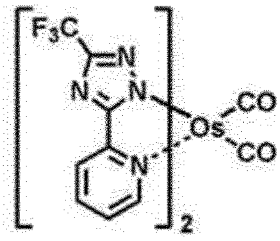

Compound 2-29

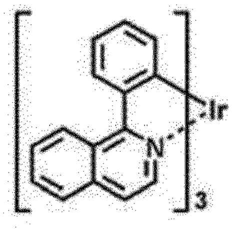

Compound 2-30

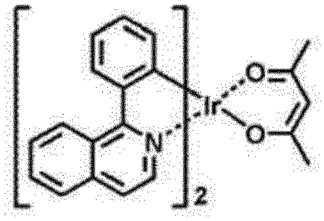

Compound 2-31

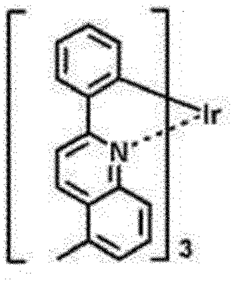

Compound 2-32

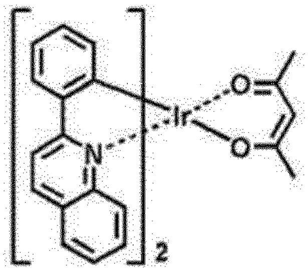

Compound 2-33

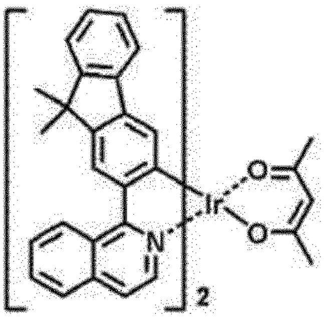

Compound 2-34

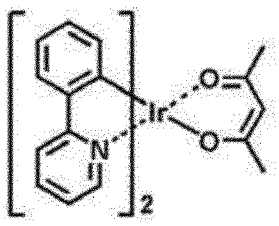

Compound 2-35

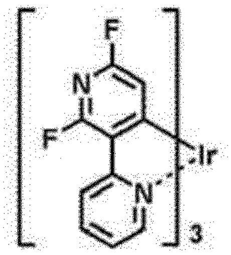

Compound 2-36

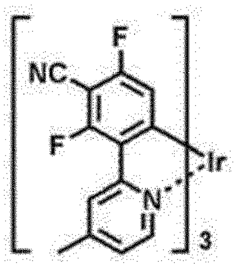

Compound 2-37

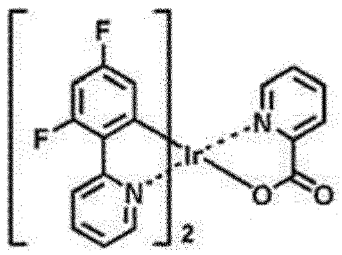

Compound 2-38

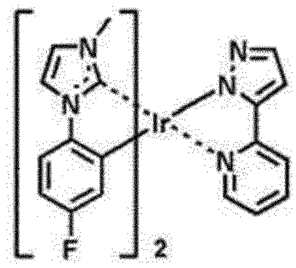

Compound 2-39

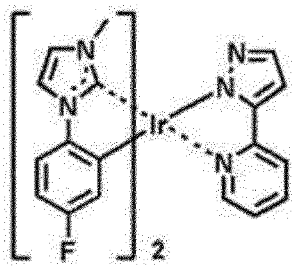

Compound 2-40

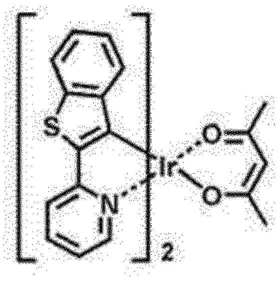

Compound 2-41

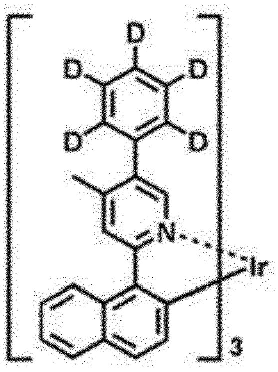

Compound 2-42

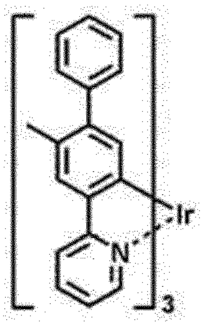

Compound 2-43

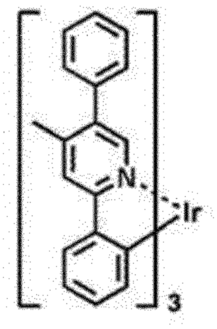

Compound 2-44

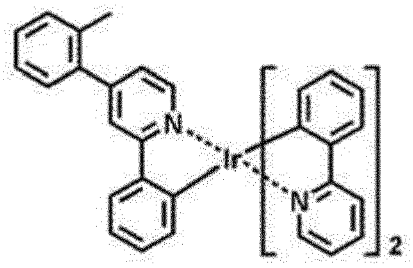

Compound 2-45

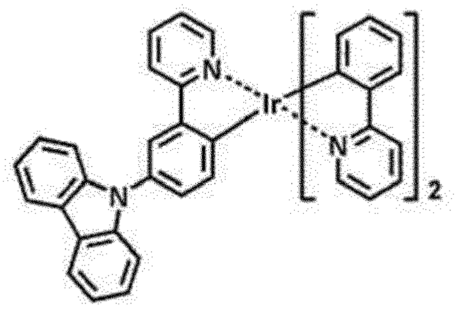

.

**15.** An organic light-emitting device according to any one of claims 1 to 14, wherein a weight percentage of the host in the emission layer is greater than a weight percentage of the phosphorescent dopant.

**16.** An organic light-emitting device according to any one of claims 1 to 15, wherein the emission layer emits blue light having a maximum emission wavelength in a range of about 420 nanometers (nm) to about 475 nm.

**17.** An organic light-emitting device according to any one of claims 1 to 16, wherein

the first electrode is an anode,
the second electrode is a cathode, and
the organic layer further comprises a hole transport region between the first electrode and the emission layer and an electron transport region between the emission layer and the second electrode,

wherein the hole transport region comprises a hole injection layer, a hole transport layer, an emission auxiliary layer, an electron blocking layer, or a combination thereof, and the electron transport region comprises a hole blocking layer, an electron transport layer, an electron injection layer, or a combination thereof.

**18.** An organic light-emitting device according to claim 17, wherein the hole transport region comprises a p-dopant, wherein a lowest unoccupied molecular orbital (LUMO) energy level of the p-dopant is -3.5 electron volts (eV) or less.

**19.** An organic light-emitting device according to claim 17 or claim 18, wherein the electron transport region comprises a metal-containing material.

**20.** An electronic apparatus comprising: an organic light-emitting device and a thin film transistor, wherein the first electrode of the organic light-emitting device is electrically connected to one of a source electrode and a drain electrode of the thin film transistor,
wherein the organic light-emitting device comprises:

a first electrode;
a second electrode facing the first electrode; and

an organic layer disposed between the first electrode and the second electrode, wherein the organic layer comprises an emission layer, the emission layer comprises a host and a phosphorescent dopant, the host satisfies Equation 1 and Equation 2, and the host and the phosphorescent dopant satisfy Equation 3:

Equation 1:

$$S1(H) - T1(H) \geq 0.3 \text{ eV}$$

Equation 2:

$$T1(H) \leq 2.7 \text{ eV}$$

Equation 3:

$$T1(D) \geq T1(H)$$

wherein, in Equations 1, 2, and 3, T1(H) indicates a triplet energy of the host, S1(H) indicates a singlet energy of the host, and T1(D) indicates a triplet energy of the phosphorescent dopant.

FIG. 1

10

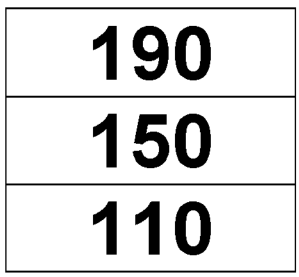

FIG. 2

20

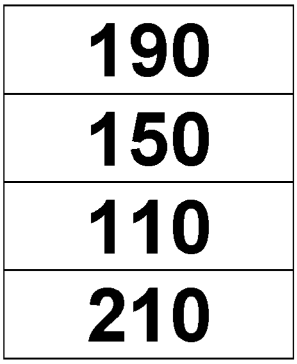

FIG. 3

30

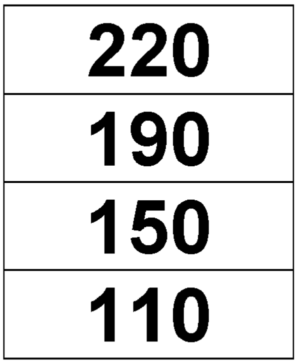

FIG. 4

40

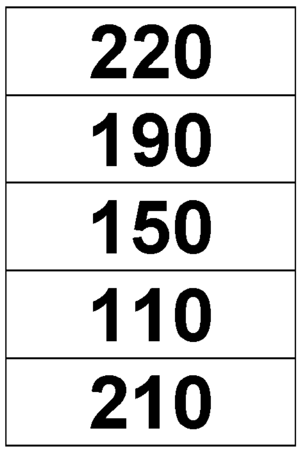

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Nature Materials,* 2015, vol. 14, 330-336
- *Adv. Mater.,* 2015, vol. 27 (15), 2515-2520
- *Chem. Sci.,* 2017, vol. 8, 953-960
- *ACS Appl. Mater. Interfaces,* 2017, vol. 9, 24035-24042
- *Adv. Mater.,* 2016, vol. 28, 2777-2781